# EUROPEAN PATENT SPECIFICATION

(11) **EP 2 491 385 B1**
(45) Date of publication and mention of the grant of the patent: **10.05.2017**
(21) Application number: 10769132.1
(22) Date of filing: 20.10.2010
(51) Int. Cl.: G01N 33/50

(54) **PROXIMITY-MEDIATED ASSAYS FOR DETECTING ONCOGENIC FUSION PROTEINS**
PROXIMITÄTSVERMITTELTE TESTS FÜR DEN NACHWEIS VON ONKOGENEN FUSIONSPROTEINEN
DOSAGES À MÉDIATION DE PROXIMITÉ POUR DÉTECTER DES PROTÉINES DE FUSION ONCOGÈNES

(30) Priority: 20.10.2009 US 253393 P; 16.02.2010 US 305084 P; 23.04.2010 US 327487 P; 15.09.2010 US 383037 P
(43) Date of publication of application: 29.08.2012
(73) Proprietor: DiaTech Holdings, Inc., Franklin, TN 37067 (US)
(72) Inventor: SINGH, Sharat, Rancho Santa Fe California 92127 (US); LIU, Xinjun, San Diego California 92130 (US)
(74) Representative: Campbell, Patrick John Henry
(86) International application number: PCT/US2010/053386
(87) International publication number: WO 2011/050069

(56) References cited:
- EP-A1- 1 507 147
- WO-A1-98/53317
- WO-A1-2007/061684
- WO-A1-2009/108637
- US-A1- 2005 214 301
- DE KEERSMAECKER KIM ET AL: "Fusion of EML1 to ABL1 in T-cell acute lymphoblastic leukemia with cryptic t(9;14)(q34;q32).", BLOOD 15 JUN 2005 LNKD- PUBMED:15713800, vol. 105, no. 12, 15 June 2005 (2005-06-15), pages 4849-4852, XP002613660, ISSN: 0006-4971
- GUO J Q ET AL: "Detection of BCR-ABL proteins in blood cells of benign phase chronic myelogenous leukemia patients.", CANCER RESEARCH 1 JUN 1991 LNKD- PUBMED:2032243, vol. 51, no. 11, 1 June 1991 (1991-06-01), pages 3048-3051, XP002613661, ISSN: 0008-5472

## Description

### BACKGROUND OF THE INVENTION

Fusion proteins, also known as chimeric proteins, are proteins created through the joining of two or more genes which originally encode separate proteins. Translation of this fusion gene results in a single polypeptide with functional properties derived from each of the original proteins. Chimeric mutant proteins occur when a large-scale mutation, typically a chromosomal translocation, creates a novel coding sequence containing parts of the coding sequences from two different genes. Naturally-occurring fusion proteins are important in cancer, where they function as oncoproteins.

The BCR-ABL fusion protein is a well-known example of an oncogenic fusion protein. It is considered to be the primary oncogenic driver of chronic myelogenous leukemia (CML), but is also associated with acute lymphoblastic leukemia (ALL). In fact, the cytogenetic hallmark of CML is the Philadelphia chromosome (Ph), which results in the formation of the *BCR-ABL* fusion gene encoding a 210 kDa protein. Indeed, the resulting BCR-ABL fusion protein is an active tyrosine kinase that is critical to the pathogenesis of CML. Although imatinib (Gleevec^{®}) is currently the first line therapy for newly diagnosed patients with CML, about 20-25% of patients do not achieve durable complete cytogenetic responses. Studies have shown that the reactivation of BCR-ABL signaling in the presence of continued imatinib treatment is the major cause of resistance. In the majority of patients, reactivation results from mutations in the BCR-ABL kinase domain which impair imatinib binding and lead to the induction of drug resistance. As such, the measurement of BCR-ABL activity finds utility in predicting response to therapy with tyrosine kinase inhibitors such as imatinib as well as in identifying patients who develop resistance to such inhibitors.

At present, methods available for detecting BCR-ABL activity rely on measuring phosphorylated CRKL (pCRKL), a BCR-ABL substrate. For example, La Rosée et al. (Haematologica, 93:765-9 (2008)) describes a Western blot analysis of total leukocyte lysates to determine the level of pCRKL as a surrogate of BCR-ABL activity (*see also,* Hochhaus et al., Leukemia, 16:2190-6 (2002); White et al., J. Clin. Oncol., 25:4445-51 (2007)). Similarly, Khorashad et al. (Haematologica, 94:861-4 (2009)) describes a flow cytometry-based method of measuring the level of pCRKL to evaluate BCR-ABL activity (*see also,* Hamilton et al., Leukemia, 20:1035-9 (2006)). However, these methods lack the specificity and sensitivity that is required for determining the presence or level of BCR-ABL activity in a sample because they are single antibody assays which rely on detecting the phosphorylation of a surrogate protein.

EP 1507147 relates to the detection of among others tumor-specific fusion proteins.

WO 98/53317 relates to the field of cancer diagnosis and the application of diagnostic techniques in pathology and haematology. Specifically, the invention relates to techniques for the detection of chromosomal aberrations and the detection of tumor specific gene products exclusively expressed by tumor cells containing said chromosomal aberrations.

WO 2009/108637 provides compositions and methods for detecting the activation states of components of signal transduction pathways in tumor cells.

WO 2007/061684 concerns the detection of gene products resulting from chromosomal translocations, including fusion proteins comprising a first and second region. In particular, the fusion proteins are identified following subjecting a sample comprising the proteins to a bead comprising an antibody to a first region, followed by subjecting the bead- antibody-fusion complex to a second antibody directed against the second region, thereby detecting the fusion protein.

US 2005/214301 provides isolated antibodies that specifically bind human P210 BCR-ABL fusion protein, but do not bind the respective wild type BCR and c-ABL proteins. The detection of this fusion protein is relevant to CML and other diseases characterized by the P210 BCR-ABL translocation. Also provided are methods for determining the level or expression of P210 BCR-ABL in a biological sample, or identifying a compound that modulates such expression, by using the disclosed BCR-ABL specific antibodies.

De keersmaecker et al. (2005); Blood: 105 (12) demonstrate the involvement of *ABL1* fusions in the pathogenesis of T-ALL and identify EML1-ABL1 as a novel therapeutic target of imatinib.

Guo et al. (1991); Cancer Res: 51;3048 relates to detection of BCR-ABL Proteins in Blood Cells of Benign Phase Chronic Myelogenous Leukemia Patients.

Accordingly, specific and sensitive methods are needed to detect BCR-ABL activity, as well as the activity of other oncogenic fusion proteins, for diagnostic, prognostic, and therapeutic purposes. The present invention satisfies this need and provides related advantages as well.

### BRIEF SUMMARY OF THE INVENTION

The present invention provides antibody-based arrays for detecting the activation state and/or total amount of one or a plurality of oncogenic fusion proteins in a biological sample such as whole blood (*e.g.,* a lysate prepared from isolated rare circulating cells or leukocytes) or tumor tissue (*e.g.,* a fine needle aspirate) and methods of use thereof In certain instances, the activation state and/or total amount of oncogenic fusion protein(s) present in a sample can be measured in combination with one or a plurality of signal transduction molecules. The compositions and methods of the present invention have the advantages of specificity associated with enzyme-linked immunosorbent assays, sensitivity associated with signal amplification, and high-throughput multiplexing associated with microarrays.

In one aspect, the present invention provides a method for determining the level or activation state of an oncogenic fusion protein, the method comprising:
(a) producing a cellular extract comprising an extract of cells isolated from a sample, wherein the isolated cells are not washed prior to lysis to produce the cellular extract;
(b) contacting a cellular extract with a first binding moiety specific for a first domain of a first full-length protein under conditions suitable to transform the first full-length protein present in the cellular extract into a complex comprising the first full-length protein and the first binding moiety, wherein the first domain of the first full-length protein is absent from a corresponding oncogenic fusion protein comprising a second, different domain of the first full-length protein fused to a first domain of a second, different full-length protein;
(c) removing the complex from step (b) from the cellular extract to form a cellular extract devoid of the first full-length protein;
(d) contacting the cellular extract from step (c) with a second binding moiety, a third binding moiety, and a fourth binding moiety under conditions suitable to transform the oncogenic fusion protein present in the cellular extract into a complex comprising the oncogenic fusion protein and the second, third, and fourth binding moieties;
wherein the second binding moiety is specific for the second, different domain of the first full-length protein, wherein the second binding moiety is restrained on a solid support in an addressable array, wherein the third binding moiety is labeled with a glucose oxidase and is specific for one of the following: (i) the first domain of the second, different full-length protein; (ii) the second, different domain of the first full-length protein; or (iii) the site of fusion between the second, different domain of the first full-length protein and the first domain of the second, different full-length protein,
wherein the fourth binding moiety is labeled with a first member of a signal amplification pair and is specific for the first domain of the second, different full-length protein, and wherein the glucose oxidase generates an oxidizing agent which channels to and reacts with the first member of the signal amplification pair;
(e) incubating the complex from step (d) with a second member of the signal amplification pair to generate an amplified signal; and
(f) detecting the amplified signal generated from the first and second members of the signal amplification pair, thereby determining the level or activation state of the oncogenic fusion protein.

In one aspect, the present invention provides a method as described in [0006a], wherein the sample is selected from the group consisting of whole blood, serum, plasma, urine, sputum, bronchial lavage fluid, tears, nipple aspirate, lymph, saliva, fine needle aspirate (FNA), and combinations thereof. In one embodiment the sample is obtained from a patient having cancer. The cancer is caused by the formation of an oncogenic fusion protein due to a chromosomal translocation in the cancer cells. In one embodiment the cancer is a hematological malignancy, osteogenic sarcoma, or soft tissue sarcoma. The hematological malignancy is a leukemia or lymphoma. The leukemia is a chronic myelogenous leukemia (CML).

In one aspect, the present invention provides a method as described in [0006a-b], wherein the oncogenic fusion protein is selected from the group consisting of BCR-ABL, DEK-CAN, E2A-PBX1, RARa-PML, IREL-URG, CBF/3-MYH11, AML1-MTG8, EWS-FLI, LYT-10-Cc , HRX-ENL, HRX-AF4, NPM-ALK, IGH-MYC, RUNX1-ETO, TEL-TRKC, TEL-AML1, MLL-AF4, TCR-RBTN2, COL1A1-PDGF, E2A-HLF, PAX3-FKHR, ETV6-NTRK3, RET-PTC, TMRSS-ERG, TPR-MET, and combinations thereof. In one embodiment, the oncogenic fusion protein is BCRABL.

In one embodiment of the present invention, the first full-length protein is BCR, or wherein the first domain of the first full-length protein comprises the carboxyl-terminal region of BCR (BCRC), wherein the second, different domain of the first full-length protein comprises the aminoterminal region of BCR (BCR-N), or wherein the second, different full-length protein is ABL, or wherein the first domain of the second, different full-length protein comprises the carboxyl-terminal region of ABL (ABL-C), or wherein the first full-length protein is ABL, or wherein the second, different full-length protein is BCR.

In one embodiment the method as described in [0006a-d], wherein the activation state is selected from the group consisting of a phosphorylation state, ubiquitination state, complexation state, and combinations thereof, or further comprising determining the level or activation state of one or more signal transduction molecules, said one or more signal transduction molecules optionally being a BCR- ABL substrate, said BCR-ABL substrate optionally being selected from the group consisting of CRKL, JAK2, STAT5, VAV, BAP-1, and combinations thereof.

In one embodiment the method as described in [0006a-d], wherein the third binding moiety is directly labeled with the glucose oxidase, or wherein the fourth binding moiety is directly labeled with the first member of the signal amplification pair, or wherein the fourth binding moiety is labeled with the first member of the signal amplification pair via binding between a first member of a binding pair conjugated to the second detection antibody and a second member of the binding pair conjugated to the first member of the signal amplification pair.

In one embodiment the method as described in [0006a-f], further comprising:
(g) contacting the cellular extract with a fifth binding moiety specific for a second domain of the second, different full-length protein under conditions suitable to transform the second, different full-length protein present in the cellular extract into a complex comprising the second, different full-length protein and the fifth binding moiety, wherein the second domain of the second, different full-length protein is absent from the oncogenic fusion protein; and
(h) removing the complex from step (g) from the cellular extract to form a cellular extract devoid of the second, different full-length protein,
wherein step (g) is performed before, during, or after step (b).

In another aspect, the present invention relates to a method for optimizing therapy and/or reducing toxicity in a subject having cancer and receiving a course of therapy for the treatment of cancer, the method comprising:
(a) lysing isolated cancer cells from a subject after administration of an anticancer drug to produce a cellular extract, wherein the isolated cancer cells are not washed prior to lysis;
(b) measuring a level of expression and/or activation of an oncogenic fusion protein in the 10 cellular extract in accordance with the method of any one of claims 1 to 25; and
(c) comparing the measured level of expression and/or activation of the oncogenic fusion protein to a level of expression and/or activation of the oncogenic fusion protein measured at an earlier time during the course of therapy; and
(d) determining a subsequent dose of the course of therapy for the subject or whether a different course of therapy should be administered to the subject based upon the comparison from step (d). In one embodiment, the oncogenic fusion protein is BCR-ABL, or wherein the subject is determined to have cancer cells that express the oncogenic fusion protein prior to receiving the course of therapy, said subject optionally being positive for BCR- ABL. In another embodiment, wherein both the level of expression and the level of activation of the oncogenic fusion protein are measured in the cellular extract, said step (b) optionally further comprises calculating a ratio of activated to total oncogenic fusion protein levels, said step (c) optionally comprises comparing the calculated ratio of activated to total oncogenic fusion protein levels to a ratio of activated to total oncogenic fusion protein levels calculated for the subject at an earlier time. In yet another embodiment, the calculated ratio of activated to total oncogenic fusion protein levels correlates with the percent inhibition of the activated oncogenic fusion protein upon anticancer drug treatment, or wherein the calculated ratio of activated to total oncogenic fusion protein levels is relative to the level of a control protein, or wherein the calculated ratio of activated to total oncogenic fusion protein levels comprises a ratio of phospho/total BCR-ABL protein levels. In one embodiment, the control protein comprises a native full-length protein containing sequences or domains found within the oncogenic fusion protein, or wherein the control protein is selected from the group consisting of full-length BCR, full-length ABL, and combinations thereof.

In one embodiment the method as described in [0006h], wherein less than about 50% inhibition of the level of activation of the oncogenic fusion protein indicates a need to increase the subsequent dose of the course of therapy or to administer a different course of therapy, or wherein less than about 50% inhibition of the level of activation of the oncogenic fusion protein indicates a lack of compliance by the subject to the course of therapy, the existence of possible side effects or toxicity associated with the course of therapy, or combinations thereof, or wherein greater than about 80% inhibition of the level of activation of the oncogenic fusion protein indicates that the subject is on the correct course of therapy at the correct dose.

In another embodiment the method as described in [0006h-i], wherein the cancer is chronic myelogenous leukemia (CML), or wherein the anticancer drug is selected from the group consisting of a monoclonal antibody, tyrosine kinase inhibitor, chemotherapeutic agent, hormonal therapeutic agent, radiotherapeutic agent, vaccine, and combinations thereof, said tyrosine kinase inhibitor is selected from the group consisting of imatinib mesylate, nilotinib, dasatinib, bosutinib (SKI-606), and combinations thereof.

In one aspect the present invention relates to a method described in [0006j], wherein steps (b) to (d) alternatively comprise:
(b') measuring a level of expression and/or activation of an oncogenic fusion protein and one or more signal transduction molecules in its pathway in the cellular extract; and
(c') comparing the measured level of expression and/or activation of the oncogenic fusion protein and signal transduction molecules to a level of expression and/or activation of the oncogenic fusion protein and signal transduction molecules measured at an earlier time during the course of therapy; and
(d') determining a subsequent dose of the course of therapy for the subject or whether a different course of therapy should be administered to the subject based upon the comparison from step (c').

In another aspect, the present invention relates to a method for selecting a suitable anticancer drug for the treatment of a cancer, or for identifying the response of a cancer to treatment with an anticancer drug, or for predicting the response of a subject having cancer to treatment with an anticancer drug, or for determining whether a subject having cancer is resistant to treatment with an anticancer drug, the method comprising:
(a) lysing isolated cancer cells from a subject after administration of an anticancer drug, or prior to incubation with an anticancer drug, to produce a cellular extract, wherein the isolated cancer cells are not washed prior to lysis;
(b) measuring a level of expression and/or activation of an oncogenic fusion protein in the cellular extract in accordance with the method of any one of claims 1 to 25; and
(c) determining whether the anticancer drug is suitable or unsuitable for the treatment of the cancer by comparing the level of expression and/or activation detected for the oncogenic fusion protein with a reference level and/or activation profile generated in the absence of the anticancer drug, identifying the cancer as responsive or non-responsive to treatment with the anticancer drug by comparing the level of expression and/or activation detected for the oncogenic fusion protein with a reference level and/or activation profile generated in the absence of the anticancer drug,
predicting the likelihood that the subject will respond to treatment with the anticancer drug by comparing the level of expression and/or activation detected for the oncogenic fusion protein with a reference level and/or activation profile generated in the absence of the anticancer drug, determining whether the subject is resistant or sensitive to treatment with the anticancer drug by comparing the level of expression and/or activation detected for the oncogenic fusion protein with a reference level and/or activation profile generated in the absence of the anticancer drug or in the presence of the anticancer drug at an earlier time, respectively.

In one embodiment, the present invention relates to a method as described in [0006l], wherein the cancer is chronic myelogenous leukemia
(CML), or wherein the anticancer drug is selected from the group consisting of a monoclonal antibody, tyrosine kinase inhibitor, chemotherapeutic agent, hormonal therapeutic agent, radiotherapeutic agent, vaccine, and combinations thereof, said monoclonal antibody optionally being selected from the group consisting of trastuzumab, alemtuzumab, bevacizumab, cetuximab, gemtuzumab, panitumumab, rituximab, tositumomab, and combinations thereof, said tyrosine kinase inhibitor optionally being selected from the group consisting of imatinib mesylate, nilotinib, dasatinib, bosutinib (SKI-606), gefitinib, sunitinib, erlotinib, lapatinib, canertinib (CI 1033), semaxinib (SU5416), vatalanib (PTK787/ZK222584), sorafenib (BAY 43-9006), leflunomide (SU101), vandetanib (ZD6474), and combinations thereof, said chemotherapeutic agent optionally being selected from the group consisting of pemetrexed, gemcitabine, sirolimus (rapamycin), rapamycin analogs, platinum compounds, carboplatin, cisplatin, satraplatin, paclitaxel, docetaxel (Taxotere®), temsirolimus (CCI-779), everolimus (RAD001), and combinations thereof, said hormonal therapeutic agent optionally being selected from the group consisting of aromatase inhibitors, selective estrogen receptor modulators, steroids, finasteride, gonadotropinreleasing hormone agonists, pharmaceutically acceptable salts thereof, stereoisomers thereof, derivatives thereof, analogs thereof, and combinations thereof, said radiotherapeutic agent optionally being selected from the group consisting of 47Sc, 64Cu, 67Cu, 89Sr, 86Y, 87Y, 90Y, 105Rh, 111Ag, 111In, 117mSn, 149Pm, 153Sm, 166Ho, 177Lu, 186Re, 188Re, 211At, 212Bi, and combinations thereof.

Other objects, features, and advantages of the present invention will be apparent to one of skill in the art from the following detailed description and figures.

### BRIEF DESCRIPTION OF THE DRAWINGS

Figure 1 shows one embodiment of the assay format of the present invention, which relies on the co-localization of two additional detector antibodies linked with enzymes for subsequent channeling events per each target oncogenic fusion protein bound.
Figure 2 shows schematically the application of the arrays of the invention for drug selection throughout the course of cancer treatment.
Figures 3 A-D show various embodiments of the assay format of the present invention for detecting expression and activation levels of oncogenic fusion proteins such as BCR-ABL.
Figure 4 shows the BCR-ABL signal in K562 cells after removal of free BCR.
Figure 5 shows the BCR signal in K562 cells after removal of free BCR.
Figure 6 shows the detection of total and phosphorylated levels of BCR-ABL in K562 cells.
Figure 7 shows the phosphorylation level ("Phospho BCR-ABL") and total amount ("Total BCR-ABL") of BCR-ABL detected in K562 human chronic myelogenous leukemia cells with or without depletion of free BCR using BCR C-terminal antibody-coupled beads.

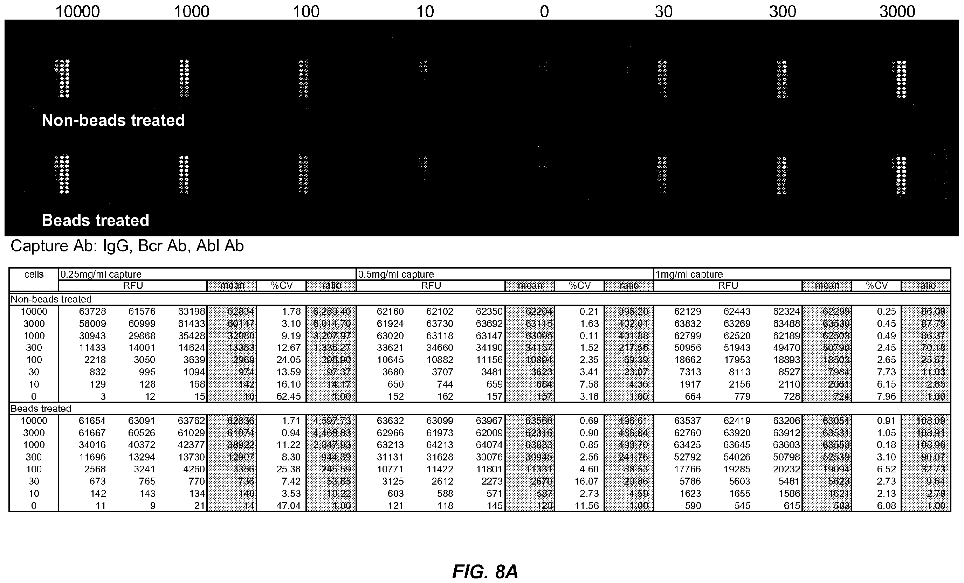

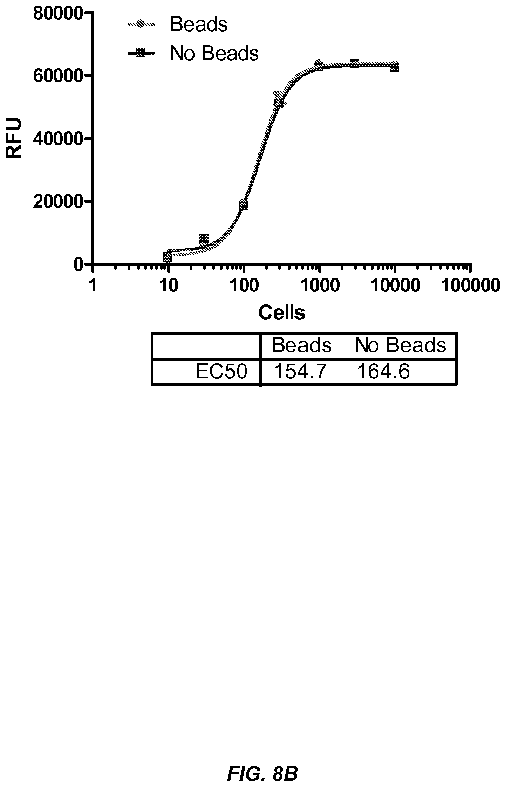

Figure 8 shows the phosphorylated BCR-ABL signal in K562 cells with or without removal of free BCR using BCR C-terminal antibody-coupled beads. In particular, Figure 8A provides a microarray comparison of the phosphorylated BCR-ABL signal detected in K562 cell lysates with or without removal of full-length BCR ("Non-beads treated" = BCR not removed versus "Beads treated" = BCR removed with beads containing an antibody specific for the C-terminus of full-length BCR conjugated thereto). Figure 8B provides a graphical depiction of the microarray data with Relative Fluorescence Units (RFU) as a function of cell number.

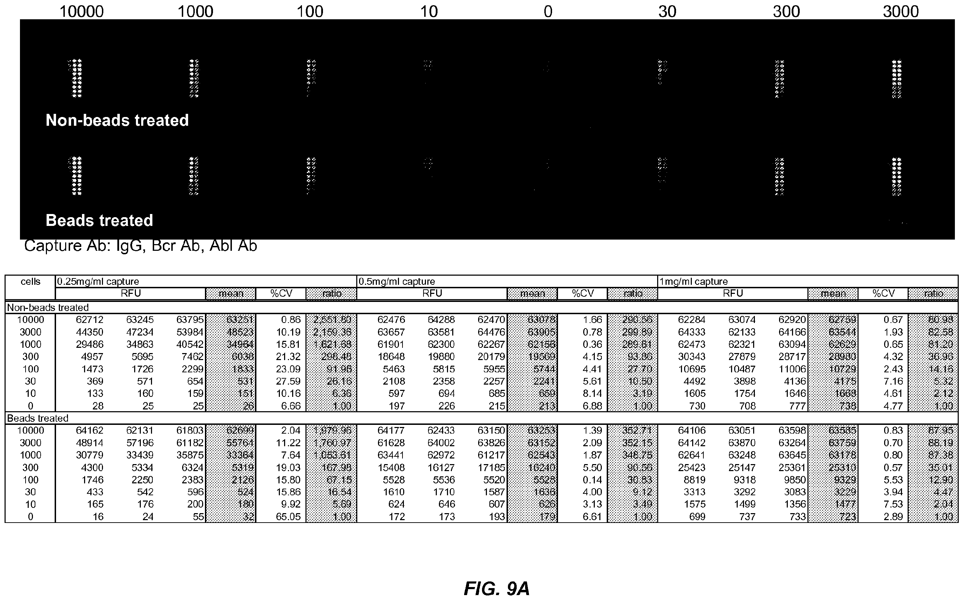

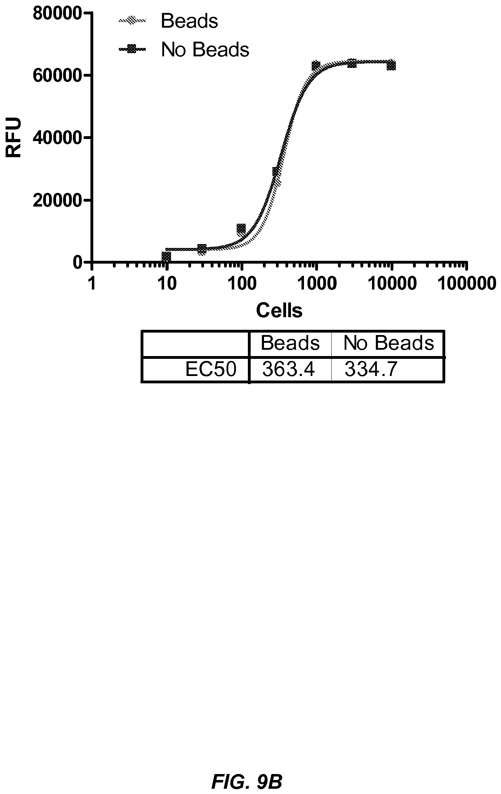

Figure 9 shows the total BCR-ABL signal in K562 cells with or without removal of free BCR using BCR C-terminal antibody-coupled beads. In particular, Figure 9A provides a microarray comparison of the total BCR-ABL signal detected in K562 cell lysates with or without removal of full-length BCR ("Non-beads treated" = BCR not removed versus "Beads treated" = BCR removed with beads containing an antibody specific for the C-terminus of full-length BCR conjugated thereto). Figure 9B provides a graphical depiction of the microarray data with Relative Fluorescence Units (RFU) as a function of cell number.

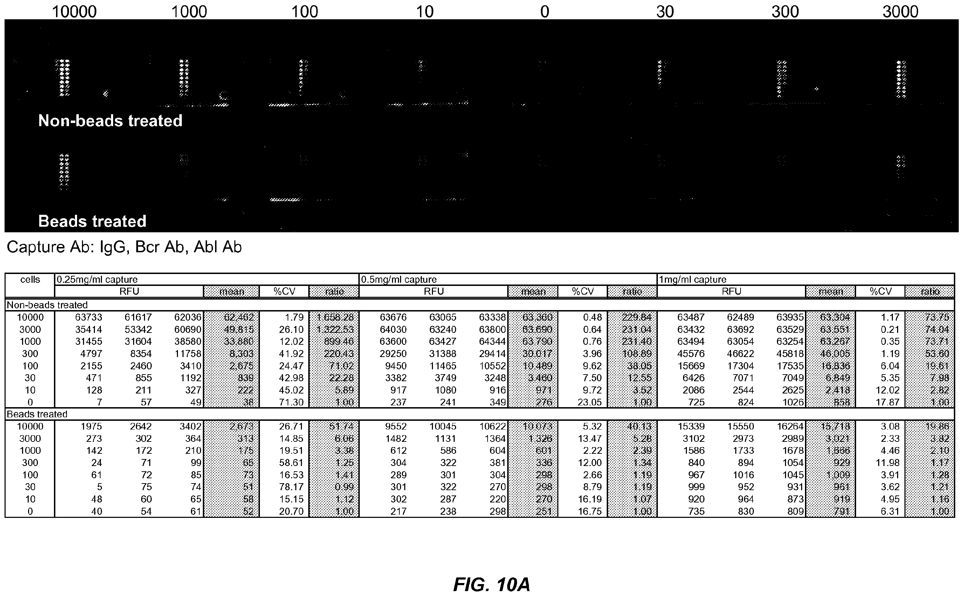

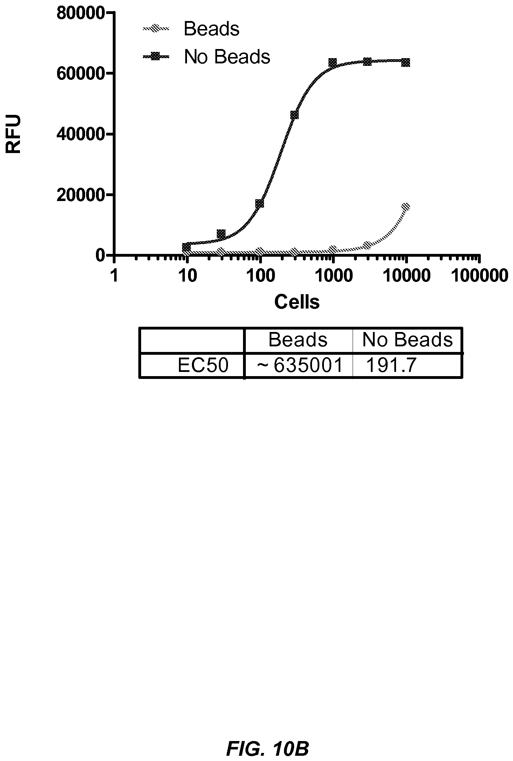

Figure 10 shows the removal of free full-length BCR from an extract of K562 cells after contacting the cellular extract with BCR C-terminal antibody-coupled beads. In particular, Figure 10A provides a microarray comparison of the total BCR signal detected in K562 cell lysates with or without removal of full-length BCR ("Non-beads treated" = BCR not removed versus "Beads treated" = BCR removed with beads containing an antibody specific for the C-terminus of full-length BCR conjugated thereto). Figure 10B provides a graphical depiction of the microarray data with Relative Fluorescence Units (RFU) as a function of cell number.
Figure 11 shows that free full-length BCR and ABL proteins, but not BCR-ABL fusion protein, are present in white blood cells (WBCs).
Figure 12 shows that the free full-length BCR present in WBCs inhibited the phospho BCR-ABL signal in K562 cell extracts when such K562 cell extracts were spiked with WBC extracts.
Figure 13 shows the total BCR-ABL signal in K562 cells spiked with WBC extracts after removal of free BCR using BCR C-terminal antibody-coupled beads. In particular, Figure 13A shows that the free BCR signal was saturated when the K562 cell extracts were spiked with WBC extracts. After treatment with BCR C-terminal antibody-coupled beads, the free BCR was removed. Figure 13B shows that the BCR-ABL signal was not changed with or without beads treatment in the same experiment.
Figure 14 shows that the BCR-ABL inhibitor imatinib (Gleevec^{®}) dose-dependently inhibited activation (*i.e.*, phosphorylation), but not expression (*i.e.*, total levels), of BCR-ABL protein in K562 cells.
Figure 15 shows that the BCR-ABL inhibitor nilotinib (Tasigna^{®}) dose-dependently inhibited activation (*i.e.*, phosphorylation), but not expression (*i.e.*, total levels), of BCR-ABL protein in K562 cells.
Figure 16 shows that the BCR-ABL inhibitor dasatinib (Sprycel^{®}) dose-dependently inhibited activation (*i.e.*, phosphorylation), but not expression (*i.e.*, total levels), of BCR-ABL protein in K562 cells.
Figure 17 shows that CRKL is both present and activated (*i.e.*, phosphorylated) in K562 cells.
Figure 18 shows that CRKL is present in A431 human epidermoid carcinoma cells and is activated (*i.e.*, phosphorylated) upon EGF treatment.
Figure 19 shows that CRKL is present in T47D human ductal breast epithelial tumor cells but is not activated (*i.e.*, phosphorylated) upon EGF treatment.
Figure 20 shows that CRKL is present in T47D cells and is activated (*i.e*., phosphorylated) at low levels upon heregulin (HRG) treatment.
Figure 21 shows that CRKL is present in MCF-7 human breast adenocarcinoma cells and is activated (*i.e.*, phosphorylated) at low levels upon heregulin (HRG) treatment.
Figure 22 illustrates the presence of activated (*i.e.*, phosphorylated) CRKL in the white blood cells (WBCs) of different donors.
Figure 23 illustrates that JAK2 is activated (*i.e.*, phosphorylated) in K562 cells and A431 cells.
Figure 24 illustrates that phosphorylated BCR-ABL can be detected and measured in cell lysates prepared from K562 cells isolated from blood using anti-CD45 magnetic beads.
Figure 25 illustrates that total BCR-ABL levels were not changed when an antibody directed to the C-terminus of native full-length BCR (which C-terminal domain is not present in BCR-ABL) was spotted on the same slide in the same pad as an antibody directed to the N-terminal region of BCR-ABL.
Figure 26 illustrates that the free native BCR signal detected with an N-terminal-specific BCR antibody was reduced when an antibody directed to the C-terminus of native BCR was spotted on the same slide in the same pad.

### DETAILED DESCRIPTION OF THE INVENTION

### I. Introduction

Hematological malignancies are the types of cancer that affect blood, bone marrow, and lymph nodes. As the three are intimately connected through the immune system, a disease affecting one of the three will often affect the others as well. For example, although lymphoma is technically a disease of the lymph nodes, it often spreads to the bone marrow and the blood. Chromosomal translocations, which create fusion proteins having novel coding sequences containing parts of the coding sequences from two different genes, are a common cause of these diseases, but are a less common cause of solid tumors. As such, it is important to identify the presence and/or activity of oncogenic fusion proteins associated with hematological malignancies in order to provide the appropriate prognosis and treatment for patients with these types of cancer.

For example, the BCR-ABL fusion protein is associated with chronic myelogenous leukemia (CML) as well as acute lymphoblastic leukemia (ALL). In particular, the BCR-ABL protein is an active tyrosine kinase that is critical to cancer pathogenesis. Although imatinib (Gleevec^{®}) is currently the first line therapy for newly diagnosed patients with CML, about 20-25% of patients do not achieve durable complete cytogenetic responses. Studies have shown that the reactivation of BCR-ABL kinase activity in the presence of continued imatinib treatment is the major cause of resistance. As such, the measurement of BCR-ABL activity finds utility in predicting response to therapy with tyrosine kinase inhibitors such as imatinib as well as in identifying patients who develop resistance to such inhibitors.

The present invention provides methods for detecting the activation state and/or total amount of one or a plurality of fusion proteins (alone or in combination with one or a plurality of signal transduction molecules) in isolated cells using an antibody-based array assay system. Cellular extracts prepared from isolated leukocytes, circulating cells, or other cell types are particularly useful in the methods described herein. In some embodiments, the multiplex, high-throughput immunoassays of the present invention can detect the activation state of one or more oncogenic fusion proteins and/or signal transduction molecules at the single cell level. In fact, signal transduction molecules such as EGFR can be detected with a sensitivity of about 100 zeptomoles and a linear dynamic range of from about 100 zeptomoles to about 100 femtomoles. As such, single-cell detection of the activation state of one or more oncogenic fusion proteins and/or signal transduction molecules facilitates cancer prognosis and diagnosis as well as the design of personalized, targeted therapies.

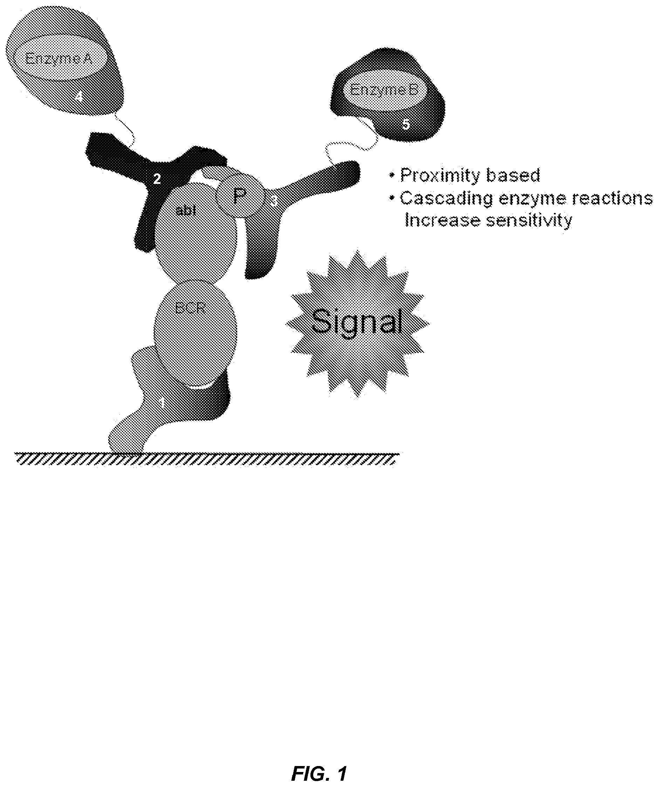

Figure 1 illustrates an exemplary proximity dual detection assay of the present invention in which an oncogenic fusion protein such as BCR-ABL is bound to a capture antibody and two detection antibodies (*i.e.*, an activation state-independent antibody and an activation state-dependent antibody). The capture antibody **1** binds the BCR portion of the fusion protein independent of its activation state. While the activation state-independent antibody **2** binds the ABL portion of the fusion protein independent of its activation state, the activation state-dependent antibody **3** binds the ABL portion of the fusion protein dependent of its activation state (*e.g.*, the activation state-dependent antibody will only bind an activated form of BCR-ABL having a phosphorylated residue). The activation state-independent antibody is labeled with a facilitating moiety **4** ("Enzyme A") and the activation state-dependent antibody is labeled with a first member of a signal amplification pair **5** ("Enzyme B"). Binding of both detection antibodies to the ABL portion of the fusion protein brings the facilitating moiety within sufficient proximity to the first member of the signal amplification pair such that a signal generated by the facilitating moiety can channel to the first member of the signal amplification pair, resulting in the generation of a detectable and/or amplifiable signal. Various methods for proximity channeling are described herein and are also known in the art including, but not limited to, FRET, time-resolved fluorescence-FRET, LOCI, *etc.* An advantage of proximity channeling, as used in the methods of the present invention, is that a single detectable signal is generated for only those analytes (*e.g.,* fusion proteins or signal transduction molecules) that have bound all three antibodies, resulting in increased assay specificity, lower background, and simplified detection.

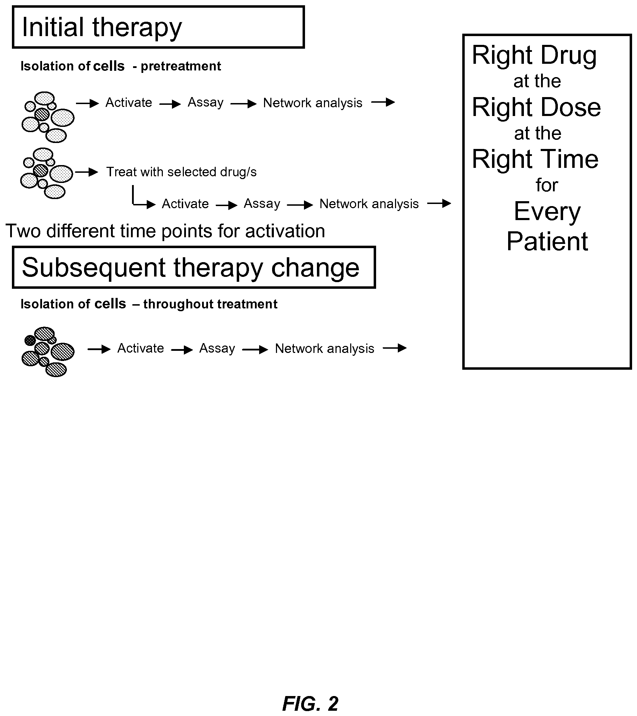

As explained in greater detail herein, to evaluate potential anticancer therapies for an individual patient, the isolated cells can be incubated with one or more anticancer drugs at varying doses. Growth factor stimulation can then be performed for a few minutes (*e.g.,* about 1-5 minutes) or for several hours (*e.g.,* about 1-6 hours). The differential activation of signaling pathways with and without anticancer drugs can aid in the selection of a suitable cancer therapy at the proper dose for each individual patent. Cells can also be isolated from a patient during anticancer drug treatment and stimulated with one or more growth factors to determine whether a change in therapy should be implemented. As such, Figure 2 shows that the methods of the present invention advantageously assist the clinician in providing the right anticancer drug at the right dose at the right time for every patient.

### II. Definitions

As used herein, the following terms have the meanings ascribed to them unless specified otherwise.

The term "cancer" includes any member of a class of diseases characterized by the uncontrolled growth of aberrant cells. The term includes all known cancers and neoplastic conditions, whether characterized as malignant, benign, soft tissue, or solid, and cancers of all stages and grades including pre- and post-metastatic cancers. Non-limiting examples of different types of cancer include hematological malignancies (*e.g.,* leukemia, lymphoma); osteogenic sarcomas (*e.g.,* Ewing sarcoma); soft tissue sarcomas (*e.g.,* Dermatofibrosarcoma Protuberans (DFSP), rhabdomyosarcoma); other soft tissue malignancies, papillary thyroid carcinomas; prostate cancer; gastric cancer (*e.g.*, stomach); breast cancer; lung cancer (*e.g.*, non-small cell lung cancer); digestive and gastrointestinal cancers (*e.g.*, colorectal cancer, gastrointestinal stromal tumors, gastrointestinal carcinoid tumors, colon cancer, rectal cancer, anal cancer, bile duct cancer, and small intestine cancer); esophageal cancer; gallbladder cancer; liver cancer; pancreatic cancer; appendix cancer; ovarian cancer; renal cancer (*e.g.,* renal cell carcinoma); cancer of the central nervous system; skin cancer; choriocarcinomas; and head and neck cancers. As used herein, a "tumor" comprises one or more cancerous cells.

A "hematological malignancy" includes any type of cancer that affects the blood, bone marrow, and/or lymph nodes. Examples of hematological malignancies include, but are not limited to, leukemia, lymphoma, and multiple myeloma. Non-limiting examples of different kinds of leukemia include chronic myelogenous leukemia (CML), acute lymphoblastic leukemia (ALL), chronic lymphocytic leukemia (CLL), acute myelogenous leukemia (AML), and large granular lymphocytic leukemia. Subtypes of CML include, *e.g.*, chronic monocytic leukemia. Subtypes of ALL include, *e.g.,* precursor B-cell acute lymphoblastic leukemia, pro-B-cell acute lymphoblastic leukemia, precursor T-cell acute lymphoblastic leukemia, and acute biphenotypic leukemia. Subtypes of CLL include, *e.g.*, B-cell prolymphocytic leukemia. Subtypes of AML include, *e.g.,* acute promyelocytic leukemia, acute myeloblastic leukemia, and acute megakaryoblastic leukemia. Examples of different kinds of lymphoma include, but are not limited to, Hodgkin's lymphoma (four subtypes) and non-Hodgkin lymphoma, such as, *e.g.,* small lymphocytic lymphoma (SLL), diffuse large B-cell lymphoma (DLBCL), follicular lymphoma (FL), mantle cell lymphoma (MCL), hairy cell leukemia (HCL), marginal zone lymphoma (MZL), Burkitt's lymphoma (BL), post-transplant lymphoproliferative disorder (PTLD), T-cell prolymphocytic leukemia (T-PLL), B-cell prolymphocytic leukemia (B-PLL), Waldenström's macroglobulinemia (also known as lymphoplasmacytic lymphoma), and other NK- or T-cell lymphomas.

The term "analyte" includes any molecule of interest, typically a macromolecule such as a polypeptide, whose presence, amount, and/or identity is determined. In certain instances, the analyte is a cellular component of a cancerous cell, preferably an oncogenic fusion protein or a signal transduction molecule.

The term "transform" or "transforming" includes a physical and/or chemical change of an analyte or sample to extract the analyte or to change or modify the analyte as defined herein. As used herein, an extraction, a manipulation, a chemical precipitation, an ELISA, a complexation, an immuno-extraction, a physical or chemical modification of the analyte or sample to measure a level or concentration or activation state of an analyte all constitute a transformation. In other words, as long as the analyte or sample is not identical before and after the transformation step, the change or modification is a transformation.

As used herein, the term "dilution series" is intended to include a series of descending concentrations of a particular sample (*e.g.,* cell lysate) or reagent (*e.g.,* antibody). A dilution series is typically produced by a process of mixing a measured amount of a starting concentration of a sample or reagent with a diluent (*e.g.,* dilution buffer) to create a lower concentration of the sample or reagent, and repeating the process enough times to obtain the desired number of serial dilutions. The sample or reagent can be serially diluted at least 2, 3, 4, 5, 6, 7, 8, 9, 10, 15, 20, 25, 30, 35, 40, 45, 50, 100, 500, or 1000-fold to produce a dilution series comprising at least 2, 3, 4, 5, 6, 7, 8, 9, 10, 11, 12, 13, 14, 15, 16, 17, 18, 19, 20, 25, 30, 35, 40, 45, or 50 descending concentrations of the sample or reagent. For example, a dilution series comprising a 2-fold serial dilution of a capture antibody reagent at a 1 mg/ml starting concentration can be produced by mixing an amount of the starting concentration of capture antibody with an equal amount of a dilution buffer to create a 0.5 mg/ml concentration of the capture antibody, and repeating the process to obtain capture antibody concentrations of 0.25 mg/ml, 0.125 mg/ml, 0.0625 mg/ml, 0.0325 mg/ml, *etc.*

The term "superior dynamic range" as used herein refers to the ability of an assay to detect a specific analyte in as few as one cell or in as many as thousands of cells. For example, the immunoassays described herein possess superior dynamic range because they advantageously detect a particular oncogenic fusion protein or signal transduction molecule of interest in about 1-10,000 cells (*e.g.,* about 1, 5, 10, 25, 50, 75, 100, 250, 500, 750, 1000, 2500, 5000, 7500, or 10,000 cells) using a dilution series of capture antibody concentrations.

The term "fusion protein" or "chimeric protein" includes a protein created through the joining of two or more genes which originally encode separate proteins. Such gene fusions are typically generated when a chromosomal translocation replaces the terminal exons of one gene with intact exons from a second gene. This creates a single gene which can be transcribed, spliced, and translated to produce a functional fusion protein. In particular embodiments, the fusion protein is an oncogenic fusion protein, *i.e.*, a fusion protein involved in oncogenesis. Examples of oncogenic fusion proteins include, but are not limited to, BCR-ABL, DEK-CAN, E2A-PBX1, RARa-PML, IREL-URG, CBFβ-MYH11, AML1-MTG8, EWS-FLI, LYT-10-Cα1, HRX-ENL, HRX-AF4, NPM-ALK, IGH-MYC, RUNX1-ETO, TEL-TRKC, TEL-AML1, MLL-AF4, TCR-RBTN2, COL1A1-PDGF, E2A-HLF, PAX3-FKHR, ETV6-NTRK3, RET-PTC, TMRSS-ERG, and TPR-MET.

The term "signal transduction molecule" or "signal transducer" includes proteins and other molecules that carry out the process by which a cell converts an extracellular signal or stimulus into a response, typically involving ordered sequences of biochemical reactions inside the cell. Examples of signal transduction molecules include, but are not limited to, receptor tyrosine kinases such as EGFR (*e.g.,* EGFR/HER-1/ErbB1, HER-2/Neu/ErbB2, HER-3/ErbB3, HER-4/ErbB4), VEGFR-1/FLT-1, VEGFR-2/FLK-1/KDR, VEGFR-3/FLT-4, FLT-3/FLK-2, PDGFR (*e.g.,* PDGFRA, PDGFRB), c-Met, c-KIT/SCFR, INSR (insulin receptor), IGF-IR, IGF-IIR, IRR (insulin receptor-related receptor), CSF-1R, FGFR 1-4, HGFR 1-2, CCK4, TRK A-C, MET, RON, EPHA 1-8, EPHB 1-6, AXL, MER, TYRO3, TIE 1-2, TEK, RYK, DDR 1-2, RET, c-ROS, V-cadherin, LTK (leukocyte tyrosine kinase), ALK (anaplastic lymphoma kinase), ROR 1-2, MUSK, AATYK 1-3, RTK 106, and truncated forms of the receptor tyrosine kinases such as p95ErbB2; non-receptor tyrosine kinases such as Src, Frk, Btk, Csk, Abl, Zap70, Fes/Fps, Fak, Jak, Ack, and LIMK; tyrosine kinase signaling cascade components such as Akt, MAPK/ERK, MEK, RAF, PLA2, MEKK, JNKK, JNK, p38, Shc (p66), PI3K, Ras (*e.g.,* K-Ras, N-Ras, H-Ras), Rho, Rac1, Cdc42, PLC, PKC, p70 S6 kinase, p53, cyclin D1, STAT1, STAT3, PIP2, PIP3, PDK, mTOR, BAD, p21, p27, ROCK, IP3, TSP-1, NOS, PTEN, RSK 1-3, JNK, c-Jun, Rb, CREB, Ki67, and paxillin; nuclear hormone receptors such as estrogen receptor (ER), progesterone receptor (PR), androgen receptor, glucocorticoid receptor, mineralocorticoid receptor, vitamin A receptor, vitamin D receptor, retinoid receptor, thyroid hormone receptor, and orphan receptors; nuclear receptor coactivators and repressors; and combinations thereof.

The term "sample" as used herein includes any biological specimen obtained from a patient. Samples include, without limitation, whole blood, plasma, serum, ductal lavage fluid, nipple aspirate, lymph (*e.g.,* disseminated tumor cells of the lymph node), bone marrow aspirate, saliva, urine, stool (*i.e.*, feces), sputum, bronchial lavage fluid, tears, fine needle aspirate (*e.g.,* harvested by random periareolar fine needle aspiration), any other bodily fluid, a tissue sample (*e.g.,* tumor tissue) such as a biopsy of a tumor (*e.g.,* needle biopsy) or a lymph node (*e.g.,* sentinel lymph node biopsy), and cellular extracts thereof In some embodiments, the sample is whole blood or a fractional component thereof such as plasma, serum, red blood cells, leukocytes such as peripheral blood mononuclear cells, and/or rare circulating cells. In particular embodiments, the sample is obtained by isolating leukocytes or circulating cells of a solid tumor from whole blood or a cellular fraction thereof using any technique known in the art. In other embodiments, the sample is a formalin fixed paraffin embedded (FFPE) tumor tissue sample, *e.g.,* from a solid tumor.

As used herein, the term "circulating cells" comprises extratumoral cells that have either metastasized or micrometastasized from a solid tumor. Examples of circulating cells include, but are not limited to, circulating tumor cells, cancer stem cells, and/or cells that are migrating to the tumor (*e.g.,* circulating endothelial progenitor cells, circulating endothelial cells, circulating pro-angiogenic myeloid cells, circulating dendritic cells, *etc.*).

A "biopsy" refers to the process of removing a tissue sample for diagnostic or prognostic evaluation, and to the tissue specimen itself. Any biopsy technique known in the art can be applied to the methods and compositions of the present invention. The biopsy technique applied will generally depend on the tissue type to be evaluated and the size and type of the tumor (*i.e.*, solid or suspended (*i.e.*, blood or ascites)), among other factors. Representative biopsy techniques include excisional biopsy, incisional biopsy, needle biopsy (*e.g.,* core needle biopsy, fine-needle aspiration biopsy, *etc.*), surgical biopsy, and bone marrow biopsy. Biopsy techniques are discussed, for example, in *Harrison's Principles of Internal Medicine*, Kasper, *et al.*, eds., 16th ed., 2005, Chapter 70, and throughout Part V. One skilled in the art will appreciate that biopsy techniques can be performed to identify cancerous and/or precancerous cells in a given tissue sample.

The term "subject" or "patient" or "individual" typically includes humans, but can also include other animals such as, *e.g*., other primates, rodents, canines, felines, equines, ovines, porcines, and the like.

An "array" or "microarray" comprises a distinct set and/or dilution series of capture antibodies immobilized or restrained on a solid support such as, for example, glass (*e.g.,* a glass slide), plastic, chips, pins, filters, beads (*e.g.,* magnetic beads, polystyrene beads, *etc.*), paper, membrane (*e.g.,* nylon, nitrocellulose, polyvinylidene fluoride (PVDF), *etc.*), fiber bundles, or any other suitable substrate. The capture antibodies are generally immobilized or restrained on the solid support via covalent or noncovalent interactions (*e.g.,* ionic bonds, hydrophobic interactions, hydrogen bonds, Van der Waals forces, dipole-dipole bonds). In certain instances, the capture antibodies comprise capture tags which interact with capture agents bound to the solid support. The arrays used in the assays of the present invention typically comprise a plurality of different capture antibodies and/or capture antibody concentrations that are coupled to the surface of a solid support in different known/addressable locations.

The term "capture antibody" is intended to include an immobilized antibody which is specific for (*i.e.*, binds, is bound by, or forms a complex with) one or more analytes of interest in a sample such as a cellular extract of leukocytes or rare circulating cells. In preferred embodiments, the capture antibody is restrained on a solid support in an array. Suitable capture antibodies for immobilizing any of a variety of oncogenic fusion proteins or signal transduction molecules on a solid support are available from Upstate (Temecula, CA), Biosource (Camarillo, CA), Cell Signaling Technologies (Danvers, MA), R&D Systems (Minneapolis, MN), Lab Vision (Fremont, CA), Santa Cruz Biotechnology (Santa Cruz, CA), Sigma (St. Louis, MO), and BD Biosciences (San Jose, CA).

The term "detection antibody" as used herein includes an antibody comprising a detectable label which is specific for (*i.e.*, binds, is bound by, or forms a complex with) one or more analytes of interest in a sample. The term also encompasses an antibody which is specific for one or more analytes of interest, wherein the antibody can be bound by another species that comprises a detectable label. Examples of detectable labels include, but are not limited to, biotin/streptavidin labels, nucleic acid (*e.g.,* oligonucleotide) labels, chemically reactive labels, fluorescent labels, enzyme labels, radioactive labels, and combinations thereof. Suitable detection antibodies for detecting the activation state and/or total amount of any of a variety of oncogenic fusion proteins or signal transduction molecules are available from Upstate (Temecula, CA), Biosource (Camarillo, CA), Cell Signaling Technologies (Danvers, MA), R&D Systems (Minneapolis, MN), Lab Vision (Fremont, CA), Santa Cruz Biotechnology (Santa Cruz, CA), Sigma (St. Louis, MO), and BD Biosciences (San Jose, CA). As a non-limiting example, phospho-specific antibodies against various phosphorylated forms of signal transduction molecules such as EGFR, c-KIT, c-Src, FLK-1, PDGFRA, PDGFRB, Akt, MAPK, PTEN, Raf, and MEK are available from Santa Cruz Biotechnology.

The term "activation state-dependent antibody" includes a detection antibody which is specific for (*i.e.*, binds, is bound by, or forms a complex with) a particular activation state of one or more analytes of interest in a sample. In preferred embodiments, the activation state-dependent antibody detects the phosphorylation, ubiquitination, and/or complexation state of one or more analytes such as one or more oncogenic fusion proteins or signal transduction molecules. In some embodiments, the phosphorylation of the ABL kinase domain of the BCR-ABL fusion protein is detected using an activation state-dependent antibody. In other embodiments, the phosphorylation of members of the EGFR family of receptor tyrosine kinases and/or the formation of heterodimeric complexes between EGFR family members is detected using activation state-dependent antibodies.

Non-limiting examples of activation states of oncogenic fusion proteins that are suitable for detection with activation state-dependent antibodies include phosphorylated forms of BCR-ABL, DEK-CAN, E2A-PBX1, RARa-PML, IREL-URG, CBFβ-MYH11, AML1-MTG8, EWS-FLI, LYT-10-Cα1, HRX-ENL, HRX-AF4, NPM-ALK, IGH-MYC, RUNX1-ETO, TEL-TRKC, TEL-AML1, MLL-AF4, TCR-RBTN2, COL1A1-PDGF, E2A-HLF, PAX3-FKHR, ETV6-NTRK3, RET-PTC, TMRSS-ERG, and TPR-MET. Examples of activation states (listed in parentheses) of signal transduction molecules that are suitable for detection with activation state-dependent antibodies include, but are not limited to, EGFR (EGFRvIII, phosphorylated (p-) EGFR, EGFR:Shc, ubiquitinated (u-) EGFR, p-EGFRvIII); ErbB2 (p95:truncated (Tr)-ErbB2, p-ErbB2, p95:Tr-p-ErbB2, HER-2:Shc, ErbB2:PI3K, ErbB2:EGFR, ErbB2:ErbB3, ErbB2:ErbB4); ErbB3 (p-ErbB3, ErbB3:PI3K, p-ErbB3:PI3K, ErbB3:Shc); ErbB4 (p-ErbB4, ErbB4:Shc); c-Met (p-c-Met or c-Met/HGF complex), ER (pER (S118, S167); IGF-1R (p-IGF-1R, IGF-1R:IRS, IRS:PI3K, p-IRS, IGF-1R:PI3K); INSR (p-INSR); KIT (p-KIT); FLT3 (p-FLT3); HGFRI (p-HGFRI); HGFR2 (p-HGFR2); RET (p-RET); PDGFRa (p-PDGFRa); PDGFRP (p-PDGFRP); VEGFRI (p-VEGFRI, VEGFRI:PLCg, VEGFR1:Src); VEGFR2 (p-VEGFR2, VEGFR2:PLCy, VEGFR2:Src, VEGFR2:heparin sulfate, VEGFR2:VE-cadherin); VEGFR3 (p-VEGFR3); FGFR1 (p-FGFR1); FGFR2 (p-FGFR2); FGFR3 (p-FGFR3); FGFR4 (p-FGFR4); Tie1 (p-Tie1); Tie2 (p-Tie2); EphA (p-EphA); EphB (p-EphB); NFKB and/or IKB (p-IK (S32), p-NFKB (S536), p-P65:IKBa); Akt (p-Akt (T308, S473)); PTEN (p-PTEN); Bad (p-Bad (S112, S136), Bad:14-3-3); mTor (p-mTor (S2448)); p70S6K (p-p70S6K (T229, T389)); Mek (p-Mek (S217, S221)); Erk (p-Erk (T202, Y204)); Rsk-1 (p-Rsk-1 (T357, S363)); Jnk (p-Jnk (T183, Y185)); P38 (p-P38 (T180, Y182)); Stat3 (p-Stat-3 (Y705, S727)); Fak (p-Fak (Y576)); Rb (p-Rb (S249, T252, S780)); Ki67; p53 (p-p53 (S392, S20)); CREB (p-CREB (S133)); c-Jun (p-c-Jun (S63)); cSrc (p-cSrc (Y416)); and paxillin (p-paxillin (Y118)).

The term "activation state-independent antibody" includes a detection antibody which is specific for (*i.e.*, binds, is bound by, or forms a complex with) one or more analytes of interest in a sample irrespective of their activation state. For example, the activation state-independent antibody can detect both phosphorylated and unphosphorylated forms of one or more analytes such as one or more oncogenic fusion proteins or signal transduction molecules.

The term "nucleic acid" or "polynucleotide" includes deoxyribonucleotides or ribonucleotides and polymers thereof in either single- or double-stranded form such as, for example, DNA and RNA. Nucleic acids include nucleic acids containing known nucleotide analogs or modified backbone residues or linkages, which are synthetic, naturally occurring, and non-naturally occurring, and which have similar binding properties as the reference nucleic acid. Examples of such analogs include, without limitation, phosphorothioates, phosphoramidates, methyl phosphonates, chiral-methyl phosphonates, 2'-O-methyl ribonucleotides, and peptide-nucleic acids (PNAs). Unless specifically limited, the term encompasses nucleic acids containing known analogues of natural nucleotides that have similar binding properties as the reference nucleic acid. Unless otherwise indicated, a particular nucleic acid sequence also implicitly encompasses conservatively modified variants thereof and complementary sequences as well as the sequence explicitly indicated.

The term "oligonucleotide" refers to a single-stranded oligomer or polymer of RNA, DNA, RNA/DNA hybrid, and/or a mimetic thereof. In certain instances, oligonucleotides are composed of naturally-occurring (*i.e.*, unmodified) nucleobases, sugars, and internucleoside (backbone) linkages. In certain other instances, oligonucleotides comprise modified nucleobases, sugars, and/or internucleoside linkages.

As used herein, the term "mismatch motif" or "mismatch region" refers to a portion of an oligonucleotide that does not have 100% complementarity to its complementary sequence. An oligonucleotide may have at least one, two, three, four, five, six, or more mismatch regions. The mismatch regions may be contiguous or may be separated by 1, 2, 3, 4, 5, 6, 7, 8, 9, 10, 11, 12, or more nucleotides. The mismatch motifs or regions may comprise a single nucleotide or may comprise two, three, four, five, or more nucleotides.

The phrase "stringent hybridization conditions" refers to conditions under which an oligonucleotide will hybridize to its complementary sequence, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions may also be achieved with the addition of destabilizing agents such as formamide. For selective or specific hybridization, a positive signal is at least two times background, preferably 10 times background hybridization.

The terms "substantially identical" or "substantial identity," in the context of two or more nucleic acids, refer to two or more sequences or subsequences that are the same or have a specified percentage of nucleotides that are the same (*i.e.*, at least about 60%, preferably at least about 65%, 70%, 75%, 80%, 85%, 90%, or 95% identity over a specified region) when compared and aligned for maximum correspondence over a comparison window or designated region as measured using a sequence comparison algorithm or by manual alignment and visual inspection. This definition, when the context indicates, also refers analogously to the complement of a sequence. Preferably, the substantial identity exists over a region that is at least about 5, 10, 15, 20, 25, 30, 35, 40, 45, 50, 75, or 100 nucleotides in length.

The term "tyrosine kinase inhibitor" includes any of a variety of therapeutic agents or drugs that act as selective or non-selective inhibitors of receptor and/or non-receptor tyrosine kinases. Without being bound to any particular theory, tyrosine kinase inhibitors generally inhibit target tyrosine kinases by binding to the ATP-binding site of the enzyme. Examples of tyrosine kinase inhibitors include, but are not limited to, imatinib (Gleevec^{®}; STI571), nilotinib (Tasigna^{®}), dasatinib (Sprycel^{®}), bosutinib (SKI-606), gefitinib (Iressa^{®}), sunitinib (Sutent^{®}; SU11248), erlotinib (Tarceva^{®}; OSI-1774), lapatinib (GW572016; GW2016), canertinib (CI 1033), semaxinib (SU5416), vatalanib (PTK787/ZK222584), sorafenib (BAY 43-9006), leflunomide (SU101), vandetanib (Zactima™; ZD6474), derivatives thereof, analogs thereof, and combinations thereof. Additional tyrosine kinase inhibitors suitable for use in the present invention are described in, *e.g.,* U.S. Patent Nos. 5,618,829, 5,639,757, 5,728,868, 5,804,396, 6,100,254, 6,127,374, 6,245,759, 6,306,874, 6,313,138, 6,316,444, 6,329,380, 6,344,459, 6,420,382, 6,479,512, 6,498,165, 6,544,988, 6,562,818, 6,586,423, 6,586,424, 6,740,665, 6,794,393, 6,875,767, 6,927,293, and 6,958,340. One of skill in the art will know of other tyrosine kinase inhibitors suitable for use in the present invention. In certain instances, the tyrosine kinase inhibitor is administered in a pharmaceutically acceptable form including, without limitation, an alkali or alkaline earth metal salt such as an aluminum, calcium, lithium, magnesium, potassium, sodium, or zinc salt; an ammonium salt such as a tertiary amine or quaternary ammonium salt; and an acid salt such as a succinate, tartarate, bitartarate, dihydrochloride, salicylate, hemisuccinate, citrate, isocitrate, malate, maleate, mesylate, hydrochloride, hydrobromide, phosphate, acetate, carbamate, sulfate, nitrate, formate, lactate, gluconate, glucuronate, pyruvate, oxalacetate, fumarate, propionate, aspartate, glutamate, or benzoate salt.

The term "incubating" is used synonymously with "contacting" and "exposing" and does not imply any specific time or temperature requirements unless otherwise indicated.

The terms "complete cytogenetic response", "complete cytogenetic remission", "CCyR" and "CCgR" include the clinically accepted criteria defined to be the absence of Philadelphia chromosome-positive cells in metaphase among a population of at least 20 cells in metaphase, as determined by chromosome banding of cells isolated from bone marrow. In certain instances when metaphase cells isolated from bone marrow cannot be obtained or evaluated by chromosome banding, the term may be defined as the presence of < 1 % BCR-ABL positive nuclei from that of at least 200 nuclei scored as determined by interphase fluorescent in situ hybridization (FISH) of blood cells. The interphase FISH may be performed, *e.g.,* with BCR-ABL extrasignal, dual color, dual fusion, or in situ hybridization probes. For additional descriptions of these terms, *see, e.g.,* O'Brien et al., N. Engl. J. Med., 348:994-1004 (2003); Hughes et al., N. Eng.l J. Med., 349:1423-1432 (2003); and Bacarani et al., J. Clin. Oncol., 27:6041-6051 (2009).

A "major molecular response", "major molecular remission" or "MMR" is achieved when the level of an oncogenic fusion protein such as BCR-ABL decreases by at least about 2-3 logs over one or more control protein levels such as full-length BCR and/or full-length ABL. In certain embodiments, a major molecular response is achieved when the ratio of oncogenic fusion protein levels (*e.g.,* BCR-ABL levels) to control protein levels (*e.g.,* BCR or ABL levels) during anticancer drug (*e.g.,* tyrosine kinase inhibitor) therapy is reduced by at least about 2-3 logs relative to the ratio of the same proteins prior to anticancer drug therapy. In contrast to definitions of major molecular response which rely on the detection of mRNA transcript levels, the antibody-based proximity dual detection assays of the present invention advantageously provide the ability to detect one cancer (*e.g.,* CML) cell in the background of about 100,000 cells from a healthy donor, thereby enabling the determination of a major molecular response by measuring and comparing BCR-ABL, BCR, and/or ABL protein levels. In other embodiments, a major molecular response is a clinical classification defined as a reduction of at least 3-logs below a standardized baseline value on a logarithmic (base 10) scale in the ratio of BCR-ABL mRNA transcripts to either ABL or BCR mRNA transcripts expressed as a percentage of ABL or BCR mRNA transcript levels. In certain instances, the level of mRNA transcripts is determined using real-time quantitative RT-PCR (qPCR) methods. In further embodiments, a major molecular response is the condition when the ratio of BCR-ABL to ABL, BCR or control mRNA transcripts is determined by qPCR to be a value defined to be ≤ 0.1% on the international scale (IS). The IS is anchored to the log reduction scale established by the participating laboratories in the International Randomized Study of Interferon versus STI571 (IRIS) clinical study. *See, e.g.,* Hughes et al., N. Engl. J. Med., 349:1423-1432 (2003); Hughes et al., Blood, 108:28-37 (2006), Brand et al., Blood, 112:3330-3338 (2008); and Baccarani et al., J. Clin. Oncol., 27:6041-6051 (2009).

A "complete molecular response", "complete molecular remission" or "CMR" is achieved when the level of an oncogenic fusion protein such as BCR-ABL decreases by at least about 3-4 logs over one or more control protein levels such as full-length BCR and/or full-length ABL. In certain embodiments, a complete molecular response is achieved when the ratio of oncogenic fusion protein levels (*e.g.,* BCR-ABL levels) to control protein levels (*e.g.,* BCR or ABL levels) during anticancer drug therapy is reduced by at least about 3-4 logs relative to the ratio of the same proteins prior to anticancer drug (*e.g.,* tyrosine kinase inhibitor) therapy. In contrast to definitions of complete molecular response which rely on the detection of mRNA transcript levels, the antibody-based proximity dual detection assays of the present invention advantageously provide the ability to detect one cancer (*e.g.,* CML) cell in the background of about 1,000,000 cells from a healthy donor, thereby enabling the determination of a complete molecular response by measuring and comparing BCR-ABL, BCR, and/or ABL protein levels. In other embodiments, a complete molecular response is a clinical classification in which BCR-ABL mRNA transcripts are undetectable by qPCR and/or nested PCR in at least two consecutive blood samples of adequate quality as to ensure the capability to detect a 4.0-4.5-log drop in BCR-ABL mRNA levels. In certain instances, a complete molecular response may be defined as a reduction of at least 4.5-logs below a standardized baseline value on a logarithmic scale in the ratio of BCR-ABL to ABL, BCR or control mRNA transcripts expressed as a percentage. *See, e.g.,* Press et al., Blood, 107:4250-4256 (2006); Muller et al., Leukemia., 23:1957-1963 (2009); and Baccarani et al., J. Clin. Oncol., 27:6041-6051 (2009).

The term "course of therapy" includes any therapeutic approach taken to relieve or prevent one or more symptoms associated with a cancer such as a hematological malignancy (*e.g.,* leukemia, lymphoma, *etc.*). The term encompasses administering any compound, drug, procedure, and/or regimen useful for improving the health of an individual with cancer and includes any of the therapeutic agents described herein. One skilled in the art will appreciate that either the course of therapy or the dose of the current course of therapy can be changed (*e.g.,* increased or decreased) based upon the expression and/or activation levels of one or more oncogenic fusion proteins and/or signal transduction molecules determined using the methods of the present invention.

### III. Description of the Embodiments

The present invention provides antibody-based arrays for detecting the activation state and/or total amount of one or more oncogenic fusion proteins and/or signal transduction molecules in a biological sample such as a cellular extract or lysate. The present invention also provides methods of using such arrays for facilitating cancer prognosis and diagnosis, the prediction or identification of resistance to drug treatment, and the design of personalized, targeted therapies. In particular embodiments, the compositions and methods of the present invention advantageously identify patients who are resistant to therapy with a tyrosine kinase inhibitor such as imatinib due to mutations in the target protein kinase (*e.g.,* BCR-ABL), non-compliance with the therapeutic regimen, and/or administration of a suboptimal drug dose.

In one particular embodiment, the present invention provides assays such as, *e.g.,* immunoassays, for the real-time detection of the level of expression and/or the degree of activation (*e.g.,* phosphorylation) of BCR-ABL, substrates thereof, and/or other signal transduction molecules in a biological sample such as a cellular extract or lysate. As such, the present invention advantageously provides benefits to patients with hematological malignancies such as CML who are receiving one or more targeted therapies by screening and monitoring them throughout the course of therapy and evaluating whether they should be moved to an alternative targeted therapy such as, *e.g.,* nilotinib (Tasigna^{®}), to effectively inhibit the target molecule (*e.g.,* BCR-ABL) with minimal toxicity.

In certain embodiments, the present invention provides a method having a superior sensitivity range for the detection of oncogenic fusion proteins such as BCR-ABL. Current immunoassays for detecting BCR-ABL proteins in cellular extracts can detect generally one BCR-ABL-positive leukemic cell in 10-100,000 normal cells, equivalent to a detection sensitivity of 10-0.001% (*see, e.g.,* Jilani et al., Leuk. Res., 32:936-943 (2008); Weerkamp et al., Leukemia, 23:1106-1117 (2009); Raponi et al., Haematologica, 94:1767-1770 (2009); and U.S. Patent Publication No. US 2006/0172345). The proximity assays described herein advantageously exhibit increased sensitivity to about 1:100,000-10,000,000 cells (*i.e.*, one leukemic cell to about 100,000-10,000,000 normal cells; equivalent to a detection sensitivity of about 0.001-0.00001%) or about 1:1,000,000-10,000,000 cells (*i.e.*, one leukemic cell to about 1,000,000-10,000,000 normal cells; equivalent to a detection sensitivity of about 0.0001-0.00001%), and include, *e.g.,* about 1:100,000 cells, 1:200,000 cells, 1:300,000 cells, 1:400,000 cells, 1:500,000 cells, 1:600,000 cells, 1:700,000 cells, 1:800,000 cells, 1:900,000 cells, 1:1,000,000 cells, 1:2,000,000 cells, 1:3,000,000 cells, 1:4,000,000 cells, 1:5,000,000 cells, 1:6,000,000 cells, 1:7,000,000 cells, 1:8,000,000 cells, 1:9,000,000 cells, 1:10,000,000 cells, 1:100,000-500,000 cells, 1:100,000-1,000,000 cells, 1:500,000-1,000,000 cells, 1:100,000-5,000,000 cells, 1:500,000-10,000,000 cells, 1:2,000,000-10,000,000 cells, 1:5,000,000-10,000,000 cells, 1:1,000,000-7,500,000 cells, 1:1,000,000-5,000,000 cells, and any other ranges therein. Sensitivity of the methods described herein may be comparable to or exceed that of standard nucleic acid-based BCR-ABL assays (*e.g.,* qPCR or nested PCR), which fall within the detection range of 1 leukemic cell to 10,000-1,000,000 normal cells. For additional descriptions of the sensitivity range of nucleic acid-based BCR-ABL assays, *see, e.g.,* Press et al., Blood, 107:4250-4256 (2006) and Radish JP, Blood, 114:3376-3381 (2009).

In particular embodiments, the antibody-based proximity assays of the present invention advantageously enable a higher degree of sensitivity and/or specificity in detecting the presence, level, and/or activation state of oncogenic fusion proteins such as BCR-ABL compared to current immunoassays and nucleic acid-based assays for detecting BCR-ABL, thereby providing a more accurate determination of response indicators such as, for example, a complete cytogenetic response, a major molecular response, a complete molecular response, and combinations thereof. As a non-limiting example, current nucleic acid-based assays are not sensitive enough to detect very low amounts of BCR-ABL transcripts in a patient sample, such that a determination of a complete molecular response using current nucleic acid-based assays does not necessarily mean that the patient is cured and no longer has the BCR-ABL-mediated disease (*e.g.,* CML).

In one aspect, the present invention provides a method for determining the level or activation state of an oncogenic fusion protein, the method comprising:
(a) contacting a cellular extract with a first binding moiety specific for a first domain of a first full-length protein under conditions suitable to transform the first full-length protein present in the cellular extract into a complex comprising the first full-length protein and the first binding moiety, wherein the first domain of the first full-length protein is absent from a corresponding oncogenic fusion protein comprising a second, different domain of the first full-length protein fused to a first domain of a second, different full-length protein;
(b) removing the complex from step (a) from the cellular extract to form a cellular extract devoid of the first full-length protein;
(c) contacting the cellular extract from step (b) with a second binding moiety specific for the second, different domain of the first full-length protein under conditions suitable to transform the oncogenic fusion protein present in the cellular extract into a complex comprising the oncogenic fusion protein and the second binding moiety; and
(d) determining the level or activation state of the complex from step (c), thereby determining the level or activation state of the oncogenic fusion protein.

In one embodiment, the cellular extract comprises an extract of cells isolated from a sample. In certain instances, the sample is selected from whole blood, serum, plasma, fine needle aspirate (FNA), urine, sputum, bronchial lavage fluid, tears, nipple aspirate, lymph, saliva, and combinations thereof. In another embodiment, the sample is obtained from a patient having cancer. In some instances, the cancer may be caused by the formation of an oncogenic fusion protein due to a chromosomal translocation in the cancer cells. Examples of such cancers include, but are not limited to, a hematological malignancy, an osteogenic sarcoma, a soft tissue sarcoma, and combinations thereof. In particular embodiments, the hematological malignancy is a leukemia or lymphoma. In one preferred embodiment, the leukemia is chronic myelogenous leukemia (CML). In another embodiment, the isolated cells from which the cellular extract or lysate is prepared may comprise circulating tumor cells, leukocytes, or combinations thereof. In certain embodiments, the isolated cells are stimulated *in vitro* with growth factors. In some instances, the isolated cells are incubated with an anticancer drug prior to growth factor stimulation. In other instances, the isolated cells are lysed following growth factor stimulation to produce the cellular extract.

In some embodiments, the cellular extract is prepared from freshly collected or frozen bone marrow or whole blood samples. As a non-limiting example, a whole blood sample treated with anticoagulants (*e.g.,* EDTA, heparin and/or acid-citrate-dextrose(ACD)) is first separated into a plasma or serum fraction and a cellular fraction. The cellular fraction may be processed by hypotonic lysis of red blood cells with ammonium chloride and/or Ficoll-HyPaque density-gradient centrifugation to isolate leukocytes from a blood sample. The isolated cells present in the cellular fraction may be lysed to thereby transform the isolated cells into a cellular extract by any technique known in the art, such as those described in Raponi et al., Leuk Res, 32:923-43 (2008); Weerkamp et al., Leukemia, 23:1106-1117 (2009); and U.S. Patent Nos. 6,610,498 and 6,686,165.

In some instances, the isolated leukocytes may be treated with one or more cell-permeable protease inhibitors prior to lysis. Cell-permeable protease inhibitors include, but are not limited to, diisopropyl flurophosphate (DFP), 4-(2-aminoethyl)benzenesulfonyl fluoride hydrochloride (AEBSF), phenylmethanesulfonyl fluoride (PMSF) and mixtures thereof. As a non-limiting example, isolated leukocytes are incubated for 10-30 min on ice in a buffer containing 20mM AEBSF and 1mM PMSF in PBS. The cells are centrifuged gently for 5 min at 520g at 4°C to separate supernatant and the isolated leukocytes. Treatment of isolated leukocytes with protease inhibitors is described, *e.g.,* in Weerkamp et al., Leukemia, 23:1106-1117 (2009).

In some instances, the isolated leukocytes may be incubated for up to 30 min on ice in RIPA lysis buffer containing one or more protease inhibitors. RIPA Buffer comprises or consists essentially of 50 mM Tris HCl, pH7.5, 150 mM NaCl, 1% NP40, and 0.1% sodium dodecyl sulfate. In certain other instances, RIPA Buffer does not contain sodium deoxycholate. Following incubation, the cell mixture is centrifuged at ≥ 18,000g for 1-10 minutes at 4°C to separate the cellular extract and the cell debris. The cellular extract is collected. For additional descriptions of cell lysis protocols, *see, e.g.,* Raponi et al., Haematologica, 94:1767-1770 (2009); Weerkamp et al., Leukemia, 23:1106-1117 (2009); and U.S. Patent Publication No. US 2006/0172345.

In some embodiments, plasma is prepared from fresh whole peripheral blood samples collected and treated with one or more anticoagulants (*e.g.,* EDTA, heparin or ACD), such as described in Jilani et al., Leuk. Res., 32:936-943 (2008). As a non-limiting example, blood samples may be separated into a plasma or serum fraction and a cellular fraction. The plasma may be stored at -70-80°C until assayed or assayed within 96 hours of the collection of blood sample.

In certain embodiments, the oncogenic fusion protein is selected from the group consisting of BCR-ABL, DEK-CAN, E2A-PBX1, RARa-PML, IREL-URG, CBFβ-MYH11, AML1-MTG8, EWS-FLI, LYT-10-Cα1, HRX-ENL, HRX-AF4, NPM-ALK, IGH-MYC, RUNX1-ETO, TEL-TRKC, TEL-AML1, MLL-AF4, TCR-RBTN2, COL1A1-PDGF, E2A-HLF, PAX3-FKHR, ETV6-NTRK3, RET-PTC, TMRSS-ERG, TPR-MET, and combinations thereof. In particular embodiments, the oncogenic fusion protein is BCR-ABL. In certain instances, the first full-length protein is BCR, the first domain of the first full-length protein comprises the carboxyl-terminal region of BCR (BCR-C), and the second, different domain of the first full-length protein comprises the amino-terminal region of BCR (BCR-N). In certain other instances, the second, different full-length protein is ABL, the first domain of the second, different full-length protein comprises the carboxyl-terminal region of ABL (ABL-C), and the second, different domain of the second, different full-length protein comprises the amino-terminal region of ABL (ABL-N). In one alternative embodiment, the first full-length protein is ABL and the second, different full-length protein is BCR.

In some embodiments, the activation state is selected from the group consisting of a phosphorylation state, ubiquitination state, complexation state, and combinations thereof. In one preferred embodiment, the oncogenic fusion protein is BCR-ABL and the activation state is a phosphorylation state.

In other embodiments, the assay methods of the present invention further comprise determining the level or activation state of one or more signal transduction molecules. In particular embodiments, the one or more signal transduction molecules comprises a BCR-ABL substrate such as, *e.g.,* CRKL, JAK2, STAT5, Src, FAK, c-ABL, c-CBL, SHC, SHP-2, VAV, BAP-1, and combinations thereof.

In one particular embodiment, the first binding moiety comprises a first antibody. In some instances, the first antibody is attached to a solid support. Non-limiting examples of solid supports include glass, plastic, chips, pins, filters, beads, paper, membrane, fiber bundles, and combinations thereof. In preferred embodiments, the first antibody is attached to a bead (*e.g.,* magnetic bead, polystyrene bead, *etc.*), and the bead functions as a depletion tag to remove the first full-length protein from the cellular extract.

In another particular embodiment, the second binding moiety comprises a second antibody. In some instances, the first antibody is attached to a solid support. Non-limiting examples of solid supports include glass, plastic, chips, pins, filters, beads, paper, membrane, fiber bundles, and combinations thereof. In one preferred embodiment, the second antibody is restrained on a solid support such as a membrane (*e.g.,* nylon, nitrocellulose, PVDF, *etc.*) in an addressable array. In another embodiment, the second antibody is attached to a bead (*e.g.,* magnetic bead, polystyrene bead, *etc.*), wherein the bead may optionally contain a dye such as a fluorophore (*e.g.,* a colored bead). In instances where a plurality of beads is used, each bead may contain an independently selected dye such as a fluorophore (*e.g.,* a red or infrared fluorophore) of differing intensities or with differing excitation and/or emission spectra.

In preferred embodiments, steps (c) and (d) comprise a proximity dual detection assay (also known as a COllaborative Proximity Immuno Assay ("COPIA")) as described herein. In other embodiments, steps (c) and (d) comprise an enzyme-linked immunosorbent assay (ELISA), a flow cytometry assay, or a tag-sorting assay as described herein.

In embodiments where steps (c) and (d) comprise a proximity dual detection assay, step (c) may further comprise:
(c') contacting the cellular extract from step (b) with a third binding moiety and a fourth binding moiety under conditions suitable to transform the oncogenic fusion protein present in the cellular extract into a complex comprising the oncogenic fusion protein and the second, third, and fourth binding moieties,
wherein the third binding moiety is labeled with a facilitating moiety and is specific for (*e.g.,* specifically binds to) one or more epitopes present in the following domains or sequences: (i) the first domain of the second, different full-length protein; (ii) the second, different domain of the first full-length protein; or (iii) the site of fusion between the second, different domain of the first full-length protein and the first domain of the second, different full-length protein,
wherein the fourth binding moiety is labeled with a first member of a signal amplification pair and is specific for the first domain of the second, different full-length protein, and
wherein the facilitating moiety generates an oxidizing agent which channels to and reacts with the first member of the signal amplification pair.

In embodiments where steps (c) and (d) comprise a proximity dual detection assay, step (d) may further comprise:
(d') incubating the complex from step (c') with a second member of the signal amplification pair to generate an amplified signal; and
(d") detecting the amplified signal generated from the first and second members of the signal amplification pair.

In certain embodiments, the cellular extract from step (b) may be contacted with a dilution series of the second binding moiety to form a plurality of complexes comprising the oncogenic fusion protein and the second binding moiety. In some embodiments, the third and fourth binding moieties may comprise third and fourth antibodies, respectively. In some instances, the third and fourth antibodies are both activation state-independent antibodies. In such instances, the amplified signal generated from the first and second members of the signal amplification pair is correlative to the total amount of the oncogenic fusion protein. In other instances, the third antibody is an activation state-independent antibody and the fourth antibody is an activation state-dependent antibody. In such instances, the amplified signal generated from the first and second members of the signal amplification pair is correlative to the amount of activated (*e.g.,* phosphorylated) oncogenic fusion protein.

In other embodiments, the third binding moiety may be directly labeled with the facilitating moiety. In yet other embodiments, the fourth binding moiety is directly labeled with the first member of the signal amplification pair. In alternative embodiments, the fourth binding moiety is labeled with the first member of the signal amplification pair via binding between a first member of a binding pair conjugated to the second detection antibody and a second member of the binding pair conjugated to the first member of the signal amplification pair. In these embodiments, the first member of the binding pair is biotin and/or the second member of the binding pair is streptavidin.

In further embodiments, the facilitating moiety is glucose oxidase. In certain instances, the glucose oxidase and the third binding moiety are conjugated to a sulfhydryl-activated dextran molecule. In such instances, the sulfhydryl-activated dextran molecule has a molecular weight of about 500kDa. In other instances, the oxidizing agent is hydrogen peroxide (H₂O₂). In such instances, the first member of the signal amplification pair is a peroxidase such as, *e.g.,* horseradish peroxidase (HRP), and/or the second member of the signal amplification pair is a tyramide reagent such as, *e.g.,* biotin-tyramide. In particular embodiments, the amplified signal is generated by peroxidase oxidization of the biotin-tyramide to produce an activated tyramide. In certain instances, the activated tyramide is directly detected. In certain other instances, the activated tyramide is detected upon the addition of a signal-detecting reagent. Non-limiting examples of signal-detecting reagents include a streptavidin-labeled fluorophore and a combination of a streptavidin-labeled peroxidase and a chromogenic reagent such as, *e.g.,* 3,3',5,5'-tetramethylbenzidine (TMB).

In certain embodiments, the assay methods of the present invention further comprise:
(e) contacting the cellular extract with a fifth binding moiety specific for a second domain of the second, different full-length protein under conditions suitable to transform the second, different full-length protein present in the cellular extract into a complex comprising the second, different full-length protein and the fifth binding moiety, wherein the second domain of the second, different full-length protein is absent from the oncogenic fusion protein; and
(f) removing the complex from step (e) from the cellular extract to form a cellular extract devoid of the second, different full-length protein,
wherein step (e) is performed before, during, or after step (a).

In one particular embodiment, the fifth binding moiety comprises a fifth antibody. In some instances, the fifth antibody is attached to a solid support. Non-limiting examples of solid supports include glass, plastic, chips, pins, filters, beads, paper, membrane, fiber bundles, and combinations thereof. In preferred embodiments, the fifth antibody is attached to a bead (*e.g.,* magnetic bead, polystyrene bead, *etc.*), and the bead functions as a depletion tag to remove the second, different full-length protein from the cellular extract.

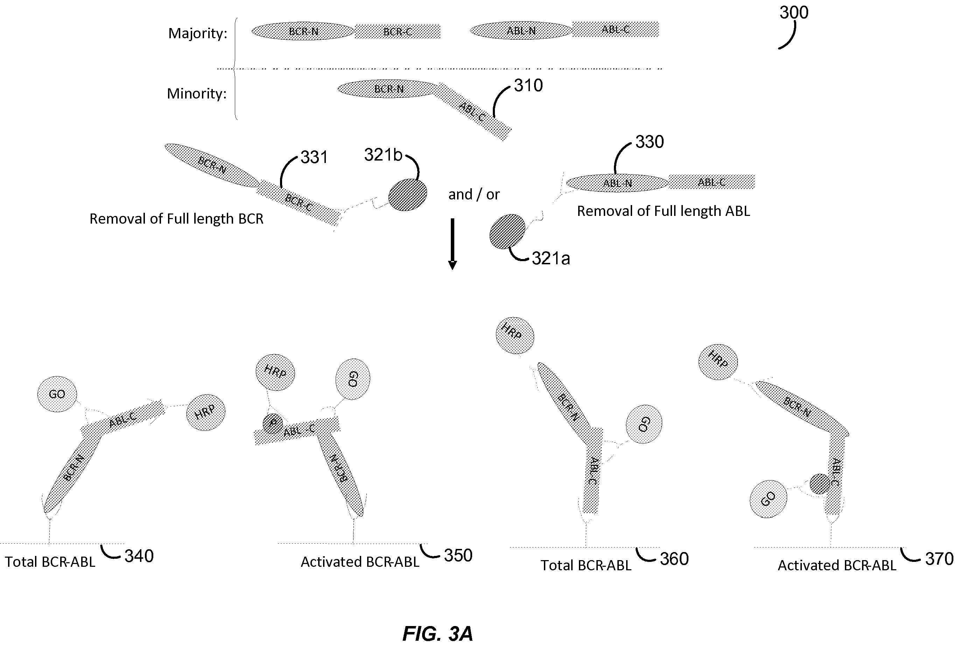

Figure 3A illustrates an exemplary proximity assay (300) for detecting the presence (total level) and/or activation state (phosphorylation level) of an oncogenic fusion protein such as BCR-ABL (310). The oncogenic fusion protein encoded by the chimeric BCR-ABL gene varies in size, depending on the breakpoint in the BCR gene. The BCR-ABL fusion gene is generated by the reciprocal translocation of the ABL gene located on the long arm of chromosome 9 and the BCR gene located on the long arm of chromosome 22, resulting in the oncogenic fusion gene on chromosome 22q-, also referred to as the Philadephia chromosome. *See, e.g.,* Kurzock et al., N. Engl. J. Med. 319:990-8 (1988); Rosenberg et al., Adv. In Virus Res. 35:39-81 (1988). Depending on the chromosomal breakpoints that translocate the ABL gene onto the N-terminal of the BCR gene, different lengths of BCR-ABL fusion genes are formed. It has been determined that the breakpoints in the ABL gene are distributed over approximately 200kb between exons 1b and a2, and the BCR gene breakpoints are clustered within three regions; the major breakpoint (M-BCR) between exons 13-15 (b2-b4); the minor breakpoint (m-BCR) between alternative exons 1 and 2 (e1 and e2); and the micro breakpoint (mu-BCR) in intron 19 (e19). Thus, depending on the BCR-ABL gene rearrangement, unique BCL-ABL proteins are formed. *See, e.g.,* Konopka et al., Cell 37:1035-42 (1984); van Dongen, JJM, Leukemia 19:1292-5 (2005).

The p210 BCR-ABL protein is generated from the b3a2 (e14a2) and/or b2a2 (e13a2) gene transcripts, which are detected in more than 95% of CML cases and a subset of ALL. This protein contains 1790 amino acids and is composed of an oligomerization domain (OLI) from BCR at the N-terminus, followed by the S/T kinase domain from BCR, an insert of amino acid sequence from BCR which is not present in normal BCR, followed by the SH3, SH2 and Y kinase domains from ABL as well as the C-terminal proline-rich domain of ABL. The p190 BCR-ABL fusion protein is encoded by the e1a2 fusion gene transcript which is principally associated with Ph-positive ALL. Rare cases of CML are due to a p190-type BCR-ABL gene translocation, and in these, the disease tends to have a prominent monocytic component, resembling chronic myelomonocytic leukemia (CMML). The p230 BCR-ABL protein is formed from the e19a2 gene transcript which has been associated with neutrophilic-CML, classical CML and AML. *See, e.g.,* Konopka et al., Cell 37:1035-42 (1984); van Dongen, JJM, Leukemia 19:1292-5 (2005). Exceptional CML cases have been described with BCR breakpoints outside the three defined cluster regions, or with unusual breakpoints in ABL (*see, e.g.,* Melo, Baillieres Clin. Haematol., 10:203-22 (1997)). The proximity assay described herein is capable of detecting both total and activated levels of a BCR-ABL protein encoded by a chimeric BCR-ABL gene having a breakpoint at any location within the BCR gene.

As depicted in Figure 3A, full-length BCR protein can first be removed from a patient sample by using a depletion tag specific for the carboxyl-terminal region of full-length BCR (331). A non-limiting example of such a depletion tag is a bead to which an antibody specific for the carboxyl-terminal region of full-length BCR is attached (321b). Once full-length BCR is removed from the sample, a capture antibody specific for the amino-terminal region of BCR (BCR-N) is used to capture the BCR-ABL fusion protein. Once BCR-ABL is captured via binding between BCR-N and the capture antibody specific for BCR-N, the total concentration of BCR-ABL is determined (340), and activated BCR-ABL (350) is also measured. In some embodiments, the facilitating moiety (*e.g.,* GO) is coupled to an antibody specific for the carboxyl-terminal region of ABL (ABL-C) and the first member of the signal amplification pair (*e.g.*, HRP) is coupled to an antibody specific for ABL-C at a different epitope than that recognized by the facilitating moiety-coupled antibody. In certain instances, the signal amplification pair-coupled antibody is an activation state-independent antibody which binds to ABL-C regardless of its activation state and thereby measures the total concentration of BCR-ABL (340). In certain other instances, the signal amplification pair-coupled antibody is an activation state-dependent antibody which binds to phospho-ABL-C (*e.g.,* pY245, pY412) and thereby measures the concentration of activated BCR-ABL (350). In one alternative embodiment, the facilitating moiety (*e.g.,* GO) is coupled to an antibody specific for BCR-N at a different epitope than that recognized by the BCR-ABL capture antibody and the first member of the signal amplification pair (*e.g.,* HRP) is coupled to an antibody specific for ABL-C. The signal amplification pair-coupled antibody can be an activation state-independent antibody or an activation state-dependent antibody as described herein.

In some embodiments, full-length ABL protein can additionally or alternatively be removed from the patient sample prior to capture and detection of BCR-ABL expression and/or activation by using a depletion tag specific for the amino-terminal region of full-length ABL (330). A non-limiting example of such a depletion tag is a bead to which an antibody specific for the amino-terminal region of full-length ABL is attached (321a). In these embodiments, once BCR-ABL is captured via binding between ABL-C and a capture antibody specific for ABL-C, the total concentration of BCR-ABL is determined (360), and activated BCR-ABL (370) is also measured. In some embodiments, the facilitating moiety (*e.g.,* GO) is coupled to an antibody specific for ABL-C at a different epitope than that recognized by the BCR-ABL capture antibody and the first member of the signal amplification pair (*e.g.,* HRP) is coupled to an antibody specific for BCR-N. In certain instances, the facilitating moiety-coupled antibody is an activation state-independent antibody which binds to ABL-C regardless of its activation state and thereby measures the total concentration of BCR-ABL (360). In certain other instances, the facilitating moiety-coupled antibody is an activation state-dependent antibody which binds to phospho-ABL-C (*e.g.,* pY245, pY412) and thereby measures the concentration of activated BCR-ABL (370).

In embodiments where the facilitating moiety is GO and the first member of the signal amplification pair is HRP, binding of both the GO-coupled antibody and the HRP-coupled antibody to the BCR-ABL fusion protein brings the GO moiety within sufficient proximity to the HRP moiety such that a signal generated by the GO moiety (*i.e.*, H₂O₂) can channel to the HRP moiety, resulting in the generation of a detectable and/or amplifiable signal. An advantage of proximity channeling, as used in the methods described herein, is that a single detectable signal which correlates to total or activated BCR-ABL protein levels is generated only upon the binding of all three antibodies (*e.g.,* capture antibody, facilitating moiety-coupled antibody, and signal amplification pair-coupled antibody), resulting in increased assay specificity, lower background, and simplified detection.

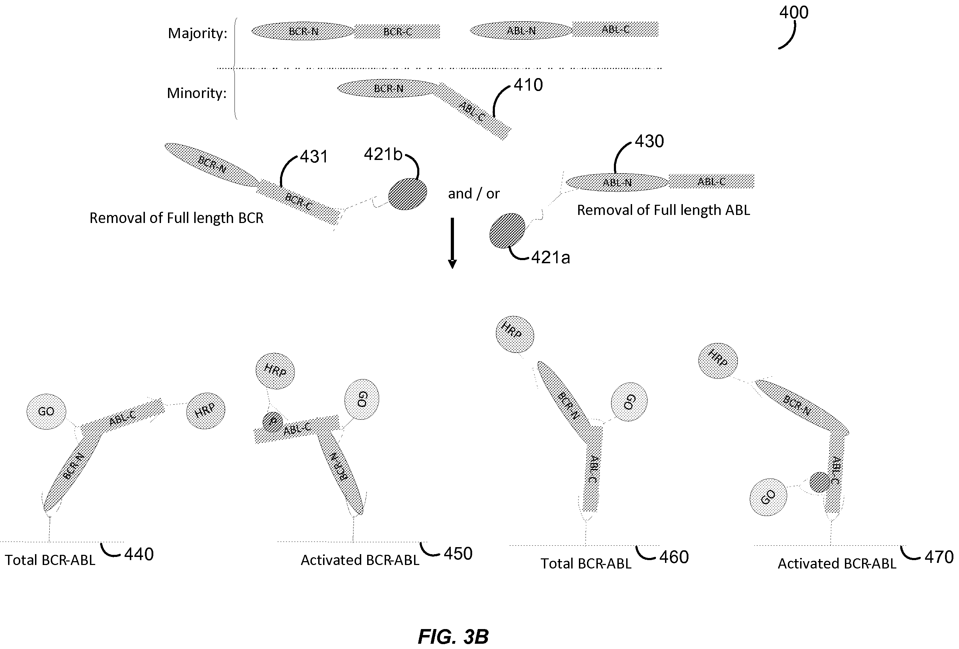

An alternative embodiment (400) for detecting the presence (total level) and/or activation state (phosphorylation level) of BCR-ABL (410) using junction antibodies is shown in Figure 3B. Again, beads (421a and/or 421b) are used to remove full length BCR and/or ABL proteins, by, *e.g.,* their carboxyl- and amino-terminus, respectively. Then, by capturing the N-terminus portion of BCR-ABL using a specific binding moiety immobilized on a solid support, the total concentration of BCR-ABL is determined (440), and activated BCR-ABL (450) is also measured. Total BCR-ABL (460) and activated protein (470) can also be determined by capturing ABL-C and either using a junction antibody for measuring total BCR-ABL levels or using an ABL phophorylation-specific antibody for measuring activated BCR-ABL levels.

In particular, Figure 3B (440) illustrates that the total amount of BCR-ABL present in a biological sample such as serum can be detected by contacting the captured analyte with (i) a first detection antibody (*i.e.*, junction antibody) specific for the site or point of fusion between the amino-terminal region of BCR (BCR-N) and the carboxyl-terminal region of ABL (ABL-C), and (ii) a second detection antibody specific for ABL-C. Both first and second detection antibodies bind to BCR-ABL independent of its activation state. The first detection antibody (*i.e.*, junction antibody) is labeled with glucose oxidase (GO), and the second detection antibody is labeled with horseradish peroxidase (HRP). Binding of both first and second detection antibodies to the BCR-ABL fusion protein brings the GO moiety within sufficient proximity to the HRP moiety such that a signal generated by the GO moiety (*i.e.*, H₂O₂) can channel to the HRP moiety, resulting in the generation of a detectable and/or amplifiable signal.

Figure 3B (450) further illustrates that the amount of activated BCR-ABL present in a biological sample such as serum can be detected by contacting the captured analyte with (i) a first detection antibody (*i.e.*, junction antibody) specific for the site or point of fusion between BCR-N and ABL-C, and (ii) a second detection antibody specific for an activated (*e.g.,* phosphorylated) form of BCR-ABL. The first detection antibody binds to BCR-ABL independent of its activation state, while the second detection antibody binds to a site of activation (*e.g.,* phosphorylation) present on the ABL-C domain of the fusion protein. The first detection antibody (*i.e.,* junction antibody) is labeled with glucose oxidase (GO), and the second detection antibody is labeled with horseradish peroxidase (HRP). Binding of both first and second detection antibodies to the BCR-ABL fusion protein brings the GO moiety within sufficient proximity to the HRP moiety such that a signal generated by the GO moiety (H₂O₂) can channel to the HRP moiety, resulting in the generation of a detectable and/or amplifiable signal.

In embodiments where steps (c) and (d) comprise an ELISA, the ELISA may comprise a sandwich ELISA. Any suitable antibody pair may be used for the capture and detecting antibodies in a sandwich ELISA. One of skill in the art will know and appreciate how to select an appropriate antibody pair for the assay. Generally, two antibodies are selected that bind to the target of interest, *e.g.*, BCR-ABL, at different epitopes such that the binding of the first (capture) antibody does not interfere with the second (detecting) antibody. In preferred embodiments, the first (capture) antibody binds to the second, different domain of the first full-length protein (*e.g.,* BCR-N) and the second (detecting) antibody binds to the first domain of the second, different full-length protein (*e.g.,* ABL-C). In certain embodiments, the detecting antibody will be conjugated to an enzyme, for example, horseradish peroxidase (HRP) or alkaline phophatase (AP), to aid in the detection of the complex. In other embodiments, a secondary antibody conjugated to an enzyme (*e.g.*, HRP or AP), which binds to the detecting antibody, may be used in the assay. Generally, the complex will then be detected by the use of a luminescent substrate, *e.g.,* Ultra LITE™ (NAG Research Laboratories); SensoLyte® (AnaSpec); SuperSignal ELISA Femto Maximum Sensitivity Substrate (Thermo Scientific); SuperSignal ELISA Pico Chemiluminescent Substrate (Thermo Scientific); and CPSD (disodium 3-(4-methoxyspiro{1,2-dioxetane-3,2'-(5'-chloro)tricyclo[3.3.1.13,7]decan}-4-yl)phenyl phosphate; Tropix, Inc). The CPSD substrate can be found in chemiluminescent detection systems, such as, *e.g.,* the ELISA-Light™ System (Applied Biosystems). In one preferred embodiment, the BCR-ABL sandwich ELISA comprises the use of an anti-BCR-N antibody as the capture antibody, wherein the capture antibody is restrained on a solid support such as a microplate well, and an HRP-conjugated anti-ABL-C antibody as the detecting antibody, wherein the detecting antibody may comprise an activation state-independent antibody or an activation state-dependent (*e.g.,* phospho-specific) antibody.

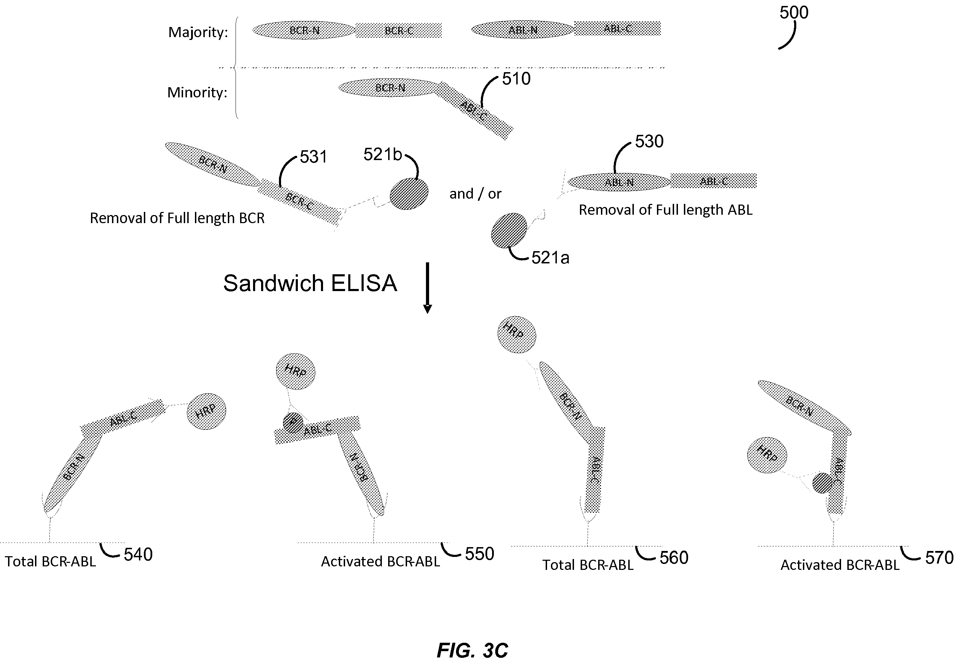

Figure 3C illustrates exemplary sandwich ELISA embodiments (500) for detecting the presence (total level) and/or activation state (phosphorylation level) of BCR-ABL (510). Beads (521a and/or 521b) are used to remove full length BCR and/or ABL proteins, by, for example, their carboxyl- and N-terminus, respectively. Then, using ELISA and capturing the N-terminus portion of BCR-ABL using a specific binding moiety, the total concentration of BCR-ABL is determined (540), and activated BCR-ABL (550) is also measured. Total BCR-ABL (560) and activated protein (570) can also be determined by capturing ABL-C and then contacting the captured BCR-ABL fusion protein with a detection antibody specific for BCR-N or a detection antibody specific for the phophorylation site on ABL-C, respectively, using ELISA.

In embodiments where steps (c) and (d) comprise a flow cytometry (FCM) assay, the FCM assay may comprise a fluorescence-activated cell sorting (FACS) assay. Flow cytometry is a technique for counting and examining microscopic particles, such as cells, by suspending them in a stream of fluid and passing them by an electronic detection apparatus. Flow cytometry allows simultaneous multiparametric analysis of the physical and/or chemical characteristics of up to thousands of particles per second. In flow cytometry, a beam of light (usually laser light) of a single wavelength is directed onto a hydrodynamically-focused stream of fluid. A number of detectors are aimed at the point where the stream passes through the light beam: one in line with the light beam (Forward Scatter or FSC) and several perpendicular to it (Side Scatter (SSC) and one or more fluorescent detectors). Each suspended particle from about 0.2 to about 150 micrometers passing through the beam scatters the ray, and fluorescent chemicals found in the particle or attached to the particle may be excited into emitting light at a longer wavelength than the light source. This combination of scattered and fluorescent light is picked up by the detectors, and, by analyzing fluctuations in brightness at each detector (one for each fluorescent emission peak), it is then possible to derive various types of information about the physical and chemical structure of each individual particle. FSC correlates with the cell volume and SSC depends on the inner complexity of the particle. Some flow cytometers have eliminated the need for fluorescence and use only light scatter for measurement, while other flow cytometers form images of each individual particle's fluorescence, scattered light, and transmitted light.

FACS is a specialized type of flow cytometry. It provides a method for sorting a heterogeneous mixture of particles into two or more containers, one particle at a time, based upon the specific light scattering and fluorescent characteristics of each particle. It is a useful scientific instrument, as it provides fast, objective and quantitative recording of fluorescent signals from individual particles as well as physical separation of particles of particular interest. The particle suspension is entrained in the center of a narrow, rapidly flowing stream of liquid. The flow is arranged so that there is a large separation between particles relative to their diameter. A vibrating mechanism causes the stream of particles to break into individual droplets. The system is adjusted so that there is a low probability of more than one particle per droplet. Just before the stream breaks into droplets, the flow passes through a fluorescence measuring station where the fluorescent character of interest of each particle is measured. An electrical charging ring is placed just at the point where the stream breaks into droplets. A charge is placed on the ring based on the immediately-prior fluorescence intensity measurement, and the opposite charge is trapped on the droplet as it breaks from the stream. The charged droplets then fall through an electrostatic deflection system that diverts droplets into containers based upon their charge. In some systems, the charge is applied directly to the stream, and the droplet breaking off retains charge of the same sign as the stream. The stream is then returned to neutral after the droplet breaks off. A FACS assay may be performed using a FACS Calibur flow cytometer available from BD Biosciences (San Jose, CA).

In certain embodiments, a FACS assay is performed using two antibodies that bind to the target of interest, *e.g.*, BCR-ABL, at different epitopes such that the binding of the first (capture) antibody does not interfere with the second (detecting) antibody. In preferred embodiments, the first (capture) antibody binds to the second, different domain of the first full-length protein (*e.g.,* BCR-N) and the second (detecting) antibody binds to the first domain of the second, different full-length protein (*e.g.,* ABL-C). In certain embodiments, the capture antibodies are attached to beads such as polystyrene beads, and the beads are internally dyed with fluorophores. In some embodiments, the detecting antibody is conjugated to a fluorophore or an enzyme, to aid in the detection of the complex. The detecting antibody may comprise an activation state-independent antibody or an activation state-dependent (*e.g.,* phospho-specific) antibody. In other embodiments, a fluorophore, enzyme, or other detection moiety, which binds to the detecting antibody, may be used in the assay. Generally, the complex may then be detected as described above.

In embodiments where steps (c) and (d) comprise a tag-sorting assay, the tag-sorting assay may comprise a Luminex^{®} assay. Luminex^{®} assays are available, *e.g.,* from Invitrogen Corporation (Carlsbad, CA). In some instances, the tag-sorting assay comprises a multiplex Luminex^{®} assay format. Generally, two antibodies are selected that bind to the target of interest, *e.g.*, BCR-ABL, at different epitopes such that the binding of the first (capture) antibody does not interfere with the second (detecting) antibody. In preferred embodiments, the first (capture) antibody binds to the second, different domain of the first full-length protein (*e.g.,* BCR-N) and the second (detecting) antibody binds to the first domain of the second, different full-length protein (*e.g.,* ABL-C). In certain embodiments, the capture antibodies are attached to polystyrene beads, and the beads are internally dyed with red and infrared fluorophores of differing intensities. In some embodiments, the detecting antibody is conjugated to a fluorophore or an enzyme, to aid in the detection of the complex. The detecting antibody may comprise an activation state-independent antibody or an activation state-dependent (*e.g.,* phospho-specific) antibody. In other embodiments, a fluorophore, enzyme, or other detection moiety, which binds to the detecting antibody, may be used in the assay. Generally, the complex may then be detected, *e.g.,* by the use of a detection system such as a Luminex® 100™ or 200™ detection system, wherein the beads can be read in single-file by dual lasers for classification and quantification of each analyte.

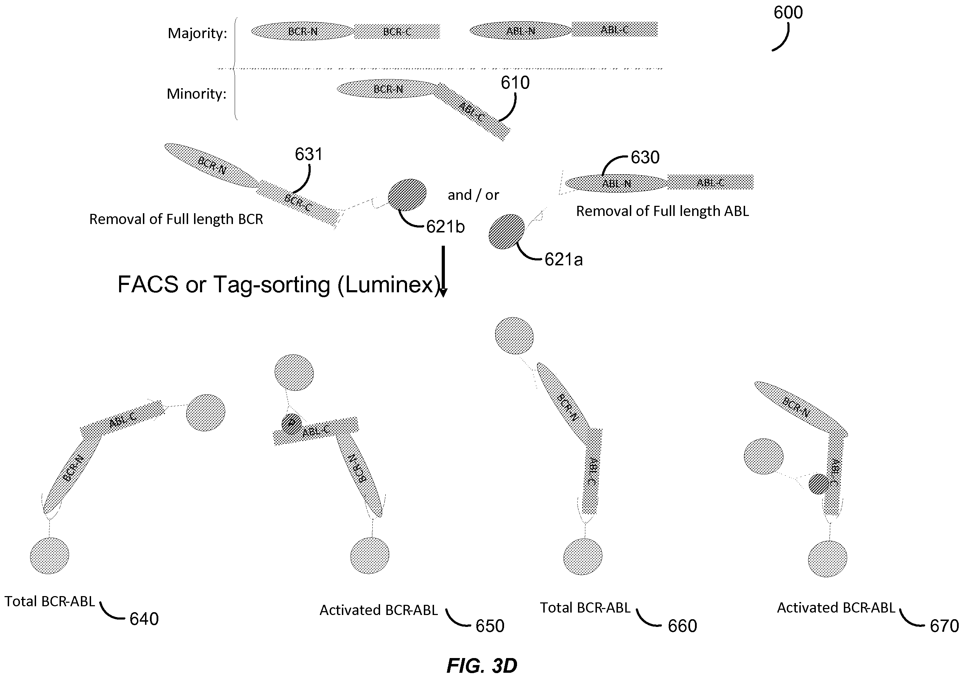

Figure 3D illustrates exemplary flow cytometry and tag-sorting embodiments (600) for detecting the presence (total level) and/or activation state (phosphorylation level) of BCR-ABL (610). Beads (621a and/or 621b) are used to remove full length BCR and/or ABL proteins, by, for example, their carboxyl- and N-terminus, respectively. Then, a bead specific for capturing the N-terminus portion of BCR-ABL using a specific capturing moiety and a bead specific for the C-terminus of ABL are used. Total protein (640) is determined by counting molecules with the two specific tags or colored beads. Similarly, the activated amount of protein can be determined by using a bead with a capture antibody specific for the phosphorylated portion of ABL and the N-terminus of BCR-ABL. By counting these two specific colored beads or tags, the amount of activated BCR-ABL is measured (650). Total BCR-ABL (660) and activated protein (670) can also be determined by capturing the C-terminus of ABL with a specific capture bead and contacting the captured BCR-ABL fusion protein with a bead specific for the BCR-N or a bead specific for the phosphorylated portion of ABL. By counting these two specific colored beads or tags, the amount of total (660) or activated (670) BCR-ABL protein is measured.

Non-limiting examples of antibodies suitable for use in the methods for measuring BCR-ABL total and/or activated protein levels as illustrated in Figures 3A-3D include those set forth in Table 1 below.

**Table 1. Exemplary antibodies for the BCR-ABL assays of the present invention.**

| Target | Ab | Clone | Epitope | Vendor |
|---|---|---|---|---|
| Bcr | AF5129 | | N-terminal | R&D |
| | sc-48422 | H-5 | N-terminal | Santa Cruz |
| | 1684 | EP535Y | C-terminal | Epitomics |
| Abl | AF5414 | | C-terminal | R&D |
| | 4G10 | | phospho-tyrosine | Millipore |
| | ab62189 | | pY245 | Abcam |
| | PAB0397 | | pY245 | Novus |
| | ab47315 | | pY412 | Abcam |
| | ab55284 | | pY412 | Abcam |
| | NB100-92665 | | pY412 | Novus |

Additional examples of antibodies that bind both tumor-specific BCR-ABL fusion proteins and either non-oncogenic native full-length BCR or ABL proteins include, but are not limited to, the BCR b2-epitope specific monoclonal antibody 7C6 that recognizes b2a2 p210 BCR-ABL, b3a2 p210 BCR-ABL, p160 BCR, and p130 BCR proteins described in Dhut et al., Oncogene, 3:561-6 (1988); the SH2 domain-specific antibody 8E9 that recognizes e1a2 p190 BCR-ABL, b2a2, b3a2, and p145 ABL proteins described in U.S. Patent Nos. 5,369,008 and 6,610,498; the amino-terminus of BCR-specific antibody described in U.S. Patent No. 6,107,457; and the carboxyl-terminus of ABL-specific (24-11) mouse monoclonal antibody #SC-23 from Santa Cruz Biotechnology, Inc. (Santa Cruz, CA).

Examples of junction antibodies suitable for binding to the site or point of fusion within the BCR-ABL chimeric protein include, but are not limited to, the BCR-ABL b2a2 junction-specific (L99H4) mouse monoclonal antibody #3908 available from Cell Signaling Technology, Inc. (Danvers, MA), the p210 BCR-ABL fusion protein-specific isolated antibodies described in U.S. Patent Publication No. 20050214301, the BCR-ABL b3a2 junction-specific polyclonal antibody BP-2 described in van Denderen et al., Leukemia, 6:1107-12 (1992), and the BCR-ABL e1a2 junction-specific monoclonal antibody (ER-FP1) described in van Denderen et al., Leukemia, 8:1503-9 (1994). BCR-ABL junction antibodies can be used for the detection of BCR-ABL fusion proteins which are associated with, but not limited to, Ph-positive leukemias.

In certain embodiments, the methods of the present invention provide for preparing the cellular extract from freshly collected or frozen bone marrow (*e.g.,* bone marrow aspirate) or whole blood samples by recovering or isolating cells of interest such as white blood cells (*e.g.,* chronic myelogenous leukemia (CML) cells), *e.g.,* using magnetic bead capture with anti-CD45 antibodies and/or anti-CD 15 antibodies, without any wash steps after cell recovery or isolation. The cellular extract thus obtained can be analyzed for the level of expression and/or activation of one or more oncogenic fusion proteins such as BCR-ABL, substrates thereof, pathways thereof, or combinations thereof. Without being bound to any particular theory, eliminating the need for any wash steps after cell isolation is advantageous because cells of interest can be recovered from blood or bone marrow samples without changing the intracellular concentration of an anticancer drug such as a tyrosine kinase inhibitor. As set forth in Example 9 below, cell isolation without any wash steps as described herein is contrary to the art-accepted practice of washing cells after isolation (*e.g.,* washing bead-bound cells) and provides cellular extracts from recovered cells without substantial dilution of an anticancer drug such as a tyrosine kinase inhibitor (*e.g.,* Gleevec^{®}, Tasigna^{®}, Sprycel^{®}, *etc.*) inside the cells.

In alternative embodiments, the methods of the present invention provide for the simultaneous detection of the total amount and/or activation state of an oncogenic fusion protein (*e.g.,* BCR-ABL) in combination with one or both of the native full-length proteins containing sequences or domains found within the oncogenic fusion protein (*e.g.,* full-length BCR and/or ABL). In one particular embodiment, the present method enables the detection and/or measurement of both total BCR-ABL levels as well as total native full-length BCR and/or ABL levels in a biological sample such as a blood or bone marrow aspirate sample. In certain embodiments, native protein (*e.g.,* full-length BCR and/or ABL) levels are determined along with oncogenic fusion protein (*e.g.,* BCR-ABL) levels in a multiplexed manner on a single pad. In these embodiments, native full-length protein can be advantageously isolated along with oncogenic fusion protein such that the levels of these molecules are determined on the same pad.

As set forth in Example 10 below, these alternative embodiments to the methods of the present invention can be used to detect and/or measure total BCR-ABL levels as well as total native full-length BCR or ABL levels, and a ratio of total BCR-ABL levels to native full-length BCR or ABL levels can be calculated. In some instances, the ratio of BCR-ABL levels to native full-length BCR or ABL levels is calculated to provide a more accurate determination of response indicators such as, for example, a major molecular response (MMR), a complete molecular response (CMR), a complete cytogenetic response (CCyR), and combinations thereof. In other instances, these alternative embodiments to the methods of the present invention can be used to monitor changes in the expression of BCR-ABL with respect to a control such as native full-length BCR or ABL (*e.g.,* by calculating a ratio of total BCR-ABL levels to native full-length BCR or ABL levels) as a function of therapy (*e.g.,* tyrosine kinase inhibitor therapy).

The methods of the present invention are particularly useful for determining the activation (*e.g.,* phosphorylation) status of one or more oncogenic fusion proteins such as BCR-ABL in patients at risk of developing, suspected of having, or diagnosed with a cancer such as a hematological malignancy (*e.g.,* leukemia, lymphoma, *etc.*). In certain instances, the methods of the present invention aid, assist, or facilitate in the diagnosis of a cancer in a subject by measuring activated (*e.g.,* phosphorylated) oncogenic fusion protein levels (*e.g.,* phospho-BCR-ABL levels) to determine whether the subject expresses an activated form of the oncogenic fusion protein (*e.g.,* a BCR-ABL-positive patient). In other embodiments, the methods of the present invention are performed on a subject already determined to express an activated form of the oncogenic fusion protein to optimize therapy, reduce toxicity, and/or monitor the efficacy of therapeutic treatment. In one particular aspect of these embodiments, the level of activated BCR-ABL protein can be determined in a BCR-ABL-positive patient during the course of therapy (*e.g.,* while the patient is on anticancer drug therapy such as Gleevec^{®}, Tasigna^{®}, Sprycel^{®}, *etc.*) to optimize therapy, reduce toxicity, and/or monitor the efficacy of therapeutic treatment. In some embodiments, both total and activated (*e.g.,* phosphorylated) oncogenic fusion protein (*e.g.,* BCR-ABL) levels are measured in accordance with the antibody-based assays of the present invention and a ratio of activated to total oncogenic fusion protein levels (*e.g.,* ratio of phospho/total BCR-ABL protein levels) can be calculated and used to evaluate the course of therapy for a subject, *e.g.,* by comparing the phospho/total ratio of oncogenic fusion protein levels to a ratio of the same calculated for the subject at an earlier time (*e.g.,* at an earlier time while on anticancer drug therapy or at a point in time prior to anticancer drug therapy). In certain embodiments, the ratio of activated to total oncogenic fusion protein levels (*e.g.,* ratio of phospho/total BCR-ABL protein levels) can be calculated with respect to the levels of one or more control proteins such as, *e.g.,* one or both of the native full-length proteins containing sequences or domains found within the oncogenic fusion protein (*e.g.,* BCR and/or ABL for BCR-ABL fusion protein). In preferred embodiments, the total level of the control protein is not affected or substantially changed by the anticancer drug therapy.

The methods of the present invention are also particularly useful for determining the activation (*e.g.,* phosphorylation) status of one or more signal transduction molecules in one or multiple pathways associated with an oncogenic fusion protein such as BCR-ABL in patients at risk of developing, suspected of having, or diagnosed with a cancer such as a hematological malignancy (*e.g.,* leukemia, lymphoma, *etc.*). Exemplary signal transduction molecules include BCR-ABL substrates such as, *e.g.,* CRKL, JAK2, STAT5, Src, FAK, c-ABL, c-CBL, SHC, SHP-2, VAV, BAP-1, and combinations thereof. In certain instances, the methods of the present invention aid, assist, or facilitate in the diagnosis of a cancer in a subject by measuring activated (*e.g.,* phosphorylated) oncogenic fusion protein levels (*e.g.,* phospho-BCR-ABL levels) and activated (*e.g.,* phosphorylated) signal transduction molecule levels (*e.g.,* levels of phospho-CRKL, phospho-JAK2, phospho-STAT5, *etc.*) to determine whether the subject expresses an activated form of the oncogenic fusion protein (*e.g.,* a BCR-ABL-positive patient) and/or an activated form of one or more signal transduction molecules in the pathway. In other embodiments, the methods of the present invention are performed on a subject already determined to express an activated form of the oncogenic fusion protein to optimize therapy, reduce toxicity, and/or monitor the efficacy of therapeutic treatment. In one particular aspect of these embodiments, the levels of activated BCR-ABL protein and one or more signal transduction pathway components (*e.g.,* CRKL, JAK2, STAT5, Src, FAK, *etc.*) can be determined in a BCR-ABL-positive patient during the course of therapy (*e.g.,* while the patient is on anticancer drug therapy such as Gleevec^{®}, Tasigna^{®}, Sprycel^{®}, *etc.*) to optimize therapy, reduce toxicity, and/or monitor the efficacy of therapeutic treatment. In some embodiments, both total and activated (*e.g.,* phosphorylated) oncogenic fusion protein (*e.g.,* BCR-ABL) levels are measured in accordance with the antibody-based assays of the present invention and a ratio of activated to total oncogenic fusion protein levels (*e.g.*, ratio of phospho/total BCR-ABL protein levels) can be calculated (*e.g.,* with respect to the levels of one or more control proteins) and used to evaluate the course of therapy for a subject, *e.g.,* by comparing the phospho/total ratio of oncogenic fusion protein levels to a ratio of the same calculated for the subject at an earlier time (*e.g.,* at an earlier time while on anticancer drug therapy or at a point in time prior to anticancer drug therapy). In other embodiments, both total and activated signal transduction pathway component levels are measured and a ratio of activated to total signal transduction pathway component levels (*e.g.,* ratio of phospho/total CRKL, JAK2, or STAT5 protein levels) can be calculated (*e.g.,* with respect to the levels of one or more control proteins) and used to evaluate the course of therapy for a subject, *e.g.,* by comparing the phospho/total ratio of signal transduction pathway component levels to a ratio of the same calculated for the subject at an earlier time (*e.g.,* at an earlier time while on anticancer drug therapy or at a point in time prior to anticancer drug therapy). In certain instances, the level of expression of an oncogenic fusion protein (*e.g.,* BCR-ABL) can be correlated or related to the level of activation (*e.g.,* phosphorylation) of downstream signal transduction components such as CRKL, JAK2, STAT5, Src, FAK, *etc*.

In one particular aspect, the present invention provides a method for optimizing therapy and/or reducing toxicity in a subject having cancer and receiving a course of therapy for the treatment of cancer, the method comprising:
(a) isolating cancer cells after administration of an anticancer drug (*e.g.,* one or more tyrosine kinase inhibitors such as Gleevec^{®}, Tasigna^{®}, Sprycel^{®}, *etc.*);
(b) lysing the isolated cells to produce a cellular extract;
(c) measuring a level of expression and/or activation (*e.g.,* phosphorylation) of an oncogenic fusion protein in the cellular extract using an assay described herein; and
(d) comparing the measured level of expression and/or activation of the oncogenic fusion protein to a level of expression and/or activation of the oncogenic fusion protein measured at an earlier time during the course of therapy; and
(e) determining a subsequent dose of the course of therapy for the subject or whether a different course of therapy should be administered to the subject based upon the comparison from step (d).

In particular embodiments, both the expression level and the activation level of the oncogenic fusion protein are measured in the cellular extract, *e.g.,* by performing one of the proximity assays described herein. In certain preferred embodiments, the oncogenic fusion protein comprises BCR-ABL. In certain other preferred embodiments, the subject expresses an activated form of the oncogenic fusion protein. In a particularly preferred embodiment, the subject is BCR-ABL-positive (*e.g.,* the subject was determined to have detectable levels of phospho-BCR-ABL prior to administration of the anticancer drug).

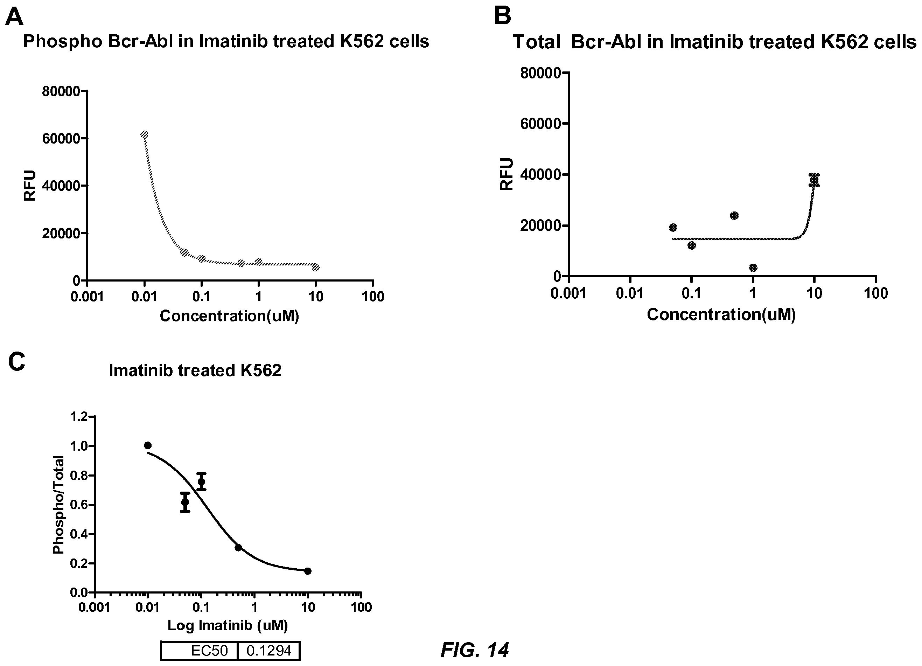

In some embodiments, both total and activated (*e.g.,* phosphorylated) oncogenic fusion protein (*e.g.,* BCR-ABL) levels are measured in the cellular extract in accordance with the antibody-based assays of the present invention and a ratio of activated to total oncogenic fusion protein levels (*e.g.,* ratio of phospho/total BCR-ABL protein levels) can be calculated and used to evaluate the course of therapy for a subject, *e.g.,* by comparing the phospho/total ratio of oncogenic fusion protein levels to a ratio of the same calculated for the subject at an earlier time (*e.g.,* at an earlier time while on anticancer drug therapy or at a point in time prior to anticancer drug therapy). As illustrated in Example 6 below, the phospho/total ratio of oncogenic fusion protein (*e.g.,* BCR-ABL) levels upon anticancer drug inhibition correlates with the percent inhibition of the activated (*e.g.,* phosphorylated) oncogenic fusion protein (*e.g.,* phospho-BCR-ABL) signal upon anticancer drug treatment (*see, e.g.,* Figures 14C, 15C, and 16C). In other embodiments, the ratio of activated to total oncogenic fusion protein levels (*e.g.,* ratio of phospho/total BCR-ABL protein levels) can be calculated with respect to the levels of one or more control proteins such as, *e.g.,* one or both of the native full-length proteins containing sequences or domains found within the oncogenic fusion protein (*e.g.,* BCR and/or ABL for BCR-ABL). In preferred embodiments, the total level of the control protein is not affected or substantially changed by the anticancer drug therapy.

In certain aspects of the methods described herein for optimizing therapy, less than about 50% inhibition of activation (*e.g.,* phosphorylation) of oncogenic fusion protein (*e.g.,* BCR-ABL) levels in a subject indicates a need to increase the subsequent dose of the course of therapy or to administer a different course of therapy (*e.g.,* change the current course of therapy by switching to a different anticancer drug) in order to prevent or reduce the risk of the cancer from relapsing in the subject. As a non-limiting example, in instances where the subject is on Gleevec^{®} therapy, less than about 50% inhibition of the level of phosphorylation of BCR-ABL fusion protein indicates a need to increase the subsequent dose of Gleevec^{®} or to switch the subject to Tasigna^{®} therapy. In certain instances, less than about 49%, 48%, 47%, 46%, 45%, 44%, 43%, 42%, 41%, 40%, 35%, 30%, 25%, 20%, 15%, 10%, or 5% inhibition of activation (*e.g.*, phosphorylation) of oncogenic fusion protein (*e.g.,* BCR-ABL) levels in a subject indicates a need to increase the subsequent dose of the course of therapy or to change the current course of therapy. In some embodiments, the percent inhibition of activation of oncogenic fusion protein levels can be determined by calculating a ratio of activated to total oncogenic fusion protein levels (*e.g.,* ratio of phospho/total BCR-ABL protein levels), optionally with respect to the levels of one or more control proteins (*e.g.,* full-length BCR and/or ABL for BCR-ABL), and comparing the phospho/total ratio of oncogenic fusion protein levels to a ratio of the same calculated for the subject at an earlier time (*e.g.,* at an earlier time while on anticancer drug therapy or at a point in time prior to anticancer drug therapy). One skilled in the art will know of suitable higher or lower doses to which the current course of therapy can be adjusted such that drug therapy is optimized, *e.g.,* a subsequent dose that is at least about 1.5, 2, 2.5, 3, 3.5, 4, 4.5, 5, 5.5, 6, 6.5, 7, 7.5, 8, 8.5, 9, 9.5, 10, 15, 20, 25, 30, 35, 40, 45, 50, or 100-fold higher or lower than the current dose.

In certain other aspects of the methods for optimizing therapy, less than about 50% inhibition of activation (*e.g.,* phosphorylation) of oncogenic fusion protein (*e.g.,* BCR-ABL) levels in a subject indicates a lack of compliance by the subject to the course of therapy (*e.g.,* the subject is not taking the anticancer drug regularly or as directed by a physician) and/or the existence of possible side-effects or toxicity associated with the course of therapy. In these embodiments, it is recommended that the current course of therapy be carefully monitored (*e.g.,* by a physician or other caregiver) for compliance or that a different course of therapy be administered (*e.g.,* the current course of therapy be changed by switching to a different anticancer drug) in order to increase compliance and/or to prevent or reduce the risk of side-effects. In certain instances, less than about 49%, 48%, 47%, 46%, 45%, 44%, 43%, 42%, 41%, 40%, 35%, 30%, 25%, 20%, 15%, 10%, or 5% inhibition of activation of oncogenic fusion protein levels in a subject indicates a lack of compliance by the subject to the course of therapy and/or the existence of possible side-effects or toxicity associated with the course of therapy. In some embodiments, the percent inhibition of activation of oncogenic fusion protein levels can be determined by calculating a ratio of activated to total oncogenic fusion protein levels (*e.g.,* ratio of phospho/total BCR-ABL protein levels), optionally with respect to the levels of one or more control proteins (*e.g.,* full-length BCR and/or ABL for BCR-ABL), and comparing the phospho/total ratio of oncogenic fusion protein levels to a ratio of the same calculated for the subject at an earlier time (*e.g.,* at an earlier time while on anticancer drug therapy or at a point in time prior to anticancer drug therapy).

In further aspects of the methods described herein for optimizing therapy, greater than about 80% inhibition of activation (*e.g.,* phosphorylation) of oncogenic fusion protein (*e.g.,* BCR-ABL) levels in a subject indicates that the subject is on the correct therapy at the correct dose. In certain instances, greater than about 81%, 82%, 83%, 84%, 85%, 86%, 87%, 88%, 89%, 90%, 91%, 92%, 93%, 94%, 95%, 96%, 97%, 98%, 99%, or 100% inhibition of activation (*e.g.,* phosphorylation) of oncogenic fusion protein (*e.g.,* BCR-ABL) levels in a subject indicates that the subject is on the correct anticancer drug therapy at the correct dose. In some embodiments, the percent inhibition of activation of oncogenic fusion protein levels can be determined by calculating a ratio of activated to total oncogenic fusion protein levels (*e.g.,* ratio of phospho/total BCR-ABL protein levels), optionally with respect to the levels of one or more control proteins (*e.g.,* full-length BCR and/or ABL for BCR-ABL), and then comparing the phospho/total ratio of oncogenic fusion protein levels to a ratio of the same calculated for the subject at an earlier time (*e.g.,* at an earlier time while on anticancer drug therapy or at a point in time prior to anticancer drug therapy).

In a related aspect, the present invention provides a method for optimizing therapy and/or reducing toxicity in a subject having cancer and receiving a course of therapy for the treatment of cancer, the method comprising:
(a) isolating cancer cells after administration of an anticancer drug (*e.g.,* one or more tyrosine kinase inhibitors such as Gleevec^{®}, Tasigna^{®}, Sprycel^{®}, *etc.*);
(b) lysing the isolated cells to produce a cellular extract;
(c) measuring a level of expression and/or activation (*e.g.,* phosphorylation) of an oncogenic fusion protein and one or more signal transduction molecules in its pathway in the cellular extract using an assay described herein; and
(d) comparing the measured level of expression and/or activation of the oncogenic fusion protein and signal transduction molecules to a level of expression and/or activation of the oncogenic fusion protein and signal transduction molecules measured at an earlier time during the course of therapy; and
(e) determining a subsequent dose of the course of therapy for the subject or whether a different course of therapy should be administered to the subject based upon the comparison from step (d).

In particular embodiments, both the expression level and the activation level of the oncogenic fusion protein and one or more signal transduction molecules are measured in the cellular extract, *e.g.,* by performing one of the proximity assays described herein. In certain preferred embodiments, the oncogenic fusion protein comprises BCR-ABL. In certain other preferred embodiments, the signal transduction molecules include BCR-ABL substrates such as, *e.g.,* CRKL, JAK2, STAT5, Src, FAK, c-ABL, c-CBL, SHC, SHP-2, VAV, BAP-1, and combinations thereof. In further embodiments, the subject expresses an activated form of the oncogenic fusion protein. In a particularly preferred embodiment, the subject is BCR-ABL-positive (*e.g.,* the subject was determined to have detectable levels of phospho-BCR-ABL prior to administration of the anticancer drug).

In some embodiments, both total and activated (*e.g.,* phosphorylated) oncogenic fusion protein (*e.g.,* BCR-ABL) levels and signal transduction pathway component (*e.g.,* CRKL, JAK2, STAT5) levels are measured in the cellular extract in accordance with the antibody-based assays of the present invention and a ratio of activated to total oncogenic fusion protein levels (*e.g.,* ratio of phospho/total BCR-ABL protein levels) and a ratio of activated to total signal transduction pathway component levels (*e.g.,* ratio of phospho/total CRKL, JAK2, or STAT5 protein levels) can be calculated and used to evaluate the course of therapy for a subject, *e.g.,* by comparing the phospho/total ratio of oncogenic fusion protein and signal transduction pathway component levels to a ratio of the same calculated for the subject at an earlier time (*e.g.,* at an earlier time while on anticancer drug therapy or at a point in time prior to anticancer drug therapy). In other embodiments, the ratio of activated to total oncogenic fusion protein levels (*e.g.,* ratio of phospho/total BCR-ABL protein levels) and the ratio of activated to total signal transduction molecule levels (*e.g.,* ratio of phospho/total CRKL, JAK2, or STAT5 protein levels) can be calculated with respect to the levels of one or more control proteins such as, *e.g.,* one or both of the native full-length proteins containing sequences or domains found within the oncogenic fusion protein (*e.g.,* BCR and/or ABL for BCR-ABL). In preferred embodiments, the total level of the control protein is not affected or substantially changed by the anticancer drug therapy.

In certain aspects of the methods described herein for optimizing therapy, less than about 50% inhibition of activation (*e.g.,* phosphorylation) of one, two, three, four, five, six, or more oncogenic fusion protein (*e.g.,* BCR-ABL) levels and/or signal transduction pathway component (*e.g.,* CRKL, JAK2, STAT5) levels in a subject indicates a need to increase the subsequent dose of the course of therapy or to administer a different course of therapy (*e.g.,* change the current course of therapy by switching to a different anticancer drug) in order to prevent or reduce the risk of the cancer from relapsing in the subject. As a non-limiting example, in instances where the subject is on Gleevec^{®} therapy, less than about 50% inhibition of the level of phosphorylation of BCR-ABL fusion protein and/or a signal transduction pathway component such as CRKL, JAK2, and/or STAT5 indicates a need to increase the subsequent dose of Gleevec^{®} or to switch the subject to Tasigna^{®} therapy. In certain instances, less than about 49%, 48%, 47%, 46%, 45%, 44%, 43%, 42%, 41%, 40%, 35%, 30%, 25%, 20%, 15%, 10%, or 5% inhibition of activation (*e.g.,* phosphorylation) of one, two, three, four, five, six, or more oncogenic fusion protein (*e.g.,* BCR-ABL) levels and/or signal transduction pathway component (*e.g.,* CRKL, JAK2, STAT5) levels in a subject indicates a need to increase the subsequent dose of the course of therapy or to change the current course of therapy. In some instances, the percent inhibition of activation of oncogenic fusion protein and/or signal transduction molecule levels can be determined by calculating a ratio of activated to total oncogenic fusion protein levels (*e.g.,* ratio of phospho/total BCR-ABL protein levels) and/or a ratio of activated to total signal transduction pathway component levels (*e.g.,* ratio of phospho/total CRKL, JAK2, or STAT5 protein levels), optionally with respect to the levels of one or more control proteins (*e.g.,* full-length BCR and/or ABL for BCR-ABL), and comparing the calculated phospho/total ratio to a ratio of the same calculated for the subject at an earlier time (*e.g.,* at an earlier time while on anticancer drug therapy or at a point in time prior to anticancer drug therapy). One skilled in the art will know of suitable higher or lower doses to which the current course of therapy can be adjusted such that drug therapy is optimized, *e.g.,* a subsequent dose that is at least about 1.5, 2, 2.5, 3, 3.5, 4, 4.5, 5, 5.5, 6, 6.5, 7, 7.5, 8, 8.5, 9, 9.5, 10, 15, 20, 25, 30, 35, 40, 45, 50, or 100-fold higher or lower than the current dose.

In certain other aspects of the methods for optimizing therapy, less than about 50% inhibition of activation (*e.g.,* phosphorylation) of one, two, three, four, five, six, or more oncogenic fusion protein (*e.g.,* BCR-ABL) levels and/or signal transduction pathway component (*e.g.,* CRKL, JAK2, STAT5) levels in a subject indicates a lack of compliance by the subject to the course of therapy (*e.g.,* the subject is not taking the anticancer drug regularly or as directed by a physician) and/or the existence of possible side-effects or toxicity associated with the course of therapy. In these embodiments, it is recommended that the current course of therapy be carefully monitored (*e.g.,* by a physician or other caregiver) for compliance or that a different course of therapy be administered (*e.g.,* the current course of therapy be changed by switching to a different anticancer drug) in order to increase compliance and/or to prevent or reduce the risk of side-effects. In certain instances, less than about 49%, 48%, 47%, 46%, 45%, 44%, 43%, 42%, 41%, 40%, 35%, 30%, 25%, 20%, 15%, 10%, or 5% inhibition of activation of one, two, three, four, five, six, or more oncogenic fusion protein (*e.g.,* BCR-ABL) levels and/or signal transduction pathway component (*e.g.,* CRKL, JAK2, STAT5) levels in a subject indicates a lack of compliance by the subject to the course of therapy and/or the existence of possible side-effects or toxicity associated with the course of therapy. In some embodiments, the percent inhibition of activation of oncogenic fusion protein and/or signal transduction molecule levels can be determined by calculating a ratio of activated to total oncogenic fusion protein levels (*e.g.,* ratio of phospho/total BCR-ABL protein levels) and/or a ratio of activated to total signal transduction pathway component levels (*e.g.,* ratio of phospho/total CRKL, JAK2, or STAT5 protein levels), optionally with respect to the levels of one or more control proteins (*e.g.,* full-length BCR and/or ABL for BCR-ABL), and comparing the calculated phospho/total ratio to a ratio of the same calculated for the subject at an earlier time (*e.g.,* at an earlier time while on anticancer drug therapy or at a point in time prior to anticancer drug therapy).

In further aspects of the methods described herein for optimizing therapy, greater than about 80% inhibition of activation (*e.g.,* phosphorylation) of one, two, three, four, five, six, or more oncogenic fusion protein (*e.g.,* BCR-ABL) levels and/or signal transduction pathway component (*e.g.,* CRKL, JAK2, STAT5) levels in a subject indicates that the subject is on the correct therapy at the correct dose. In certain instances, greater than about 81 %, 82%, 83%, 84%, 85%, 86%, 87%, 88%, 89%, 90%, 91%, 92%, 93%, 94%, 95%, 96%, 97%, 98%, 99%, or 100% inhibition of activation (*e.g.,* phosphorylation) of one, two, three, four, five, six, or more oncogenic fusion protein (*e.g.,* BCR-ABL) levels and/or signal transduction pathway component (*e.g.,* CRKL, JAK2, STAT5) levels in a subject indicates that the subject is on the correct anticancer drug therapy at the correct dose. In some embodiments, the percent inhibition of activation of oncogenic fusion protein and/or signal transduction molecule levels can be determined by calculating a ratio of activated to total oncogenic fusion protein levels (*e.g.,* ratio of phospho/total BCR-ABL protein levels) and/or a ratio of activated to total signal transduction pathway component levels (*e.g.,* ratio of phospho/total CRKL, JAK2, or STAT5 protein levels), optionally with respect to the levels of one or more control proteins (*e.g.,* full-length BCR and/or ABL for BCR-ABL), and then comparing the calculated phospho/total ratio to a ratio of the same calculated for the subject at an earlier time (*e.g.,* at an earlier time while on anticancer drug therapy or at a point in time prior to anticancer drug therapy).

In other aspects of the methods described herein for optimizing therapy, activation of an alternative signal transduction pathway indicates a need to change or adjust the current course of therapy (*e.g.,* switch to a different anticancer drug). As a non-limiting example, in instances where the subject is on Gleevec^{®} therapy, activation (*e.g.,* phosphorylation) of an alternative signal transduction pathway such as Src indicates a need to switch the subject to therapy with Sprycel^{®} or Tasigna^{®}.

In another aspect, the present invention provides a method for selecting a suitable anticancer drug for the treatment of a cancer, the method comprising:
(a) isolating cells of a cancer after administration of an anticancer drug, or prior to incubation with an anticancer drug;
(b) lysing the isolated cells to produce a cellular extract;
(c) determining a level of expression and/or activation (*e.g.,* phosphorylation) of an oncogenic fusion protein in the cellular extract using an assay described herein; and
(d) determining whether the anticancer drug is suitable or unsuitable for the treatment of the cancer by comparing the level of expression and/or activation detected for the oncogenic fusion protein with a reference expression and/or activation profile generated in the absence of the anticancer drug.

In a preferred embodiment, the method for selecting a suitable anticancer drug for the treatment of a cancer comprises:
(a) isolating cells of a cancer after administration of an anticancer drug, or prior to incubation with an anticancer drug;
(b) lysing the isolated cells to produce a cellular extract;
(c) determining a level of expression and/or activation (*e.g.,* phosphorylation) of an oncogenic fusion protein in the cellular extract using an assay comprising a dilution series of capture antibodies specific for the oncogenic fusion protein, wherein the capture antibodies are restrained on a solid support;
(d) comparing the level of expression and/or activation detected for the oncogenic fusion protein with a reference expression and/or activation profile generated in the absence of the anticancer drug; and
(e) indicating that the anticancer drug is suitable for the treatment of the cancer when the level of expression and/or activation detected for the oncogenic fusion protein is changed (*e.g.,* substantially decreased) compared to the reference expression and/or activation profile.

In some embodiments, the methods of the present invention may be useful to aid or assist in the selection of a suitable anticancer drug for the treatment of a cancer such as, *e.g.,* a hematological malignancy. In other embodiments, the methods of the present invention may be useful for improving the selection of a suitable anticancer drug for the treatment of a cancer such as, *e.g.,* a hematological malignancy. In certain embodiments, the method further or alternatively comprises the step of indicating that the anticancer drug is unsuitable for the treatment of the cancer when the level of expression and/or activation detected for the oncogenic fusion protein is not changed (*e.g.,* not substantially decreased) compared to the reference expression and/or activation profile. In further embodiments, one or more signal transduction molecules present in the cellular extract are detected in addition to one or more oncogenic fusion proteins, and the anticancer drug is determined to be suitable or unsuitable based on this "molecular profile."

In yet another aspect, the present invention provides a method for identifying the response of a cancer to treatment with an anticancer drug, the method comprising:
(a) isolating cells of a cancer after administration of an anticancer drug, or prior to incubation with an anticancer drug;
(b) lysing the isolated cells to produce a cellular extract;
(c) determining a level of expression and/or activation (*e.g.,* phosphorylation) of an oncogenic fusion protein in the cellular extract using an assay described herein; and
(d) identifying the cancer as responsive or non-responsive to treatment with the anticancer drug by comparing the level of expression and/or activation detected for the oncogenic fusion protein with a reference expression and/or activation profile generated in the absence of the anticancer drug.

In a preferred embodiment, the method for identifying the response of a cancer to treatment with an anticancer drug comprises:
(a) isolating cells of a cancer after administration of an anticancer drug, or prior to incubation with an anticancer drug;
(b) lysing the isolated cells to produce a cellular extract;
(c) determining a level of expression and/or activation (*e.g.,* phosphorylation) of an oncogenic fusion protein in the cellular extract using an assay comprising a dilution series of capture antibodies specific for the oncogenic fusion protein, wherein the capture antibodies are restrained on a solid support;
(d) comparing the level of expression and/or activation detected for the oncogenic fusion protein with a reference expression and/or activation profile generated in the absence of the anticancer drug; and
(e) indicating that the cancer is responsive to treatment with the anticancer drug when the level of expression and/or activation detected for the oncogenic fusion protein is changed (*e.g.,* substantially decreased) compared to the reference expression and/or activation profile.

In some embodiments, the methods of the present invention may be useful to aid or assist in the identification of the response of a cancer such as, *e.g.,* a hematological malignancy, to treatment with an anticancer drug. In other embodiments, the methods of the present invention may be useful for improving the identification of the response of a cancer such as, *e.g.,* a hematological malignancy, to treatment with an anticancer drug. In certain embodiments, the method further or alternatively comprises the step of indicating that the cancer is non-responsive to treatment with the anticancer drug when the level of expression and/or activation detected for the oncogenic fusion protein is not changed (*e.g.,* not substantially decreased) compared to the reference expression and/or activation profile. In further embodiments, one or more signal transduction molecules present in the cellular extract are detected in addition to one or more oncogenic fusion proteins, and the cancer is identified as responsive or non-responsive to treatment based on this "molecular profile."

In still yet another aspect, the present invention provides a method for predicting the response of a subject having cancer to treatment with an anticancer drug, the method comprising:
(a) isolating cells of a cancer after administration of an anticancer drug, or prior to incubation with an anticancer drug;
(b) lysing the isolated cells to produce a cellular extract;
(c) determining a level of expression and/or activation (*e.g.,* phosphorylation) of an oncogenic fusion protein in the cellular extract using an assay described herein; and
(d) predicting the likelihood that the subject will respond to treatment with the anticancer drug by comparing the level of expression and/or activation detected for the oncogenic fusion protein with a reference expression and/or activation profile generated in the absence of the anticancer drug.

In a preferred embodiment, the method for predicting the response of a subject having cancer to treatment with an anticancer drug comprises:
(a) isolating cells of a cancer after administration of an anticancer drug, or prior to incubation with an anticancer drug;
(b) lysing the isolated cells to produce a cellular extract;
(c) determining a level of expression and/or activation (*e.g.,* phosphorylation) of an oncogenic fusion protein in the cellular extract using an assay comprising a dilution series of capture antibodies specific for the oncogenic fusion protein, wherein the capture antibodies are restrained on a solid support;
(d) comparing the level of expression and/or activation detected for the oncogenic fusion protein with a reference expression and/or activation profile generated in the absence of the anticancer drug; and
(e) indicating that the subject will likely respond to treatment with the anticancer drug when the level of expression and/or activation detected for the oncogenic fusion protein is changed (*e.g.,* substantially decreased) compared to the reference expression and/or activation profile.

In some embodiments, the methods of the present invention may be useful to aid or assist in the prediction of a subject's likelihood of responding to treatment with an anticancer drug for a cancer such as, *e.g.,* a hematological malignancy. In other embodiments, the methods of the present invention may be useful for improving the prediction of a subject's likelihood of responding to treatment with an anticancer drug for a cancer such as, *e.g.,* a hematological malignancy. In certain embodiments, the method further or alternatively comprises the step of indicating that the subject will not likely respond to treatment with the anticancer drug when the level of expression and/or activation detected for the oncogenic fusion protein is not changed (*e.g.,* not substantially decreased) compared to the reference expression and/or activation profile. In further embodiments, one or more signal transduction molecules present in the cellular extract are detected in addition to one or more oncogenic fusion proteins, and the likelihood that the subject will respond to treatment is predicted based on this "molecular profile."

In a further aspect, the present invention provides a method for determining whether a subject having cancer is resistant to treatment with an anticancer drug, the method comprising:
(a) isolating cells of a cancer after administration of an anticancer drug, or prior to incubation with an anticancer drug;
(b) lysing the isolated cells to produce a cellular extract;
(c) determining a level of expression and/or activation (*e.g.,* phosphorylation) of an oncogenic fusion protein in the cellular extract using an assay described herein; and
(d) determining whether the subject is resistant or sensitive to treatment with the anticancer drug by comparing the level of expression and/or activation detected for the oncogenic fusion protein with a reference expression and/or activation profile generated in the absence of the anticancer drug or in the presence of the anticancer drug at an earlier time.

In a preferred embodiment, the method for determining whether a subject having cancer is resistant to treatment with an anticancer drug comprises:
(a) isolating cells of a cancer after administration of an anticancer drug, or prior to incubation with an anticancer drug;
(b) lysing the isolated cells to produce a cellular extract;
(c) determining a level of expression and/or activation (*e.g.,* phosphorylation) of an oncogenic fusion protein in the cellular extract using an assay comprising a dilution series of capture antibodies specific for the oncogenic fusion protein, wherein the capture antibodies are restrained on a solid support;
(d) comparing the level of expression and/or activation detected for the oncogenic fusion protein with a reference expression and/or activation profile generated in the absence of the anticancer drug or in the presence of the anticancer drug at an earlier time; and
(e) indicating that the subject is resistant to treatment with the anticancer drug when the level of expression and/or activation detected for the oncogenic fusion protein is not changed (*e.g.,* not substantially decreased) compared to the reference expression and/or activation profile.

In some embodiments, the methods of the present invention may be useful to aid or assist in the identification of a subject having cancer who is resistant to treatment with an anticancer drug or in the determination of whether a subject having cancer is resistant to treatment with an anticancer drug, wherein the subject has a cancer such as, *e.g.,* a hematological malignancy. In other embodiments, the methods of the present invention may be useful for improving the identification of a subject having cancer who is resistant to treatment with an anticancer drug or the determination of whether a subject having cancer is resistant to treatment with an anticancer drug, wherein the subject has a cancer such as, *e.g.,* a hematological malignancy.

In certain embodiments, the method further or alternatively comprises the step of indicating that the subject is sensitive to treatment with the anticancer drug when the level of expression and/or activation (*e.g*., phosphorylation) detected for the oncogenic fusion protein is changed (*e.g*., substantially decreased) compared to the reference expression or activation profile. Non-limiting examples of reasons why a subject having cancer would be resistant to treatment with an anticancer drug include the presence of one or more mutations in the oncogenic fusion protein of interest (*e.g*., BCR-ABL), non-compliance with the therapeutic regimen, and/or administration of a suboptimal drug dose. With regard to a suboptimal drug dose of the anticancer drug, the method can further comprise the step of increasing the next or subsequent dose of the anticancer drug administered to the subject. In further embodiments, one or more signal transduction molecules present in the cellular extract are detected in addition to one or more oncogenic fusion proteins, and the subject is identified as resistant or sensitive to treatment based on this "molecular profile."

In particular embodiments, the expression (*e.g.,* total) level and/or activation (*e.g.,* phosphorylation) level of the oncogenic fusion protein or signal transduction molecule is considered to be "changed" in the presence of an anticancer drug when it is at least about 5%, 10%, 15%, 20%, 25%, 30%, 35%, 40%, 45%, 50%, 55%, 60%, 65%, 70%, 75%, 80%, 85%, 90%, or 95% more or less expressed or activated than in the absence of the anticancer drug. In one embodiment, the expression (*e.g.,* total) level and/or activation (*e.g.,* phosphorylation) level of the oncogenic fusion protein or signal transduction molecule is considered to be "substantially decreased" in the presence of an anticancer drug when it is at least about 50%, 55%, 60%, 65%, 70%, 75%, 80%, 85%, 90%, or 95% less expressed or activated than in the absence of the anticancer drug. In further embodiments, the expression (*e.g*., total) level and/or activation (*e.g*., phosphorylation) level of the oncogenic fusion protein or signal transduction molecule is considered to be "substantially decreased" in the presence of an anticancer drug (1) when there is a change from high or strong expression and/or activation of the oncogenic fusion protein or signal transduction molecule without the anticancer drug to medium, weak, low, or very weak expression and/or activation of the oncogenic fusion protein or signal transduction molecule with the anticancer drug, or (2) when there is a change from medium expression and/or activation of the oncogenic fusion protein or signal transduction molecule without the anticancer drug to weak, low, or very weak expression and/or activation of the oncogenic fusion protein or signal transduction molecule with the anticancer drug.

In some embodiments, the expression level and/or activation level of the oncogenic fusion protein or signal transduction molecule is expressed as a relative fluorescence unit (RFU) value that corresponds to the signal intensity for a particular analyte of interest that is determined using, *e.g.,* a proximity assay such as the Collaborative Proximity Immunoassay (COPIA) described herein. In other embodiments, the expression level and/or activation level of the oncogenic fusion protein or signal transduction molecule is expressed as "-", "±", "+", "++", "+++", or "++++" that corresponds to increasing signal intensity for a particular analyte of interest that is determined using, *e.g.,* a proximity assay such as COPIA. In some instances, an undetectable or minimally detectable level of expression or activation of a particular analyte of interest that is determined using, *e.g.,* a proximity assay such as COPIA, may be expressed as "-" or "±". In other instances, a low level of expression or activation of a particular analyte of interest that is determined using, *e.g.,* a proximity assay such as COPIA, may be expressed as "+". In yet other instances, a moderate level of expression or activation of a particular analyte of interest that is determined using, *e.g.,* a proximity assay such as COPIA, may be expressed as "++". In still yet other instances, a high level of expression or activation of a particular analyte of interest that is determined using, *e.g.,* a proximity assay such as COPIA, may be expressed as "+++". In further instances, a very high level of expression or activation of a particular analyte of interest that is determined using, *e.g.,* a proximity assay such as COPIA, may be expressed as "++++".

In yet other embodiments, the expression level and/or activation level of the oncogenic fusion protein or signal transduction molecule is quantitated by calibrating or normalizing the RFU value that is determined using, *e.g.,* a proximity assay such as COPIA, against a standard curve generated for the particular analyte of interest. In certain instances, a computed units (CU) value can be calculated based upon the standard curve. In other instances, the CU value can be expressed as "-", "±", "+", "++", "+++", or "++++" in accordance with the description above for signal intensity.

In certain embodiments, the expression or activation level of a particular analyte of interest, when expressed as "-", "±", "+", "++", "+++", or "++++", may correspond to a level of expression or activation that is at least about 1.5, 2, 2.5, 3, 3.5, 4, 4.5, 5, 5.5, 6, 6.5, 7, 7.5, 8, 8.5, 9, 9.5, 10, 15, 20, 25, 30, 35, 40, 45, 50, or 100-fold higher or lower (*e.g*., about 1.5-3, 2-3, 2-4, 2-5, 2-10, 2-20, 2-50, 3-5, 3-10, 3-20, 3-50, 4-5, 4-10, 4-20, 4-50, 5-10, 5-15, 5-20, or 5-50-fold higher or lower) than a reference expression level or activation level, *e.g*., when compared to a negative control such as an IgG control, when compared to a standard curve generated for the analyte of interest, when compared to a positive control such as a pan-CK control, when compared to an expression or activation level determined in the presence of an anticancer drug, and/or when compared to an expression or activation level determined in the absence of an anticancer drug. In some instances, the correlation is analyte-specific. As a non-limiting example, a "+" level of expression or activation determined using, *e.g.,* a proximity assay such as COPIA, may correspond to a 2-fold increase in expression or activation for one analyte and a 5-fold increase for another analyte when compared to a reference expression or activation level.

In particular embodiments, both total and activated (*e.g*., phosphorylated) oncogenic fusion protein (*e.g*., BCR-ABL) or signal transduction molecule levels are measured in the cellular extract in accordance with the antibody-based assays of the present invention and a ratio of activated to total oncogenic fusion protein levels (*e.g*., ratio of phospho/total BCR-ABL protein levels) or a ratio of activated to total signal transduction molecule levels (*e.g.,* ratio of phospho/total CRKL, JAK2, or STAT5 protein levels) can be calculated and then compared to a ratio of the same calculated based upon the reference expression and activation profiles generated in the absence of the anticancer drug.

In some embodiments, the reference expression or activation level of the oncogenic fusion protein or signal transduction molecule determined in step (c) is obtained from a normal cell such as a non-cancerous cell from a healthy individual not having a cancer such as a hematological malignancy. In certain other embodiments, the reference expression or activation level of the oncogenic fusion protein or signal transduction molecule determined in step (c) is obtained from a tumor cell from a sample (*e.g*., cellular extract) from a patient with a cancer such leukemia or lymphoma.

In some embodiments, the reference expression or activation level of the oncogenic fusion protein or signal transduction molecule determined in step (c) is obtained from a cell (*e.g.,* a tumor cell obtained from a patient sample) that is not treated with the anticancer drug. In particular embodiments, the cell that is not treated with the anticancer drug is obtained from the same sample that the isolated cell (*e.g.,* a test cell to be interrogated) used to produce the cellular extract is obtained. In certain instances, the presence of a lower level of expression or activation of the oncogenic fusion protein or signal transduction molecule compared to the reference expression or activation level indicates that the anticancer drug is suitable for the treatment of the cancer (*e.g.,* the tumor has an increased likelihood of response to the anticancer drug). In certain other instances, the presence of an identical, similar, or higher level of expression or activation of the oncogenic fusion protein or signal transduction molecule compared to the reference expression or activation level indicates that the anticancer drug is unsuitable for the treatment of the cancer (*e.g.,* the tumor has a decreased likelihood of response to the anticancer drug).

In alternative embodiments, the reference expression or activation level of the oncogenic fusion protein or signal transduction molecule determined in step (c) is obtained from a cell sensitive to the anticancer drug that is treated with the anticancer drug. In such embodiments, the presence of an identical, similar, or lower level of expression or activation of the oncogenic fusion protein or signal transduction molecule compared to the reference expression or activation level indicates that the anticancer drug is suitable for the treatment of the cancer (*e.g.,* the tumor has an increased likelihood of response to the anticancer drug). In certain other alternative embodiments, the reference expression or activation level of the oncogenic fusion protein or signal transduction molecule determined in step (c) is obtained from a cell resistant to the anticancer drug that is treated with the anticancer drug. In such embodiments, the presence of an identical, similar, or higher level of expression or activation of the oncogenic fusion protein or signal transduction molecule compared to the reference expression or activation level indicates that the anticancer drug is unsuitable for the treatment of the cancer (*e.g.,* the tumor has a decreased likelihood of response to the anticancer drug).

In certain embodiments, a higher level of expression or activation of the oncogenic fusion protein or signal transduction molecule determined in step (c) is considered to be present in a cellular extract when the expression or activation level is at least about 1.5, 2, 2.5, 3, 3.5, 4, 4.5, 5, 5.5, 6, 6.5, 7, 7.5, 8, 8.5, 9, 9.5, 10, 15, 20, 25, 30, 35, 40, 45, 50, or 100-fold higher (*e.g*., about 1.5-3, 2-3, 2-4, 2-5, 2-10, 2-20, 2-50, 3-5, 3-10, 3-20, 3-50, 4-5, 4-10, 4-20, 4-50, 5-10, 5-15, 5-20, or 5-50-fold higher) than the reference expression or activation level of the corresponding analyte in a cell (*e.g.,* a cancer cell obtained from a patient sample) not treated with the anticancer drug, in an anticancer drug-sensitive cell treated with the anticancer drug, or in an anticancer drug-resistant cell treated with the anticancer drug.

In other embodiments, a lower level of expression or activation of the oncogenic fusion protein or signal transduction molecule determined in step (c) is considered to be present in a cellular extract when the expression or activation level is at least about 1.5, 2, 2.5, 3, 3.5, 4, 4.5, 5, 5.5, 6, 6.5, 7, 7.5, 8, 8.5, 9, 9.5, 10, 15, 20, 25, 30, 35, 40, 45, 50, or 100-fold lower (*e.g*., about 1.5-3, 2-3, 2-4, 2-5, 2-10, 2-20, 2-50, 3-5, 3-10, 3-20, 3-50, 4-5, 4-10, 4-20, 4-50, 5-10, 5-15, 5-20, or 5-50-fold lower) than the reference expression or activation level of the corresponding analyte in a cell (*e.g.,* a cancer cell obtained from a patient sample) not treated with the anticancer drug, in an anticancer drug-sensitive cell treated with the anticancer drug, or in an anticancer drug-resistant cell treated with the anticancer drug.

### A. Antibody Arrays

In one aspect, the present invention provides an array having superior dynamic range comprising a plurality of dilution series of capture antibodies specific for one or more analytes in a cellular extract, wherein the capture antibodies are restrained on a solid support.

In some embodiments, the cellular extract is prepared from a whole blood, urine, sputum, bronchial lavage fluid, tear, nipple aspirate, lymph, saliva, and/or fine needle aspirate (FNA) sample. As a non-limiting example, a whole blood sample is first separated into a plasma or serum fraction and a cellular fraction (*i.e.,* cell pellet). The cellular fraction typically contains red blood cells, white blood cells (leukocytes), and/or circulating cells of a solid tumor such as circulating tumor cells (CTCs), circulating endothelial cells (CECs), circulating endothelial progenitor cells (CEPCs), cancer stem cells (CSCs), and combinations thereof. Isolated cells present in the cellular fraction may be lysed to thereby transform the isolated cells into a cellular extract by any technique known in the art.

In some instances, the cellular extract comprises an extract of circulating cells of a solid tumor. The circulating cells are typically isolated from a patient sample using one or more separation methods including, for example, immunomagnetic separation (*see, e.g.,* Racila et al., Proc. Natl. Acad. Sci. USA, 95:4589-4594 (1998); Bilkenroth et al., Int. J. Cancer, 92:577-582 (2001)), microfluidic separation (*see, e.g.,* Mohamed et al., IEEE Trans. Nanobiosci., 3:251-256 (2004); Lin et al., Abstract No. 5147, 97th AACR Annual Meeting, Washington, D.C. (2006)), FACS (*see, e.g.,* Mancuso et al., Blood, 97:3658-3661 (2001)), density gradient centrifugation (*see, e.g.,* Baker et al., Clin. Cancer Res., 13:4865-4871 (2003)), and depletion methods (*see, e.g.,* Meye et al., Int. J. Oncol., 21:521-530 (2002)).

In other instances, the cellular extract comprises an extract of leukocytes such as granulocytes (polymorphonuclear leukocytes), which include, *e.g*., neutrophils, basophils, and eosinophils; agranulocytes (mononuclear leukocytes), which include, *e.g*., peripheral blood mononuclear cells such as lymphocytes and monocytes, and macrophages; and mixtures thereof. Leukocytes can be isolated from whole blood using any separation method known in the art, including, *e.g*., Ficoll-HyPaque density-gradient centrifugation, hypotonic lysis of red blood cells, and the use of density gradient media such as Lymphoprep™ and Polymorphprep™ (Axis-Shield; Oslo, Norway).

In certain embodiments, the isolated leukocytes, circulating cells, or other cells (*e.g.,* cells obtained from a solid tumor via fine needle aspirate) can be stimulated *in vitro* with one or more growth factors before, during, and/or after incubation with one or more anticancer drugs of interest. Stimulatory growth factors include, but are not limited to, epidermal growth factor (EGF), heregulin (HRG), TGF-α, PIGF, angiopoietin (Ang), NRG1, PGF, TNF-α, VEGF, PDGF, IGF, FGF, HGF, cytokines, and the like. In other instances, the isolated cells can be lysed, *e.g*., following growth factor stimulation and/or anticancer drug treatment, to produce the cellular extract (*e.g.,* cell lysate) using any technique known in the art. Preferably, the cell lysis is initiated between about 1-360 minutes after growth factor stimulation, and more preferably at two different time intervals: (1) at about 1-5 minutes after growth factor stimulation; and (2) between about 30-180 minutes after growth factor stimulation. Alternatively, the cell lysate can be stored at -80°C until use. Protocols for the isolation, stimulation, and lysis of circulating cells are described in PCT Publication No. WO 2008/036802. Protocols for the preparation of tumor cell extracts from tissue, biopsy, or primary cultures are described in PCT Publication No. WO 2009/108637.

In certain embodiments, the anticancer drug comprises an anti-signaling agent (*i.e.,* a cytostatic drug) such as a monoclonal antibody or a tyrosine kinase inhibitor; an anti-proliferative agent; a chemotherapeutic agent (*i.e*., a cytotoxic drug); a hormonal therapeutic agent; a radiotherapeutic agent; a vaccine; and/or any other compound with the ability to reduce or abrogate the uncontrolled growth of aberrant cells such as cancerous cells. In some embodiments, the isolated cells are treated with one or more anti-signaling agents, anti-proliferative agents, and/or hormonal therapeutic agents in combination with at least one chemotherapeutic agent.

Examples of anti-signaling agents include, without limitation, monoclonal antibodies such as trastuzumab (Herceptin^{®}), alemtuzumab (Campath^{®}), bevacizumab (Avastin^{®}), cetuximab (Erbitux^{®}), gemtuzumab (Mylotarg^{®}), panitumumab (Vectibix™), rituximab (Rituxan^{®}), and tositumomab (BEXXAR^{®}); tyrosine kinase inhibitors such as imatinib mesylate (Gleevec^{®}), nilotinib (Tasigna^{®}), dasatinib (Sprycel^{®}), bosutinib (SKI-606), gefitinib (Iressa^{®}), sunitinib (Sutent^{®}), erlotinib (Tarceva^{®}), lapatinib (GW-572016; Tykerb^{®}), canertinib (CI 1033), semaxinib (SU5416), vatalanib (PTK787/ZK222584), sorafenib (BAY 43-9006; Nexavar^{®}), leflunomide (SU101), and vandetanib (ZACTIMA™; ZD6474); and combinations thereof.

Exemplary anti-proliferative agents include mTOR inhibitors such as sirolimus (rapamycin), temsirolimus (CCI-779), and everolimus (RAD001); Akt inhibitors such as 1L6-hydroxymethyl-chiro-inositol-2-(R)-2-O-methyl-3-O-octadecyl-*sn*-glycerocarbonate, 9-methoxy-2-methylellipticinium acetate, 1,3-dihydro-1-(1-((4-(6-phenyl-1H-imidazo[4,5-g]quinoxalin-7-yl)phenyl)methyl)-4-piperidinyl)-2H-benzimidazol-2-one, 10-(4'-(N-diethylamino)butyl)-2-chlorophenoxazine, 3-formylchromone thiosemicarbazone (Cu(II)Cl₂ complex), API-2, a 15-mer peptide derived from amino acids 10-24 of the proto-oncogene TCL1 (Hiromura et al., J. Biol. Chem., 279:53407-53418 (2004), KP372-1, and the compounds described in Kozikowski et al., J. Am. Chem. Soc., 125:1144-1145 (2003) and Kau et al., Cancer Cell, 4:463-476 (2003); and combinations thereof.

Non-limiting examples of chemotherapeutic agents include platinum-based drugs (*e.g.,* oxaliplatin, cisplatin, carboplatin, spiroplatin, iproplatin, satraplatin, *etc*.), alkylating agents (*e.g*., cyclophosphamide, ifosfamide, chlorambucil, busulfan, melphalan, mechlorethamine, uramustine, thiotepa, nitrosoureas, *etc*.), anti-metabolites (*e.g.,* 5-fluorouracil, azathioprine, 6-mercaptopurine, methotrexate, leucovorin, capecitabine, cytarabine, floxuridine, fludarabine, gemcitabine (Gemzar^{®}), pemetrexed (ALIMTA^{®}), raltitrexed, *etc.),* plant alkaloids (*e.g.,* vincristine, vinblastine, vinorelbine, vindesine, podophyllotoxin, paclitaxel (Taxol^{®}), docetaxel (Taxotere^{®}), *etc*.), topoisomerase inhibitors (*e.g*., irinotecan, topotecan, amsacrine, etoposide (VP16), etoposide phosphate, teniposide, *etc*.), antitumor antibiotics (*e.g.,* doxorubicin, adriamycin, daunorubicin, epirubicin, actinomycin, bleomycin, mitomycin, mitoxantrone, plicamycin, *etc*.), pharmaceutically acceptable salts thereof, stereoisomers thereof, derivatives thereof, analogs thereof, and combinations thereof.

Examples of hormonal therapeutic agents include, without limitation, aromatase inhibitors (*e.g*., aminoglutethimide, anastrozole (Arimidex^{®}), letrozole (Femara^{®}), vorozole, exemestane (Aromasin^{®}), 4-androstene-3,6,17-trione (6-OXO), 1,4,6-androstatrien-3,17-dione (ATD), formestane (Lentaron^{®}), *etc*.), selective estrogen receptor modulators (*e.g.,* bazedoxifene, clomifene, fulvestrant, lasofoxifene, raloxifene, tamoxifen, toremifene, *etc*.), steroids (*e.g*., dexamethasone), finasteride, and gonadotropin-releasing hormone agonists (GnRH) such as goserelin, pharmaceutically acceptable salts thereof, stereoisomers thereof, derivatives thereof, analogs thereof, and combinations thereof

Non-limiting examples of cancer vaccines include ANYARA from Active Biotech, DCVax-LB from Northwest Biotherapeutics, EP-2101 from IDM Pharma, GV1001 from Pharmexa, IO-2055 from Idera Pharmaceuticals, INGN 225 from Introgen Therapeutics and Stimuvax from Biomira/Merck.

Examples of radiotherapeutic agents include, but are not limited to, radionuclides such as ⁴⁷Sc, ⁶⁴Cu, ⁶⁷Cu, ⁸⁹Sr, ⁸⁶Y, ⁸⁷Y, ⁹⁰Y, ¹⁰⁵Rh, ¹¹¹Ag, ¹¹¹In, ^{117m}Sn, ¹⁴⁹Pm, ¹⁵³Sm, ¹⁶⁶Ho, ¹⁷⁷Lu, ¹⁸⁶Re, ¹⁸⁸Re, ²¹¹At, and ²¹²Bi, optionally conjugated to antibodies directed against tumor antigens.

In particular embodiments, the one or more analytes present in the cellular extract comprise one or a plurality of oncogenic fusion proteins, alone or in combination with one or a plurality of signal transduction molecules. Non-limiting examples of oncogenic fusion proteins and signal transduction molecules of interest are described above.

In some embodiments, each dilution series of capture antibodies comprises a series of descending capture antibody concentrations. In certain instances, the capture antibodies are serially diluted at least 2-fold (*e.g.,* 2, 5, 10, 20, 50, 100, 500, or 1000-fold) to produce a dilution series comprising a set number (*e.g*., 2, 3, 4, 5, 6, 7, 8, 9, 10, 15, 20, 25, or more) of descending capture antibody concentrations which are spotted onto the array. Preferably, at least 2, 3, 4, 5, or 6 replicates of each capture antibody dilution are spotted onto the array.

In other embodiments, the solid support comprises glass (*e.g.,* a glass slide), plastic, chips, pins, filters, beads, paper, membrane (*e.g*., nylon, nitrocellulose, polyvinylidene fluoride (PVDF), *etc*.), fiber bundles, or any other suitable substrate. In a preferred embodiment, the capture antibodies are restrained (*e.g*., via covalent or non-covalent interactions) on glass slides coated with a nitrocellulose polymer such as, for example, FAST^{®} Slides, which are commercially available from Whatman Inc. (Florham Park, NJ).

As a non-limiting example, an addressable microarray of the present invention may comprise a capture antibody dilution series to determine the activation state of BCR-ABL in a cellular extract, in which the capture antibody is directed to the BCR domain of the BCR-ABL fusion protein and the detection antibodies (*e.g*., both the activation state-dependent and activation state-independent antibodies) are directed to the ABL domain. In an alternative embodiment, both the capture and activation state-independent antibodies are directed to the BCR domain of the BCR-ABL fusion protein and the activation state-dependent antibody is directed to the ABL domain. The arrays may further comprise a plurality of different capture antibodies directed to additional fusion proteins and/or signal transduction molecules in a series of descending concentrations (*i.e.,* serial dilutions), wherein the capture antibodies are coupled to the surface of the solid support in different addressable locations.

One skilled in the art will appreciate that the array can be any configuration that allows discrete signals for each of the activated oncogenic fusion proteins and/or signal transduction molecules to be detected. For example, the array can be a line or a grid of distinct regions (*e.g*., dots or spots) on the support surface, where each region contains a different capture antibody or capture agent (i*.e.,* to bind the capture tag present on the capture antibody). The array can be configured for use in methods where the activation states of a plurality of oncogenic fusion proteins and/or signal transduction molecules are detected in a single, multiplex assay. In various embodiments, the plurality comprises at least 2, 3, 4, 5, 6, 7, 8, 9, 10, 15, 20, 25, 30, 35, 40, 45, 50, or more oncogenic fusion proteins and/or signal transduction molecules. In particular embodiments, the plurality comprises the BCR-ABL fusion protein in combination with at least 1, 2, 3, 4, 5, 6, 7, 8, 9, 10, 15, 20, 25, 30, 35, 40, 45, 50, or more additional oncogenic fusion proteins and/or signal transduction molecules.

### B. Proximity Dual Detection Assays

In particular aspects, the assays of the present invention for detecting the activation state of a particular analyte of interest in a cellular extract of leukocytes or other cell types such as circulating cells of a solid tumor is a multiplex, high-throughput proximity (*i.e.,* three-antibody) assay having superior dynamic range.

As a non-limiting example, in situations where the analyte is a single protein (*e.g*., EGFR), the three antibodies used in the proximity assay can comprise: (1) a capture antibody specific for the analyte; (2) a detection antibody specific for an activated form of the analyte (*i.e.,* activation state-dependent antibody); and (3) a detection antibody which detects the total amount of the analyte (*i.e.,* activation state-independent antibody). The activation state-dependent antibody is capable of detecting, for example, the phosphorylation, ubiquitination, and/or complexation state of the analyte. The activation state-independent antibody is generally capable of detecting both the activated and non-activated forms of the analyte.

As another non-limiting example, in situations where the analyte is a fusion protein (*e.g.,* BCR-ABL) containing a first domain corresponding to one protein (*e.g.,* BCR) that is fused to a second domain corresponding to another protein (*e.g*., ABL), the three antibodies used in the proximity assay can comprise: (1) a capture antibody specific for the first domain of the fusion protein; (2) a detection antibody specific for an activated form of the second domain of the fusion protein (*i.e.,* activation state-dependent antibody); and (3) a detection antibody which detects the total amount of the fusion protein by specifically binding to the second domain of the fusion protein regardless of its activation state (*i.e*., activation state-independent antibody). The activation state-dependent antibody is capable of detecting, for example, the phosphorylation, ubiquitination, and/or complexation state of the fusion protein. The activation state-independent antibody is generally capable of detecting both the activated and non-activated forms of the fusion protein.

In a preferred aspect, the present invention provides a method for performing a multiplex, high-throughput immunoassay having superior dynamic range, the method comprising:
(a) incubating a cellular extract with one or a plurality of dilution series of capture antibodies specific for one or more fusion proteins to form a plurality of captured fusion proteins, wherein the capture antibodies are restrained on a solid support, wherein each fusion protein comprises a first domain corresponding to a first protein and a second domain corresponding to a second, different protein, and wherein the capture antibodies are specific for the first domain of the fusion proteins;
(b) incubating the plurality of captured fusion proteins with detection antibodies specific for the second domain of the fusion proteins to form a plurality of detectable captured fusion proteins, wherein the detection antibodies comprise:
   (1) a plurality of activation state-independent antibodies labeled with a facilitating moiety, and
   (2) a plurality of activation state-dependent antibodies labeled with a first member of a signal amplification pair,
   wherein the facilitating moiety generates an oxidizing agent which channels to and reacts with the first member of the signal amplification pair;
(c) incubating the plurality of detectable captured fusion proteins with a second member of the signal amplification pair to generate an amplified signal; and
(d) detecting the amplified signal generated from the first and second members of the signal amplification pair.

In one alternative aspect, the method for performing a multiplex, high-throughput immunoassay having superior dynamic range comprises:
(a) incubating a cellular extract with one or a plurality of dilution series of capture antibodies specific for one or more fusion proteins to form a plurality of captured fusion proteins, wherein the capture antibodies are restrained on a solid support, wherein each fusion protein comprises a first domain corresponding to a first protein and a second domain corresponding to a second, different protein, and wherein the capture antibodies are specific for the first domain of the fusion proteins;
(b) incubating the plurality of captured fusion proteins with detection antibodies to form a plurality of detectable captured fusion proteins, wherein the detection antibodies comprise:
   (1) a plurality of activation state-independent antibodies labeled with a facilitating moiety, wherein the activation state-independent antibodies are specific for the first domain of the fusion proteins, and
   (2) a plurality of activation state-dependent antibodies labeled with a first member of a signal amplification pair, wherein the activation state-dependent antibodies are specific for the second domain of the fusion proteins,
   wherein the facilitating moiety generates an oxidizing agent which channels to and reacts with the first member of the signal amplification pair;
(c) incubating the plurality of detectable captured fusion proteins with a second member of the signal amplification pair to generate an amplified signal; and
(d) detecting the amplified signal generated from the first and second members of the signal amplification pair.

In another alternative aspect, the method for performing a multiplex, high-throughput immunoassay having superior dynamic range comprises:
(a) incubating a cellular extract with one or a plurality of dilution series of capture antibodies specific for one or more fusion proteins to form a plurality of captured fusion proteins, wherein the capture antibodies are restrained on a solid support, wherein each fusion protein comprises a first domain corresponding to a first protein and a second domain corresponding to a second, different protein, and wherein the capture antibodies are specific for the first domain of the fusion proteins;
(b) incubating the plurality of captured fusion proteins with detection antibodies to form a plurality of detectable captured fusion proteins, wherein the detection antibodies comprise:
   (1) a plurality of activation state-independent antibodies labeled with a facilitating moiety, wherein the activation state-independent antibodies are specific for the sequence, site, or point of fusion between the first and second domains of the fusion proteins (*i.e.,* junction antibodies), and
   (2) a plurality of activation state-dependent antibodies labeled with a first member of a signal amplification pair, wherein the activation state-dependent antibodies are specific for the second domain of the fusion proteins,
   wherein the facilitating moiety generates an oxidizing agent which channels to and reacts with the first member of the signal amplification pair;
(c) incubating the plurality of detectable captured fusion proteins with a second member of the signal amplification pair to generate an amplified signal; and
(d) detecting the amplified signal generated from the first and second members of the signal amplification pair.

In certain embodiments, one or more signal transduction molecules present in the cellular extract are detected in addition to one or more fusion proteins. Examples of signal transduction molecules of interest are described above and include, without limitation, receptor tyrosine kinases, non-receptor tyrosine kinases, and/or tyrosine kinase signaling cascade components. The signal transduction molecules can be detected using the methods described herein, except that all three antibodies (*i.e.,* the capture antibody and both detection antibodies) are directed to the same protein, or using any method known to one of skill in the art. In addition, the signal transduction molecules can be detected using the single detection (*i.e.,* two-antibody) assays described in PCT Publication No. WO 2008/036802. In particular embodiments, one or more of the signal transduction molecules present in the cellular extract are detected in conjunction with one or more fusion proteins using the immunoassays and arrays described herein.

In some instances, the cellular extract is incubated with capture antibodies already restrained on a solid support. In other instances, the cellular extract is first incubated with capture antibodies in solution and then contacted with a solid support to immobilize the captured analytes, *e.g*., via capture tags present on the capture antibodies which interact with capture agents bound to the solid support.

In some embodiments, the detection antibodies are incubated with analytes that are bound to capture antibodies in solution or restrained on a solid support. In certain instances, the cellular extract comprising a plurality of analytes is first incubated with the detection antibodies in solution and then contacted with capture antibodies in solution or restrained on a solid support. In certain other instances, the cellular extract comprising a plurality of analytes is first incubated with capture antibodies and detection antibodies in solution and then contacted with a solid support to immobilize the antibody-analyte complexes, *e.g*., via capture tags present on the capture antibodies or detection antibodies which interact with capture agents bound to the solid support. Prior to the detecting step, the immobilized complexes can be washed to remove uncomplexed antibodies, the washed complexes can be sequentially released from the support surface, and proximity channeling for each of the analytes being assayed can be detected by a suitable method as described herein.

In embodiments where the support surface comprises capture agents restrained in an array, the incubating step can comprise contacting the cellular extract comprising a plurality of analytes in solution with the capture antibodies and detection antibodies, using an excess of all three antibodies to drive the reaction to completion. In one variation of the method, the resulting antibody-analyte complexes are attached to a solid phase and washed to remove unbound antibodies. As depicted in Figure 2 of PCT Publication No. WO 2008/036802, the capture antibody **1** can comprise a capture tag **10**. The complexes are attached to a solid phase **12** via a capture agent **11** that is adhered to the solid phase and binds the capture tag, thereby immobilizing the complex. The immobilized complex is washed with a suitable buffer, and then released from the solid phase by the addition of a releasing agent **13**. The releasing agent may function by any mechanism that results in the release of the washed complex. In one embodiment, the capture tag comprises a cleavable site that is recognized and cleaved by the releasing agent. In another embodiment, depicted in Figure 2, the releasing agent competes with the capture tag for binding to the capture agent. For example, the capture agent may be a first oligonucleotide that hybridizes with a partially complementary oligonucleotide (*i.e.,* the capture tag) attached to the capture antibody; and the releasing agent may be an oligonucleotide that is fully complementary to the capture agent, resulting in strand displacement and release of the washed complex from the solid phase. Other examples of suitable capture tags/capture agents/releasing agents that can be used include, but are not limited to, 2,4-dinitrophenol (DNP)/anti-DNP antibody/2,4-DNP lysine; T2/anti-T3 antibody/T3; ouabain/anti-digoxin antibody/digoxin; and dethiobiotin/streptavidin/biotin (*see, e.g.,* Ishikawa et al., J. Clin. Lab Anal., 12:98-107 (1998)).

After the washed complex is released from the solid phase, it is either: (1) contacted with a support surface comprising capture molecules restrained in an array that specifically bind capture tags on the capture antibody, or (2) dissociated, and the dissociated detection antibodies are contacted with a support surface comprising capture agents that specifically bind capture tags on the detection antibodies. Figure 2 of PCT Publication No. WO 2008/036802 depicts the embodiment where the washed complex is dissociated and the dissociated detection antibodies are contacted with the support surface **14**. The support surface comprises a plurality of capture molecules restrained in an "addressable" or "zip code" array. Each distinct region of the array comprises a unique capture agent **9** that specifically binds the capture tag **8** present on the activation state-independent detection antibody **2** or the activation state-dependent antibody **3**, thereby restraining and organizing the tagged detection antibodies in the array. In a preferred embodiment, the capture agents and capture tags are oligonucleotides that specifically hybridize to each other. Addressable arrays comprising oligonucleotide capture molecules are well known in the art (*see*, *e.g.,* Keramas et al., Lab Chip, 4:152-158 (2004); Delrio-Lafreniere et al., Diag. Microbiol. Infect. Dis., 48:23-31 (2004)).

The presence of the detection antibodies at each distinct region of the array can be directly or indirectly detected with a moiety such as a facilitating moiety or a first member of a signal amplification pair. Examples of moieties that can be directly detected include fluorophores, chromophores, colloidal gold, colored latex, *etc.* In one embodiment, both moieties are independently selected fluorophores. Any pair of fluorophores that provide a distinguishable readout while in close proximity to each other can be used, such as, for example, Cy3/Cy5, Cy5/phycoerthrin, and the like. Alternatively, if an oligonucleotide addressable array is used, both moieties can be the same fluorophore delivered to different zip codes. Laser scanning confocal microscopy can be used to detect fluorophore moieties that are adhered on the array. In assays where the complexes are released from the array prior to detection, such as in strand displacement assays, suitable methods for detecting the fluorophore moieties include capillary flow confocal laser induced fluorescence, nano-HPLC, micro-capillary electrophoresis, *etc.*

In some embodiments, the activation state-independent antibodies further comprise a detectable moiety. In such instances, the amount of the detectable moiety is correlative to the amount of one or more of the analytes in the cellular extract. Examples of detectable moieties include, but are not limited to, fluorescent labels, chemically reactive labels, enzyme labels, radioactive labels, and the like. Preferably, the detectable moiety is a fluorophore such as an Alexa Fluor^{®} dye (*e.g.,* Alexa Fluor^{®} 647), fluorescein, fluorescein isothiocyanate (FITC), Oregon Green™; rhodamine, Texas red, tetrarhodamine isothiocynate (TRITC), a CyDye™ fluor (*e.g.,* Cy2, Cy3, Cy5), and the like. The detectable moiety can be coupled directly or indirectly to the activation state-independent antibodies using methods well known in the art.

In certain instances, the activation state-independent antibodies are directly labeled with the facilitating moiety. The facilitating moiety can be coupled to the activation state-independent antibodies using methods well-known in the art. A suitable facilitating moiety for use in the present invention includes any molecule capable of generating an oxidizing agent which channels to (*i.e.,* is directed to) and reacts with (*i.e.,* binds, is bound by, or forms a complex with) another molecule in proximity (*i*.*e*., spatially near or close) to the facilitating moiety. Examples of facilitating moieties include, without limitation, enzymes such as glucose oxidase (GO) or any other enzyme that catalyzes an oxidation/reduction reaction involving molecular oxygen (O₂) as the electron acceptor, and photosensitizers such as methylene blue, rose bengal, porphyrins, squarate dyes, phthalocyanines, and the like. Non-limiting examples of oxidizing agents include hydrogen peroxide (H₂O₂), a singlet oxygen, and any other compound that transfers oxygen atoms or gains electrons in an oxidation/reduction reaction. Preferably, in the presence of a suitable substrate (*e.g*., glucose, light, *etc*.), the facilitating moiety (*e.g.,* glucose oxidase, photosensitizer, *etc*.) generates an oxidizing agent (*e.g.,* hydrogen peroxide (H₂O₂), single oxygen, *etc.)* which channels to and reacts with the first member of the signal amplification pair (*e.g.*, horseradish peroxidase (HRP), hapten protected by a protecting group, an enzyme inactivated by thioether linkage to an enzyme inhibitor, *etc*.) when the two moieties are in proximity to each other.

The preparation of sulfhydryl-modified dextran molecules and their use in making conjugates between an antibody and a facilitating moiety such as glucose oxidase (GO) is described in PCT Publication No. WO 2009/108637.

In certain other instances, the activation state-independent antibodies are indirectly labeled with the facilitating moiety via hybridization between an oligonucleotide linker conjugated to the activation state-independent antibodies and a complementary oligonucleotide linker conjugated to the facilitating moiety. The oligonucleotide linkers can be coupled to the facilitating moiety or to the activation state-independent antibodies using methods well-known in the art. In some embodiments, the oligonucleotide linker conjugated to the facilitating moiety has 100% complementarity to the oligonucleotide linker conjugated to the activation state-independent antibodies. In other embodiments, the oligonucleotide linker pair comprises at least one, two, three, four, five, six, or more mismatch regions, *e.g*., upon hybridization under stringent hybridization conditions. One skilled in the art will appreciate that activation state-independent antibodies specific for different analytes can either be conjugated to the same oligonucleotide linker or to different oligonucleotide linkers.

The length of the oligonucleotide linkers that are conjugated to the facilitating moiety or to the activation state-independent antibodies can vary. In general, the linker sequence can be at least about 5, 10, 15, 20, 25, 30, 35, 40, 45, 50, 75, or 100 nucleotides in length. Typically, random nucleic acid sequences are generated for coupling. As a non-limiting example, a library of oligonucleotide linkers can be designed to have three distinct contiguous domains: a spacer domain; signature domain; and conjugation domain. Preferably, the oligonucleotide linkers are designed for efficient coupling without destroying the function of the facilitating moiety or activation state-independent antibodies to which they are conjugated.

The oligonucleotide linker sequences can be designed to prevent or minimize any secondary structure formation under a variety of assay conditions. Melting temperatures are typically carefully monitored for each segment within the linker to allow their participation in the overall assay procedures. Generally, the range of melting temperatures of the segment of the linker sequence is no greater than 5°C. Computer algorithms (*e.g*., OLIGO 6.0) for determining the melting temperature, secondary structure, and hairpin structure under defined ionic concentrations can be used to analyze each of the three different domains within each linker. The overall combined sequences can also be analyzed for their structural characterization and their comparability to other conjugated oligonucleotide linker sequences, *e.g*., whether they will hybridize under stringent hybridization conditions to a complementary oligonucleotide linker.

The spacer region of the oligonucleotide linker provides adequate separation of the conjugation domain from the oligonucleotide crosslinking site. The conjugation domain functions to link molecules labeled with a complementary oligonucleotide linker sequence to the conjugation domain via nucleic acid hybridization. The nucleic acid-mediated hybridization can be performed either before or after antibody-analyte (*i.e.,* antigen) complex formation, providing a more flexible assay format. Unlike many direct antibody conjugation methods, linking relatively small oligonucleotides to antibodies or other molecules has minimal impact on the specific affinity of antibodies towards their target analyte or on the function of the conjugated molecules.

In some embodiments, the signature sequence domain of the oligonucleotide linker can be used in complex multiplexed protein assays. Multiple antibodies can be conjugated with oligonucleotide linkers with different signature sequences. In multiplex immunoassays, reporter oligonucleotide sequences labeled with appropriate probes can be used to detect cross-hybridization between antibodies and their antigens in the multiplex assay format.

Oligonucleotide linkers can be conjugated to antibodies or other molecules using several different methods. For example, oligonucleotide linkers can be synthesized with a thiol group on either the 5' or 3' end. The thiol group can be deprotected using reducing agents (*e.g*., TCEP-HCl) and the resulting linkers can be purified by using a desalting spin column. The resulting deprotected oligonucleotide linkers can be conjugated to the primary amines of antibodies or other types of proteins using heterobifunctional cross linkers such as SMCC. Alternatively, 5'-phosphate groups on oligonucleotides can be treated with watersoluble carbodiimide EDC to form phosphate esters and subsequently coupled to amine-containing molecules. In certain instances, the diol on the 3'-ribose residue can be oxidized to aldehyde groups and then conjugated to the amine groups of antibodies or other types of proteins using reductive amination. In certain other instances, the oligonucleotide linker can be synthesized with a biotin modification on either the 3' or 5' end and conjugated to streptavidin-labeled molecules.

Oligonucleotide linkers can be synthesized using any of a variety of techniques known in the art, such as those described in Usman et al., J. Am. Chem. Soc., 109:7845 (1987); Scaringe et al., Nucl. Acids Res., 18:5433 (1990); Wincott et al., Nucl. Acids Res., 23:2677-2684 (1995); and Wincott et al., Methods Mol. Bio., 74:59 (1997). In general, the synthesis of oligonucleotides makes use of common nucleic acid protecting and coupling groups, such as dimethoxytrityl at the 5'-end and phosphoramidites at the 3'-end. Suitable reagents for oligonucleotide synthesis, methods for nucleic acid deprotection, and methods for nucleic acid purification are known to those of skill in the art.

The preparation and use of oligonucleotide-conjugated antibodies for simultaneous detection of total and phosphorylated analytes is described in PCT Publication No. WO 2008/036802.

In certain instances, the activation state-dependent antibodies are directly labeled with the first member of the signal amplification pair. The signal amplification pair member can be coupled to the activation state-dependent antibodies using methods well-known in the art. In certain other instances, the activation state-dependent antibodies are indirectly labeled with the first member of the signal amplification pair via binding between a first member of a binding pair conjugated to the activation state-dependent antibodies and a second member of the binding pair conjugated to the first member of the signal amplification pair. The binding pair members (*e.g*., biotin/streptavidin) can be coupled to the signal amplification pair member or to the activation state-dependent antibodies using methods well-known in the art. Examples of signal amplification pair members include, but are not limited to, peroxidases such horseradish peroxidase (HRP), catalase, chloroperoxidase, cytochrome c peroxidase, eosinophil peroxidase, glutathione peroxidase, lactoperoxidase, myeloperoxidase, thyroid peroxidase, deiodinase, and the like. Other examples of signal amplification pair members include haptens protected by a protecting group and enzymes inactivated by thioether linkage to an enzyme inhibitor.

The capture antibodies, activation state-independent antibodies, and activation state-dependent antibodies are typically selected to minimize competition between them with respect to analyte binding (*i.e*., all antibodies can simultaneously bind their corresponding fusion proteins or signal transduction molecules).

In one example of proximity channeling, the facilitating moiety is glucose oxidase (GO) and the first member of the signal amplification pair is horseradish peroxidase (HRP). When the GO is contacted with a substrate such as glucose, it generates an oxidizing agent (*i.e*., hydrogen peroxide (H₂O₂)). If the HRP is within channeling proximity to the GO, the H₂O₂ generated by the GO is channeled to and complexes with the HRP to form an HRP-H₂O₂ complex, which, in the presence of the second member of the signal amplification pair (*e.g.,* a chemiluminescent substrate such as luminol or isoluminol or a fluorogenic substrate such as tyramide (*e.g*., biotin-tyramide), homovanillic acid, or 4-hydroxyphenyl acetic acid), generates an amplified signal. Methods of using GO and HRP in a proximity assay are described in, *e.g.,* Langry *et al*., U.S. Dept. of Energy Report No. UCRL-ID-136797 (1999). When biotin-tyramide is used as the second member of the signal amplification pair, the HRP-H₂O₂ complex oxidizes the tyramide to generate a reactive tyramide radical that covalently binds nearby nucleophilic residues. The activated tyramide is either directly detected or detected upon the addition of a signal-detecting reagent such as, for example, a streptavidin-labeled fluorophore or a combination of a streptavidin-labeled peroxidase and a chromogenic reagent. Examples of fluorophores suitable for use in the present invention include, but are not limited to, an Alexa Fluor^{®} dye (*e.g.,* Alexa Fluor^{®} 555), fluorescein, fluorescein isothiocyanate (FITC), Oregon Green™; rhodamine, Texas red, tetrarhodamine isothiocynate (TRITC), a CyDye™ fluor (*e.g.,* Cy2, Cy3, Cy5), and the like. The streptavidin label can be coupled directly or indirectly to the fluorophore or peroxidase using methods well-known in the art. Non-limiting examples of chromogenic reagents suitable for use in the present invention include 3,3',5,5'-tetramethylbenzidine (TMB), 3,3'-diaminobenzidine (DAB), 2,2'-azino-bis(3-ethylbenzothiazoline-6-sulfonic acid) (ABTS), 4-chloro-1-napthol (4CN), and/or porphyrinogen.

In another example of proximity channeling, the facilitating moiety is a photosensitizer and the first member of the signal amplification pair is a large molecule labeled with multiple haptens that are protected with protecting groups that prevent binding of the haptens to a specific binding partner (*e.g.,* ligand, antibody, *etc*.). For example, the signal amplification pair member can be a dextran molecule labeled with protected biotin, coumarin, and/or fluorescein molecules. Suitable protecting groups include, but are not limited to, phenoxy-, analino-, olefin-, thioether-, and selenoether-protecting groups. Additional photosensitizers and protected hapten molecules suitable for use in the proximity assays of the present invention are described in U.S. Patent No. 5,807,675. When the photosensitizer is excited with light, it generates an oxidizing agent (*i.e.,* singlet oxygen). If the hapten molecules are within channeling proximity to the photosensitizer, the singlet oxygen generated by the photosensitizer is channeled to and reacts with thioethers on the protecting groups of the haptens to yield carbonyl groups (ketones or aldehydes) and sulphinic acid, releasing the protecting groups from the haptens. The unprotected haptens are then available to specifically bind to the second member of the signal amplification pair (*e.g.,* a specific binding partner that can generate a detectable signal). For example, when the hapten is biotin, the specific binding partner can be an enzyme-labeled streptavidin. Exemplary enzymes include alkaline phosphatase, β-galactosidase, HRP, *etc.* After washing to remove unbound reagents, the detectable signal can be generated by adding a detectable (*e.g.,* fluorescent, chemiluminescent, chromogenic, *etc*.) substrate of the enzyme and detected using suitable methods and instrumentation known in the art. Alternatively, the detectable signal can be amplified using tyramide signal amplification and the activated tyramide either directly detected or detected upon the addition of a signal-detecting reagent as described above.

In yet another example of proximity channeling, the facilitating moiety is a photosensitizer and the first member of the signal amplification pair is an enzyme-inhibitor complex. The enzyme and inhibitor (*e.g*., phosphonic acid-labeled dextran) are linked together by a cleavable linker (*e.g*., thioether). When the photosensitizer is excited with light, it generates an oxidizing agent *(i.e.,* singlet oxygen). If the enzyme-inhibitor complex is within channeling proximity to the photosensitizer, the singlet oxygen generated by the photosensitizer is channeled to and reacts with the cleavable linker, releasing the inhibitor from the enzyme, thereby activating the enzyme. An enzyme substrate is added to generate a detectable signal, or alternatively, an amplification reagent is added to generate an amplified signal.

In a further example of proximity channeling, the facilitating moiety is HRP, the first member of the signal amplification pair is a protected hapten or an enzyme-inhibitor complex as described above, and the protecting groups comprise p-alkoxy phenol. The addition of phenylenediamine and H₂O₂ generates a reactive phenylene diimine which channels to the protected hapten or the enzyme-inhibitor complex and reacts with p-alkoxy phenol protecting groups to yield exposed haptens or a reactive enzyme. The amplified signal is generated and detected as described above (*see, e.g.,* U.S. Patent Nos. 5,532,138 and 5,445,944).

One skilled in the art will appreciate that binding partners other than antibodies can be used to immobilize and/or detect one or more analytes from a cellular extract in accordance with the proximity (*i.e*., three-antibody) assays described herein. Non-limiting examples of such binding partners include ligands or receptors of the analyte, substrates of the analyte, binding domains (*e.g.,* PTB, SH2, *etc*.), aptamers, and the like.

An exemplary protocol for performing the proximity assays described herein is provided in Example 1.

In another embodiment, the present invention provides kits for performing the proximity assays described above comprising: (a) a dilution series of a plurality of capture antibodies restrained on a solid support; and (b) a plurality of detection antibodies (*e.g*., activation state-independent antibodies and activation state-dependent antibodies). In some instances, the kits can further contain instructions for methods of using the kit to detect the activation states of one or a plurality of fusion proteins and/or signal transduction molecules. The kits may also contain any of the additional reagents described above with respect to performing the specific methods of the present invention such as, for example, first and second members of the signal amplification pair, tyramide signal amplification reagents, substrates for the facilitating moiety, wash buffers, *etc.*

### IV. Construction of Antibody Arrays

In certain aspects, the present invention provides antibody-based arrays for detecting the activation state of one or a plurality of fusion proteins in a cellular extract using a dilution series of capture antibodies restrained on a solid support. The arrays used in the assays of the present invention typically comprise a plurality of one or more different capture antibodies at a range of capture antibody concentrations that are coupled to the surface of a solid support in different addressable locations.

The solid support can comprise any suitable substrate for immobilizing proteins. Examples of solid supports include, but are not limited to, glass (*e.g.,* a glass slide), plastic, chips, pins, filters, beads (*e.g.,* magnetic beads, polystyrene beads, *etc*.), paper, membranes, fiber bundles, gels, metal, ceramics, and the like. Membranes such nylon (Biotrans^{™}, ICN Biomedicals, Inc. (Costa Mesa, CA); Zeta-Probe^{®}, Bio-Rad Laboratories (Hercules, CA)), nitrocellulose (Protran^{®}, Whatman Inc. (Florham Park, NJ)), and PVDF (Immobilon™, Millipore Corp. (Billerica, MA)) are suitable for use as solid supports in the arrays of the present invention. Preferably, the capture antibodies are restrained on glass slides coated with a nitrocellulose polymer, *e.g*., FAST^{®} Slides, which are commercially available from Whatman Inc. (Florham Park, NJ).

Particular aspects of the solid support which are desirable include the ability to bind large amounts of capture antibodies, the ability to bind capture antibodies with minimal denaturation, and the inability to bind other proteins. Another suitable aspect is that the solid support displays minimal "wicking" when antibody solutions containing capture antibodies are applied to the support. A solid support with minimal wicking allows small aliquots of capture antibody solution applied to the support to result in small, defined spots of immobilized capture antibody.

The capture antibodies are typically directly or indirectly (*e.g*., via capture tags) restrained on the solid support via covalent or non-covalent interactions (*e.g*., ionic bonds, hydrophobic interactions, hydrogen bonds, Van der Waals forces, dipole-dipole bonds). In some embodiments, the capture antibodies are covalently attached to the solid support using a homobifunctional or heterobifunctional crosslinker using standard crosslinking methods and conditions. Suitable crosslinkers are commercially available from vendors such as, *e.g.,* Pierce Biotechnology (Rockford, IL).

Methods for generating the arrays of the present invention include, but are not limited to, any technique used to construct protein or nucleic acid arrays. In some embodiments, the capture antibodies are spotted onto an array using a microspotter, which are typically robotic printers equipped with split pins, blunt pins, or ink jet printing. Suitable robotic systems for printing the antibody arrays described herein include the PixSys 5000 robot (Cartesian Technologies; Irvine, CA) with ChipMaker2 split pins (TeleChem International; Sunnyvale, CA) as well as other robotic printers available from BioRobics (Woburn, MA) and Packard Instrument Co. (Meriden, CT). Preferably, at least 2, 3, 4, 5, or 6 replicates of each capture antibody dilution are spotted onto the array.

Another method for generating the antibody arrays of the present invention comprises dispensing a known volume of a capture antibody dilution at each selected array position by contacting a capillary dispenser onto a solid support under conditions effective to draw a defined volume of liquid onto the support, wherein this process is repeated using selected capture antibody dilutions at each selected array position to create a complete array. The method may be practiced in forming a plurality of such arrays, where the solution-depositing step is applied to a selected position on each of a plurality of solid supports at each repeat cycle. A further description of such a method can be found, *e.g.,* in U.S. Patent No. 5,807,522.

In certain instances, devices for printing on paper can be used to generate the antibody arrays of the present invention. For example, the desired capture antibody dilution can be loaded into the printhead of a desktop jet printer and printed onto a suitable solid support (s*ee*, *e.g.,* Silzel et al., Clin. Chem., 44:2036-2043 (1998)).

In some embodiments, the array generated on the solid support has a density of at least about 5 spots/cm², and preferably at least about 10, 20, 30, 40, 50, 60, 70, 80, 90, 100, 110, 120, 130, 140, 150, 160, 170, 180, 190, 200, 210, 220, 230, 250, 275, 300, 325, 350, 375, 400, 425, 450, 475, 500, 550, 600, 650, 700, 750, 800, 850, 900, 950, 1000, 2000, 3000, 4000, 5000, 6000, 7000, 8000 or 9000, or 10,000 spots/cm².

In certain instances, the spots on the solid support each represents a different capture antibody. In certain other instances, multiple spots on the solid support represent the same capture antibody, *e.g*., as a dilution series comprising a series of descending capture antibody concentrations.

Additional examples of methods for preparing and constructing antibody arrays on solid supports are described in U.S. Patent Nos. 6,197,599, 6,777,239, 6,780,582, 6,897,073, 7,179,638, and 7,192,720; U.S. Patent Publication Nos. 20060115810, 20060263837, 20060292680, and 20070054326; and Varnum et al., Methods Mol. Biol., 264:161-172 (2004).

Methods for scanning antibody arrays are known in the art and include, without limitation, any technique used to scan protein or nucleic acid arrays. Microarray scanners suitable for use in the present invention are available from PerkinElmer (Boston, MA), Agilent Technologies (Palo Alto, CA), Applied Precision (Issaquah, WA), GSI Lumonics Inc. (Billerica, MA), and Axon Instruments (Union City, CA). As a non-limiting example, a GSI ScanArray3000 for fluorescence detection can be used with ImaGene software for quantitation.

### V. Drug Selection and Optimization for Cancer Therapy

In certain aspects, the present invention provides methods for the selection of appropriate therapies to down-regulate or shut down one or more deregulated signaling pathways. In certain other aspects, the present invention provides methods for optimizing therapy and/or reducing toxicity in a subject having cancer and receiving a course of therapy for the treatment of cancer. Thus, the present invention may be used to facilitate the design of personalized therapies based on the particular molecular signature provided by the collection of activated oncogenic fusion proteins and/or signal transduction proteins in a given patient's cancer or tumor.

Accordingly, in one particular aspect, the present invention provides a method for optimizing therapy and/or reducing toxicity in a subject having cancer and receiving a course of therapy for the treatment of cancer, the method comprising:
(a) isolating cancer cells after administration of an anticancer drug *(e.g.,* one or more tyrosine kinase inhibitors such as Gleevec^{®}, Tasigna^{®}, Sprycel^{®}, *etc*.);
(b) lysing the isolated cells to produce a cellular extract;
(c) measuring a level of expression and/or activation (*e.g*., phosphorylation) of an oncogenic fusion protein in the cellular extract using an assay described herein; and
(d) comparing the measured level of expression and/or activation of the oncogenic fusion protein to a level of expression and/or activation of the oncogenic fusion protein measured at an earlier time during the course of therapy; and
(e) determining a subsequent dose of the course of therapy for the subject or whether a different course of therapy should be administered to the subject based upon the comparison from step (d).

In particular embodiments, both total and activated (*e.g*., phosphorylated) oncogenic fusion protein (*e.g*., BCR-ABL) levels are measured in the cellular extract in accordance with the antibody-based assays of the present invention and a ratio of activated to total oncogenic fusion protein levels (*e.g*., ratio of phospho/total BCR-ABL protein levels) can be calculated and used to evaluate the course of therapy for a subject, *e.g*., by comparing the phospho/total ratio of oncogenic fusion protein levels to a ratio of the same calculated for the subject at an earlier time (*e.g.,* at an earlier time while on anticancer drug therapy or at a point in time prior to anticancer drug therapy).

In a related aspect, the present invention provides a method for optimizing therapy and/or reducing toxicity in a subject having cancer and receiving a course of therapy for the treatment of cancer, the method comprising:
(a) isolating cancer cells after administration of an anticancer drug (*e.g.,* one or more tyrosine kinase inhibitors such as Gleevec^{®}, Tasigna^{®}, Sprycel^{®}, *etc*.);
(b) lysing the isolated cells to produce a cellular extract;
(c) measuring a level of expression and/or activation (*e.g*., phosphorylation) of an oncogenic fusion protein and one or more signal transduction molecules in its pathway in the cellular extract using an assay described herein; and
(d) comparing the measured level of expression and/or activation of the oncogenic fusion protein and signal transduction molecules to a level of expression and/or activation of the oncogenic fusion protein and signal transduction molecules measured at an earlier time during the course of therapy; and
(e) determining a subsequent dose of the course of therapy for the subject or whether a different course of therapy should be administered to the subject based upon the comparison from step (d).

In particular embodiments, both total and activated (*e.g*., phosphorylated) oncogenic fusion protein *(e.g.,* BCR-ABL) levels and signal transduction pathway component (*e.g.,* CRKL, JAK2, STAT5) levels are measured in the cellular extract in accordance with the antibody-based assays of the present invention and a ratio of activated to total oncogenic fusion protein levels (*e.g*., ratio of phospho/total BCR-ABL protein levels) and a ratio of activated to total signal transduction pathway component levels (*e.g.*, ratio of phospho/total CRKL, JAK2, or STAT5 protein levels) can be calculated and used to evaluate the course of therapy for a subject, *e.g*., by comparing the phospho/total ratio of oncogenic fusion protein and signal transduction pathway component levels to a ratio of the same calculated for the subject at an earlier time (*e.g.,* at an earlier time while on anticancer drug therapy or at a point in time prior to anticancer drug therapy).

In another aspect, the present invention provides a method for selecting a suitable anticancer drug for the treatment of a cancer, the method comprising:
(a) isolating cells of a cancer after administration of an anticancer drug, or prior to incubation with an anticancer drug;
(b) lysing the isolated cells to produce a cellular extract;
(c) determining a level of expression and/or activation (*e.g*., phosphorylation) of an oncogenic fusion protein in the cellular extract using an assay described herein; and
(d) determining whether the anticancer drug is suitable or unsuitable for the treatment of the cancer by comparing the level of expression and/or activation detected for the oncogenic fusion protein with a reference expression and/or activation profile generated in the absence of the anticancer drug.

In a preferred embodiment, the method for selecting a suitable anticancer drug for the treatment of a cancer comprises:
(a) isolating cells of a cancer after administration of an anticancer drug, or prior to incubation with an anticancer drug;
(b) lysing the isolated cells to produce a cellular extract;
(c) determining a level of expression and/or activation (*e.g*., phosphorylation) of an oncogenic fusion protein in the cellular extract using an assay comprising a dilution series of capture antibodies specific for the oncogenic fusion protein, wherein the capture antibodies are restrained on a solid support;
(d) comparing the level of expression and/or activation detected for the oncogenic fusion protein with a reference expression and/or activation profile generated in the absence of the anticancer drug; and
(e) indicating that the anticancer drug is suitable for the treatment of the cancer when the level of expression and/or activation detected for the oncogenic fusion protein is changed (*e*.*g.*, substantially decreased) compared to the reference expression and/or activation profile.

In certain instances, the preferred embodiment may further comprise, *i.e.,* as step (f), or alternatively comprise, *i.e.,* as step (e), the step of indicating that the anticancer drug is unsuitable for the treatment of the cancer when the level of expression and/or activation detected for the oncogenic fusion protein is not changed (*e.g*., not substantially decreased) compared to the reference expression and/or activation profile. In other instances, one or more signal transduction molecules present in the cellular extract are detected in addition to one or more oncogenic fusion proteins, and the anticancer drug is determined to be suitable or unsuitable based on this "molecular profile."

In particular embodiments, both total and activated (*e.g*., phosphorylated) oncogenic fusion protein (*e.g*., BCR-ABL) levels are measured in the cellular extract in accordance with the antibody-based assays of the present invention and a ratio of activated to total oncogenic fusion protein levels (*e.g*., ratio of phospho/total BCR-ABL protein levels) can be calculated and used to determine whether the anticancer drug is suitable or unsuitable for the treatment of the cancer, *e.g.,* by comparing the phospho/total ratio of oncogenic fusion protein levels to a ratio of the same calculated based upon the reference expression and activation profiles that were generated in the absence of the anticancer drug.

In another aspect, the present invention provides a method for identifying the response of a cancer to treatment with an anticancer drug, the method comprising:
(a) isolating cells of a cancer after administration of an anticancer drug, or prior to incubation with an anticancer drug;
(b) lysing the isolated cells to produce a cellular extract;
(c) determining a level of expression and/or activation (*e.g*., phosphorylation) of an oncogenic fusion protein in the cellular extract using an assay described herein; and
(d) identifying the cancer as responsive or non-responsive to treatment with the anticancer drug by comparing the level of expression and/or activation detected for the oncogenic fusion protein with a reference expression and/or activation profile generated in the absence of the anticancer drug.

In a preferred embodiment, the method for identifying the response of a cancer to treatment with an anticancer drug comprises:
(a) isolating cells of a cancer after administration of an anticancer drug, or prior to incubation with an anticancer drug;
(b) lysing the isolated cells to produce a cellular extract;
(c) determining a level of expression and/or activation (*e.g*., phosphorylation) of an oncogenic fusion protein in the cellular extract using an assay comprising a dilution series of capture antibodies specific for the oncogenic fusion protein, wherein the capture antibodies are restrained on a solid support;
(d) comparing the level of expression and/or activation detected for the oncogenic fusion protein with a reference expression and/or activation profile generated in the absence of the anticancer drug; and
(e) indicating that the cancer is responsive to treatment with the anticancer drug when the level of expression and/or activation detected for the oncogenic fusion protein is changed (*e.g*., substantially decreased) compared to the expression and/or reference activation profile.

In certain instances, the preferred embodiment may further comprise, *i.e.,* as step (f), or alternatively comprise, *i.e*., as step (e), the step of indicating that the cancer is non-responsive to treatment with the anticancer drug when the level of expression and/or activation detected for the oncogenic fusion protein is not changed (*e.g*., not substantially decreased) compared to the reference expression and/or activation profile. In other instances, one or more signal transduction molecules present in the cellular extract are detected in addition to one or more oncogenic fusion proteins, and the cancer is identified as responsive or non-responsive to treatment based on this "molecular profile."

In particular embodiments, both total and activated (*e.g*., phosphorylated) oncogenic fusion protein (*e.g*., BCR-ABL) levels are measured in the cellular extract in accordance with the antibody-based assays of the present invention and a ratio of activated to total oncogenic fusion protein levels (*e.g*., ratio of phospho/total BCR-ABL protein levels) can be calculated and used to identify whether the cancer is responsive or non-responsive to treatment with the anticancer drug, *e.g.,* by comparing the phospho/total ratio of oncogenic fusion protein levels to a ratio of the same calculated based upon the reference expression and activation profiles that were generated in the absence of the anticancer drug.

In yet another aspect, the present invention provides a method for predicting the response of a subject having cancer to treatment with an anticancer drug, the method comprising:
(a) isolating cells of a cancer after administration of an anticancer drug, or prior to incubation with an anticancer drug;
(b) lysing the isolated cells to produce a cellular extract;
(c) determining a level of expression and/or activation (*e.g*., phosphorylation) of an oncogenic fusion protein in the cellular extract using an assay described herein; and
(d) predicting the likelihood that the subject will respond to treatment with the anticancer drug by comparing the level of expression and/or activation detected for the oncogenic fusion protein with a reference expression and/or activation profile generated in the absence of the anticancer drug.

In a preferred embodiment, the method for predicting the response of a subject having cancer to treatment with an anticancer drug comprises:
(a) isolating cells of a cancer after administration of an anticancer drug, or prior to incubation with an anticancer drug;
(b) lysing the isolated cells to produce a cellular extract;
(c) determining a level of expression and/or activation (*e.g*., phosphorylation) of an oncogenic fusion protein in the cellular extract using an assay comprising a dilution series of capture antibodies specific for the oncogenic fusion protein, wherein the capture antibodies are restrained on a solid support;
(d) comparing the level of expression and/or activation detected for the oncogenic fusion protein with a reference expression and/or activation profile generated in the absence of the anticancer drug; and
(e) indicating that the subject will likely respond to treatment with the anticancer drug when the level of expression and/or activation detected for the oncogenic fusion protein is changed (*e.g*., substantially decreased) compared to the reference expression and/or activation profile.

In certain instances, the preferred embodiment may further comprise, *i.e.,* as step (f), or alternatively comprise, *i.e.,* as step (e), the step of indicating that the subject will not likely respond to treatment with the anticancer drug when the level of expression and/or activation detected for the oncogenic fusion protein is not changed (*e.g*., not substantially decreased) compared to the reference expression and/or activation profile. In other instances, one or more signal transduction molecules present in the cellular extract are detected in addition to one or more oncogenic fusion proteins, and the likelihood that the subject will respond to treatment is predicted based on this "molecular profile."

In particular embodiments, both total and activated (*e.g*., phosphorylated) oncogenic fusion protein (*e.g*., BCR-ABL) levels are measured in the cellular extract in accordance with the antibody-based assays of the present invention and a ratio of activated to total oncogenic fusion protein levels (*e.g*., ratio of phospho/total BCR-ABL protein levels) can be calculated and used to predict whether the subject will have a likelihood of responding to treatment with the anticancer drug, *e.g.,* by comparing the phospho/total ratio of oncogenic fusion protein levels to a ratio of the same calculated based upon the reference expression and activation profiles that were generated in the absence of the anticancer drug.

In a further aspect, the present invention provides a method for determining whether a subject having cancer is resistant to treatment with an anticancer drug, the method comprising:
(a) isolating cells of a cancer after administration of an anticancer drug, or prior to incubation with an anticancer drug;
(b) lysing the isolated cells to produce a cellular extract;
(c) determining a level of expression and/or activation (*e.g*., phosphorylation) of an oncogenic fusion protein in the cellular extract using an assay described herein; and
(d) determining whether the subject is resistant or sensitive to treatment with the anticancer drug by comparing the level of expression and/or activation detected for the oncogenic fusion protein with a reference expression and/or activation profile generated in the absence of the anticancer drug or in the presence of the anticancer drug at an earlier time.

In a preferred embodiment, the method for determining whether a subject having cancer is resistant to treatment with an anticancer drug comprises:
(a) isolating cells of a cancer after administration of an anticancer drug, or prior to incubation with an anticancer drug;
(b) lysing the isolated cells to produce a cellular extract;
(c) determining a level of expression and/or activation (*e.g*., phosphorylation) of an oncogenic fusion protein in the cellular extract using an assay comprising a dilution series of capture antibodies specific for the oncogenic fusion protein, wherein the capture antibodies are restrained on a solid support;
(d) comparing the level of expression and/or activation detected for the oncogenic fusion protein with a reference expression and/or activation profile generated in the absence of the anticancer drug or in the presence of the anticancer drug at an earlier time; and
(e) indicating that the subject is resistant to treatment with the anticancer drug when the level of expression and/or activation detected for the oncogenic fusion protein is not changed (*e.g*., not substantially decreased) compared to the reference expression and/or activation profile.

In certain instances, the preferred embodiment may further comprise, *i.e.,* as step (f), or alternatively comprise, *i.e.,* as step (e), the step of indicating that the subject is sensitive to treatment with the anticancer drug when the level of expression and/or activation detected for the oncogenic fusion protein is changed (*e.g*., substantially decreased) compared to the reference expression and/or activation profile. In other instances, one or more signal transduction molecules present in the cellular extract are detected in addition to one or more oncogenic fusion proteins, and the subject is identified as resistant or sensitive to treatment based on this "molecular profile."

In particular embodiments, both total and activated (*e.g*., phosphorylated) oncogenic fusion protein (*e.g*., BCR-ABL) levels are measured in the cellular extract in accordance with the antibody-based assays of the present invention and a ratio of activated to total oncogenic fusion protein levels (*e.g*., ratio of phospho/total BCR-ABL protein levels) can be calculated and used to determine whether the subject is resistant or sensitive to treatment with the anticancer drug, *e.g.,* by comparing the phospho/total ratio of oncogenic fusion protein levels to a ratio of the same calculated based upon the reference expression and activation profiles that were generated in the absence of the anticancer drug or in the presence of the anticancer drug at an earlier time.

In some embodiments, the methods of the present invention may further comprise sending or reporting the results of step (d) to a clinician, *e.g*., an oncologist or a general practitioner. In other embodiments, the methods of the present invention may further comprise recording or storing the results of step (d) in a computer database or other suitable machine or device for storing information, *e.g.*, at a laboratory.

In some embodiments, the methods of the present invention may further comprise the step of obtaining a sample from a subject having cancer from which cells such as cancer cells are isolated. The sample may be obtained from a subject either before anticancer drug treatment (*e.g*., prior to incubation with an anticancer drug) or after administration of an anticancer drug *(e.g.,* at any time throughout the course of cancer treatment). Suitable samples include, but are not limited to, whole blood, plasma, serum, ductal lavage fluid, nipple aspirate, lymph (*e.g*., disseminated tumor cells of the lymph node), bone marrow aspirate, saliva, urine, stool (*i.e.,* feces), sputum, bronchial lavage fluid, tears, fine needle aspirate (*e.g*., harvested by random periareolar fine needle aspiration), any other bodily fluid, a tissue sample (*e.g.,* tumor tissue) such as a biopsy of a tumor (*e.g.,* needle biopsy) or a lymph node (*e.g*., sentinel lymph node biopsy), and cellular extracts thereof. In some embodiments, the sample is whole blood or a fractional component thereof such as plasma, serum, red blood cells, leukocytes such as peripheral blood mononuclear cells, and/or rare circulating cells. In particular embodiments, the sample is obtained by isolating leukocytes or circulating cells of a solid tumor from whole blood or a cellular fraction thereof using any technique known in the art. If isolated cells are obtained from a subject who has not received treatment with an anticancer drug, the isolated cells may be incubated *in vitro* under suitable conditions with one or a cocktail of anticancer drugs which target one or more of the analytes to be detected in step (c).

In certain embodiments, the cancer is a hematological malignancy (*e.g*., leukemia, lymphoma), osteogenic sarcoma (*e.g.,* Ewing sarcoma), soft tissue sarcoma (*e.g.,* DFSP, rhabdomyosarcoma), other soft tissue malignancy, papillary thyroid carcinoma, or prostate cancer. In particular embodiments, the cancer is caused by the formation of an oncogenic fusion protein due to a chromosomal translocation in the cancerous cells or tumor.

In some embodiments, the isolated cells are stimulated *in vitro* with growth factors as described herein. In other embodiments, the anticancer drug may comprise one or more of the therapeutic agents described herein, including but not limited to monoclonal antibodies, tyrosine kinase inhibitors, chemotherapeutic agents, hormonal therapeutic agents, radiotherapeutic agents, and vaccines.

In some embodiments, the activation state detected for the oncogenic fusion protein present in the cellular extract may be, *e.g.,* a phosphorylation state, a ubiquitination state, a complexation state, or combinations thereof. In other embodiments, the solid support may comprise, *e.g*., glass, plastic, chips, pins, filters, beads, paper, membrane, fiber bundles, and combinations thereof. In yet other embodiments, the capture antibodies are restrained on the solid support in an addressable array.

In certain embodiments, the assay in step (c) comprises:
(i) incubating (*e.g*., contacting) the cellular extract with the dilution series of capture antibodies to form a plurality of captured oncogenic fusion proteins (*e.g.,* to transform the oncogenic fusion proteins present in the cellular extract into complexes of captured oncogenic fusion proteins comprising the oncogenic fusion proteins and capture antibodies);
(ii) incubating (*e.g*., contacting) the plurality of captured oncogenic fusion proteins with detection antibodies comprising activation state-independent antibodies and activation state-dependent antibodies specific for the same or different domain of the oncogenic fusion protein to form a plurality of detectable captured oncogenic fusion proteins (*e.g.,* to transform the complexes of captured oncogenic fusion proteins into complexes of detectable captured oncogenic fusion proteins comprising the captured oncogenic fusion proteins and detection antibodies),
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(iii) incubating (*e.g*., contacting) the plurality of detectable captured analytes with a second member of the signal amplification pair to generate an amplified signal; and
(iv) detecting the amplified signal generated from the first and second members of the signal amplification pair.

The activation state-independent antibodies may be directly labeled with the facilitating moiety or indirectly labeled with the facilitating moiety, *e.g*., via hybridization between an oligonucleotide conjugated to the activation state-independent antibodies and a complementary oligonucleotide conjugated to the facilitating moiety. Similarly, the activation state-dependent antibodies may be directly labeled with the first member of the signal amplification pair or indirectly labeled with the first member of the signal amplification pair, *e.g.,* via binding between a first member of a binding pair conjugated to the activation state-dependent antibodies and a second member of the binding pair conjugated to the first member of the signal amplification pair. In certain instances, the first member of the binding pair is biotin and the second member of the binding pair is an avidin such as streptavidin or neutravidin.

In some embodiments, the facilitating moiety may be, for example, glucose oxidase (GO). In certain instances, the GO and the activation state-independent antibodies can be conjugated to a sulfhydryl-activated dextran molecule. The sulfhydryl-activated dextran molecule typically has a molecular weight of about 500kDa (*e.g.*, about 250, 300, 350, 400, 450, 500, 550, 600, 650, 700, or 750kDa). In other embodiments, the oxidizing agent may be, for example, hydrogen peroxide (H₂O₂). In yet other embodiments, the first member of the signal amplification pair may be, for example, a peroxidase such as horseradish peroxidase (HRP). In further embodiments, the second member of the signal amplification pair may be, for example, a tyramide reagent (*e.g*., biotin-tyramide). Preferably, the amplified signal is generated by peroxidase oxidization of biotin-tyramide to produce an activated tyramide (*e.g*., to transform the biotin-tyramide into an activated tyramide). The activated tyramide may be directly detected or indirectly detected, *e.g*., upon the addition of a signal-detecting reagent. Non-limiting examples of signal-detecting reagents include streptavidin-labeled fluorophores and combinations of streptavidin-labeled peroxidases and chromogenic reagents such as, *e.g*., 3,3',5,5'-tetramethylbenzidine (TMB).

In certain instances, the HRP and the activation state-dependent antibodies can be conjugated to a sulfhydryl-activated dextran molecule. The sulfhydryl-activated dextran molecule typically has a molecular weight of about 70kDa (*e.g*., about 40, 45, 50, 55, 60, 65, 70, 75, 80, 85, 90, 95, or 100kDa).

### VI. Production of Antibodies

The generation and selection of antibodies not already commercially available for analyzing the activation states of oncogenic fusion proteins or signal transduction molecules in a biological sample such as a cellular extract or lysate in accordance with the invention can be accomplished several ways. For example, one way is to express and/or purify a polypeptide of interest (*i.e*., antigen) using protein expression and purification methods known in the art, while another way is to synthesize the polypeptide of interest using solid phase peptide synthesis methods known in the art. *See, e.g.,* Guide to Protein Purification, Murray P. Deutcher, ed., Meth. Enzymol., Vol. 182 (1990); Solid Phase Peptide Synthesis, Greg B. Fields, ed., Meth. Enzymol., Vol. 289 (1997); Kiso et al., Chem. Pharm. Bull., 38:1192-99 (1990); Mostafavi et al., Biomed. Pept. Proteins Nucleic Acids, 1:255-60, (1995); and Fujiwara et al., Chem. Pharm. Bull., 44:1326-31 (1996). The purified or synthesized polypeptide can then be injected, for example, into mice or rabbits, to generate polyclonal or monoclonal antibodies. One skilled in the art will recognize that many procedures are available for the production of antibodies, for example, as described in Antibodies, A Laboratory Manual, Harlow and Lane, Eds., Cold Spring Harbor Laboratory, Cold Spring Harbor, N.Y. (1988). One skilled in the art will also appreciate that binding fragments or Fab fragments which mimic (*e.g*., retain the functional binding regions of) antibodies can also be prepared from genetic information by various procedures. *See, e.g.,* Antibody Engineering: A Practical Approach, Borrebaeck, Ed., Oxford University Press, Oxford (1995); and Huse et al., J. Immunol., 149:3914-3920 (1992).

In addition, numerous publications have reported the use of phage display technology to produce and screen libraries of polypeptides for binding to a selected target antigen (*see, e.g,* Cwirla et al., Proc. Natl. Acad. Sci. USA, 87:6378-6382 (1990); Devlin et al., Science, 249:404-406 (1990); Scott et al., Science, 249:386-388 (1990); and Ladner et al., U.S. Patent No. 5,571,698). A basic concept of phage display methods is the establishment of a physical association between a polypeptide encoded by the phage DNA and a target antigen. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target antigen bind to the target antigen and these phage are enriched by affinity screening to the target antigen. The identity of polypeptides displayed from these phage can be determined from their respective genomes. Using these methods, a polypeptide identified as having a binding affinity for a desired target antigen can then be synthesized in bulk by conventional means (*see, e.g.,* U.S. Patent No. 6,057,098).

The antibodies that are generated by these methods can then be selected by first screening for affinity and specificity with the purified polypeptide antigen of interest and, if required, comparing the results to the affinity and specificity of the antibodies with other polypeptide antigens that are desired to be excluded from binding. The screening procedure can involve immobilization of the purified polypeptide antigens in separate wells of microtiter plates. The solution containing a potential antibody or group of antibodies is then placed into the respective microtiter wells and incubated for about 30 minutes to 2 hours. The microtiter wells are then washed and a labeled secondary antibody (*e.g*., an anti-mouse antibody conjugated to alkaline phosphatase if the raised antibodies are mouse antibodies) is added to the wells and incubated for about 30 minutes and then washed. Substrate is added to the wells and a color reaction will appear where antibody to the immobilized polypeptide antigen is present.

The antibodies so identified can then be further analyzed for affinity and specificity. In the development of immunoassays for a target protein, the purified target protein acts as a standard with which to judge the sensitivity and specificity of the immunoassay using the antibodies that have been selected. Because the binding affinity of various antibodies may differ, *e.g*., certain antibody combinations may interfere with one another sterically, assay performance of an antibody may be a more important measure than absolute affinity and specificity of that antibody.

Those skilled in the art will recognize that many approaches can be taken in producing antibodies or binding fragments and screening and selecting for affinity and specificity for the various polypeptides of interest, but these approaches do not change the scope of the present invention.

### A. Polyclonal Antibodies

Polyclonal antibodies are preferably raised in animals by multiple subcutaneous (sc) or intraperitoneal (ip) injections of a polypeptide of interest and an adjuvant. It may be useful to conjugate the polypeptide of interest to a protein carrier that is immunogenic in the species to be immunized, such as, *e.g.,* keyhole limpet hemocyanin, serum albumin, bovine thyroglobulin, or soybean trypsin inhibitor using a bifunctional or derivatizing agent. Non-limiting examples of bifunctional or derivatizing agents include maleimidobenzoyl sulfosuccinimide ester (conjugation through cysteine residues), N-hydroxysuccinimide (conjugation through lysine residues), glutaraldehyde, succinic anhydride, SOCl₂, and R₁N=C=NR, wherein R and R₁ are different alkyl groups.

Animals are immunized against the polypeptide of interest or an immunogenic conjugate or derivative thereof by combining, *e.g*., 100 µg (for rabbits) or 5 µg (for mice) of the antigen or conjugate with 3 volumes of Freund's complete adjuvant and injecting the solution intradermally at multiple sites. One month later, the animals are boosted with about 1/5 to 1/10 the original amount of polypeptide or conjugate in Freund's incomplete adjuvant by subcutaneous injection at multiple sites. Seven to fourteen days later, the animals are bled and the serum is assayed for antibody titer. Animals are typically boosted until the titer plateaus. Preferably, the animal is boosted with the conjugate of the same polypeptide, but conjugation to a different immunogenic protein and/or through a different cross-linking reagent may be used. Conjugates can also be made in recombinant cell culture as fusion proteins. In certain instances, aggregating agents such as alum can be used to enhance the immune response.

### B. Monoclonal Antibodies

Monoclonal antibodies are generally obtained from a population of substantially homogeneous antibodies, *i.e.,* the individual antibodies comprising the population are identical except for possible naturally-occurring mutations that may be present in minor amounts. Thus, the modifier "monoclonal" indicates the character of the antibody as not being a mixture of discrete antibodies. For example, monoclonal antibodies can be made using the hybridoma method described by Kohler et al., Nature, 256:495 (1975) or by any recombinant DNA method known in the art (*see, e.g.,* U.S. Patent No. 4,816,567).

In the hybridoma method, a mouse or other appropriate host animal (*e.g*., hamster) is immunized as described above to elicit lymphocytes that produce or are capable of producing antibodies which specifically bind to the polypeptide of interest used for immunization. Alternatively, lymphocytes are immunized *in vitro*. The immunized lymphocytes are then fused with myeloma cells using a suitable fusing agent, such as polyethylene glycol, to form hybridoma cells (*see, e.g.,* Goding, Monoclonal Antibodies: Principles and Practice, Academic Press, pp. 59-103 (1986)). The hybridoma cells thus prepared are seeded and grown in a suitable culture medium that preferably contains one or more substances which inhibit the growth or survival of the unfused, parental myeloma cells. For example, if the parental myeloma cells lack the enzyme hypoxanthine guanine phosphoribosyl transferase (HGPRT), the culture medium for the hybridoma cells will typically include hypoxanthine, aminopterin, and thymidine (HAT medium), which prevent the growth of HGPRT-deficient cells.

Preferred myeloma cells are those that fuse efficiently, support stable high-level production of antibody by the selected antibody-producing cells, and/or are sensitive to a medium such as HAT medium. Examples of such preferred myeloma cell lines for the production of human monoclonal antibodies include, but are not limited to, murine myeloma lines such as those derived from MOPC-21 and MPC-11 mouse tumors (available from the Salk Institute Cell Distribution Center; San Diego, CA), SP-2 or X63-Ag8-653 cells (available from the American Type Culture Collection; Rockville, MD), and human myeloma or mouse-human heteromyeloma cell lines (*see, e.g.,* Kozbor, J. Immunol., 133:3001 (1984); and Brodeur et al., Monoclonal Antibody Production Techniques and Applications, Marcel Dekker, Inc., New York, pp. 51-63 (1987)).

The culture medium in which hybridoma cells are growing can be assayed for the production of monoclonal antibodies directed against the polypeptide of interest. Preferably, the binding specificity of monoclonal antibodies produced by hybridoma cells is determined by immunoprecipitation or by an *in vitro* binding assay, such as a radioimmunoassay (RIA) or an enzyme-linked immunoabsorbent assay (ELISA). The binding affinity of monoclonal antibodies can be determined using, *e.g.,* the Scatchard analysis of Munson et al., Anal. Biochem., 107:220 (1980).

After hybridoma cells are identified that produce antibodies of the desired specificity, affinity, and/or activity, the clones may be subcloned by limiting dilution procedures and grown by standard methods *(see, e.g.,* Goding, Monoclonal Antibodies: Principles and Practice, Academic Press, pp. 59-103 (1986)). Suitable culture media for this purpose include, for example, D-MEM or RPMI-1640 medium. In addition, the hybridoma cells may be grown *in vivo* as ascites tumors in an animal. The monoclonal antibodies secreted by the subclones can be separated from the culture medium, ascites fluid, or serum by conventional antibody purification procedures such as, for example, protein A-Sepharose, hydroxylapatite chromatography, gel electrophoresis, dialysis, or affinity chromatography.

DNA encoding the monoclonal antibodies can be readily isolated and sequenced using conventional procedures (*e.g*., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody, to induce the synthesis of monoclonal antibodies in the recombinant host cells. *See, e.g.,* Skerra et al., Curr. Opin. Immunol., 5:256-262 (1993); and Pluckthun, Immunol Rev., 130:151-188 (1992). The DNA can also be modified, for example, by substituting the coding sequence for human heavy chain and light chain constant domains in place of the homologous murine sequences *(see, e.g.,* U.S. Patent No. 4,816,567; and Morrison et al., Proc. Natl. Acad. Sci. USA, 81:6851 (1984)), or by covalently joining to the immunoglobulin coding sequence all or part of the coding sequence for a non-immunoglobulin polypeptide.

In a further embodiment, monoclonal antibodies or antibody fragments can be isolated from antibody phage libraries generated using the techniques described in, for example, McCafferty et al., Nature, 348:552-554 (1990); Clackson et al., Nature, 352:624-628 (1991); and Marks et al., J. Mol. Biol., 222:581-597 (1991). The production of high affinity (nM range) human monoclonal antibodies by chain shuffling is described in Marks et al., BioTechnology, 10:779-783 (1992). The use of combinatorial infection and *in vivo* recombination as a strategy for constructing very large phage libraries is described in Waterhouse et al., Nuc. Acids Res., 21:2265-2266 (1993). Thus, these techniques are viable alternatives to traditional monoclonal antibody hybridoma methods for the generation of monoclonal antibodies.

### C. Humanized Antibodies

Methods for humanizing non-human antibodies are known in the art. Preferably, a humanized antibody has one or more amino acid residues introduced into it from a source which is non-human. These non-human amino acid residues are often referred to as "import" residues, which are typically taken from an "import" variable domain. Humanization can be essentially performed by substituting the hypervariable region sequences of a non-human antibody for the corresponding sequences of a human antibody. *See, e.g.,* Jones et al., Nature, 321:522-525 (1986); Riechmann et al., Nature, 332:323-327 (1988); and Verhoeyen et al., Science, 239:1534-1536 (1988). Accordingly, such "humanized" antibodies are chimeric antibodies (*see, e.g.,* U.S. Patent No. 4,816,567), wherein substantially less than an intact human variable domain has been substituted by the corresponding sequence from a non-human species. In practice, humanized antibodies are typically human antibodies in which some hypervariable region residues and possibly some framework region (FR) residues are substituted by residues from analogous sites of rodent antibodies.

The choice of human variable domains, both light and heavy, to be used in making the humanized antibodies described herein is an important consideration for reducing antigenicity. According to the so-called "best-fit" method, the sequence of the variable domain of a rodent antibody is screened against the entire library of known human variable-domain sequences. The human sequence which is closest to that of the rodent is then accepted as the human FR for the humanized antibody (*see, e.g.,* Sims et al., J. Immunol., 151:2296 (1993); and Chothia et al., J. Mol. Biol., 196:901 (1987)). Another method uses a particular FR derived from the consensus sequence of all human antibodies of a particular subgroup of light or heavy chains. The same FR may be used for several different humanized antibodies (*see, e.g.,* Carter et al., Proc. Natl. Acad. Sci. USA, 89:4285 (1992); and Presta et al., J. Immunol., 151:2623 (1993)).

It is also important that antibodies be humanized with retention of high affinity for the antigen and other favorable biological properties. To achieve this goal, humanized antibodies can be prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e*., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and specifically involved in influencing antigen binding.

Various forms of humanized antibodies are contemplated in accordance with the present invention. For example, the humanized antibody can be an antibody fragment, such as a Fab fragment. Alternatively, the humanized antibody can be an intact antibody, such as an intact IgA, IgG, or IgM antibody.

### D. Human Antibodies

As an alternative to humanization, human antibodies can be generated. In some embodiments, transgenic animals (*e.g*., mice) can be produced that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. *See, e.g.,* Jakobovits et al., Proc. Natl. Acad. Sci. USA, 90:2551 (1993); Jakobovits et al., Nature, 362:255-258 (1993); Bruggermann et al., Year in Immun., 7:33 (1993); and U.S. Patent Nos. 5,591,669, 5,589,369, and 5,545,807.

Alternatively, phage display technology (*see, e.g.,* McCafferty et al., Nature, 348:552-553 (1990)) can be used to produce human antibodies and antibody fragments *in vitro*, using immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats as described in, *e.g.,* Johnson et al., Curr. Opin. Struct. Biol., 3:564-571 (1993). Several sources of V-gene segments can be used for phage display. *See, e.g.,* Clackson et al., Nature, 352:624-628 (1991). A repertoire of V genes from unimmunized human donors can be constructed and antibodies to a diverse array of antigens (including self-antigens) can be isolated essentially following the techniques described in Marks et al., J. Mol. Biol., 222:581-597 (1991); Griffith et al., EMBO J., 12:725-734 (1993); and U.S. Patent Nos. 5,565,332 and 5,573,905.

In certain instances, human antibodies can be generated by *in vitro* activated B cells as described in, *e.g.,* U.S. Patent Nos. 5,567,610 and 5,229,275.

### E. Antibody Fragments

Various techniques have been developed for the production of antibody fragments. Traditionally, these fragments were derived via proteolytic digestion of intact antibodies (*see, e.g.,* Morimoto et al., J. Biochem. Biophys. Meth., 24:107-117 (1992); and Brennan et al., Science, 229:81 (1985)). However, these fragments can now be produced directly using recombinant host cells. For example, the antibody fragments can be isolated from the antibody phage libraries discussed above. Alternatively, Fab'-SH fragments can be directly recovered from *E. coli* cells and chemically coupled to form F(ab')₂ fragments (*see, e.g.,* Carter et al., BioTechnology, 10:163-167 (1992)). According to another approach, F(ab')₂ fragments can be isolated directly from recombinant host cell culture. Other techniques for the production of antibody fragments will be apparent to those skilled in the art. In other embodiments, the antibody of choice is a single chain Fv fragment (scFv). *See, e.g.,* PCT Publication No. WO 93/16185; and U.S. Patent Nos. 5,571,894 and 5,587,458. The antibody fragment may also be a linear antibody as described, *e.g*., in U.S. Patent No. 5,641,870. Such linear antibody fragments may be monospecific or bispecific.

### F. Bispecific Antibodies

Bispecific antibodies are antibodies that have binding specificities for at least two different epitopes. Exemplary bispecific antibodies may bind to two different epitopes of the same polypeptide of interest. Other bispecific antibodies may combine a binding site for the polypeptide of interest with binding site(s) for one or more additional antigens. Bispecific antibodies can be prepared as full-length antibodies or antibody fragments (*e.g*., F(ab')₂ bispecific antibodies).

Methods for making bispecific antibodies are known in the art. Traditional production of full-length bispecific antibodies is based on the co-expression of two immunoglobulin heavy chain-light chain pairs, where the two chains have different specificities (*see*, *e.g.,* Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule is usually performed by affinity chromatography. Similar procedures are disclosed in PCT Publication No. WO 93/08829 and Traunecker et al., EMBO J., 10:3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy chain constant region (CH1) containing the site necessary for light chain binding present in at least one of the fusions. DNA encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains into one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. This asymmetric structure facilitates the separation of the desired bispecific compound from unwanted immunoglobulin chain combinations, as the presence of an immunoglobulin light chain in only one half of the bispecific molecule provides for a facile way of separation. *See, e.g.,* PCT Publication No. WO 94/04690 and Suresh et al., Meth. Enzymol., 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the CH3 domain of an antibody constant domain. In this method, one or more small amino acid side-chains from the interface of the first antibody molecule are replaced with larger side chains (*e.g*., tyrosine or tryptophan). Compensatory "cavities" of identical or similar size to the large side-chain(s) are created on the interface of the second antibody molecule by replacing large amino acid side-chains with smaller ones (*e.g*., alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Heteroconjugate antibodies can be made using any convenient cross-linking method. Suitable cross-linking agents and techniques are well-known in the art, and are disclosed in, *e.g*., U.S. Patent No. 4,676,980.

Suitable techniques for generating bispecific antibodies from antibody fragments are also known in the art. For example, bispecific antibodies can be prepared using chemical linkage. In certain instances, bispecific antibodies can be generated by a procedure in which intact antibodies are proteolytically cleaved to generate F(ab')₂ fragments *(see, e.g.,* Brennan et al., Science, 229:81 (1985)). These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody.

In some embodiments, Fab'-SH fragments can be directly recovered from *E. coli* and chemically coupled to form bispecific antibodies. For example, a fully humanized bispecific antibody F(ab')₂ molecule can be produced by the methods described in Shalaby et al., J. Exp. Med., 175: 217-225 (1992). Each Fab' fragment was separately secreted from *E. coli* and subjected to directed chemical coupling *in vitro* to form the bispecific antibody.

Various techniques for making and isolating bispecific antibody fragments directly from recombinant cell culture have also been described. For example, bispecific antibodies have been produced using leucine zippers. *See, e.g.,* Kostelny et al., J. Immunol., 148:1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy chain variable domain (VH) connected to a light chain variable domain (VL) by a linker which is too short to allow pairing between the two domains on the same chain. Accordingly, the VH and VL domains of one fragment are forced to pair with the complementary VL and VH domains of another fragment, thereby forming two antigen binding sites. Another strategy for making bispecific antibody fragments by the use of single-chain Fv (sFv) dimers is described in Gruber et al., J. Immunol., 152:5368 (1994).

Antibodies with more than two valencies are also contemplated. For example, trispecific antibodies can be prepared. *See, e.g.,* Tutt et al., J. Immunol., 147:60 (1991).

### G. Antibody Purification

When using recombinant techniques, antibodies can be produced inside an isolated host cell, in the periplasmic space of a host cell, or directly secreted from a host cell into the medium. If the antibody is produced intracellularly, the particulate debris is first removed, for example, by centrifugation or ultrafiltration. Carter et al., BioTech., 10:163-167 (1992) describes a procedure for isolating antibodies which are secreted into the periplasmic space of *E. coli*. Briefly, cell paste is thawed in the presence of sodium acetate (pH 3.5), EDTA, and phenylmethylsulfonylfluoride (PMSF) for about 30 min. Cell debris can be removed by centrifugation. Where the antibody is secreted into the medium, supernatants from such expression systems are generally concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from cells can be purified using, for example, hydroxylapatite chromatography, gel electrophoresis, dialysis, and affinity chromatography. The suitability of protein A as an affinity ligand depends on the species and isotype of any immunoglobulin Fc domain that is present in the antibody. Protein A can be used to purify antibodies that are based on human γ1, γ2, or γ4 heavy chains (*see, e.g.,* Lindmark et al., J. Immunol. Meth., 62:1-13 (1983)). Protein G is recommended for all mouse isotypes and for human γ3 (*see*, *e.g.,* Guss et al., EMBO J., 5:1567-1575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a CH3 domain, the Bakerbond ABX™ resin (J. T. Baker; Phillipsburg, N.J.) is useful for purification. Other techniques for protein purification such as fractionation on an ion-exchange column, ethanol precipitation, reverse phase HPLC, chromatography on silica, chromatography on heparin SEPHAROSE™, chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (*e.g*., from about 0-0.25 M salt).

One of skill in the art will appreciate that any binding molecule having a function similar to an antibody, *e.g*., a binding molecule or binding partner which is specific for one or more analytes of interest in a sample, can also be used in the methods and compositions of the present invention. Examples of suitable antibody-like molecules include, but are not limited to, domain antibodies, unibodies, nanobodies, shark antigen reactive proteins, avimers, adnectins, anticalms, affinity ligands, phylomers, aptamers, affibodies, trinectins, and the like.

### VII. Methods of Administration

According to the methods of the present invention, the anticancer drugs described herein are administered to a subject by any convenient means known in the art. The methods of the present invention can be used to select a suitable anticancer drug or combination of anticancer drugs for the treatment of a cancer (*e.g.,* a hematological malignancy) in a subject. The methods of the invention can also be used to identify the response of a cancer (*e.g.,* a hematological malignancy) in a subject to treatment with an anticancer drug or combination of anticancer drugs. In addition, the methods of the invention can be used to predict the response of a subject having cancer (*e.g.,* a hematological malignancy) to treatment with an anticancer drug or combination of anticancer drugs. Furthermore, the methods of the present invention can be used to identify a subject having cancer (*e.g.,* a hematological malignancy) who is resistant to treatment with an anticancer drug or combination of anticancer drugs. Those of skill in the art will appreciate that the anticancer drugs described herein can be administered alone or as part of a combined therapeutic approach with conventional chemotherapy, radiotherapy, hormonal therapy, immunotherapy, and/or surgery.

In certain embodiments, the anticancer drug comprises an anti-signaling agent (*i.e.,* a cytostatic drug) such as a monoclonal antibody or a tyrosine kinase inhibitor; an anti-proliferative agent; a chemotherapeutic agent (*i.e.,* a cytotoxic drug); a hormonal therapeutic agent; a radiotherapeutic agent; a vaccine; and/or any other compound with the ability to reduce or abrogate the uncontrolled growth of aberrant cells such as cancerous cells. In some embodiments, the subject is treated with one or more anti-signaling agents, anti-proliferative agents, and/or hormonal therapeutic agents in combination with at least one chemotherapeutic agent. Exemplary monoclonal antibodies, tyrosine kinase inhibitors, anti-proliferative agents, chemotherapeutic agents, hormonal therapeutic agents, radiotherapeutic agents, and vaccines are described above.

In some embodiments, the anticancer drugs described herein can be co-administered with conventional immunotherapeutic agents including, but not limited to, immunostimulants *(e.g.,* Bacillus Calmette-Guérin (BCG), levamisole, interleukin-2, alpha-interferon, *etc.),* immunotoxins (*e.g*., anti-CD33 monoclonal antibody-calicheamicin conjugate, anti-CD22 monoclonal antibody-pseudomonas exotoxin conjugate, *etc*.), and radioimmunotherapy (*e.g.,* anti-CD20 monoclonal antibody conjugated to ¹¹¹In, ⁹⁰Y, or ¹³¹I, *etc*.).

Anticancer drugs can be administered with a suitable pharmaceutical excipient as necessary and can be carried out via any of the accepted modes of administration. Thus, administration can be, for example, oral, buccal, sublingual, gingival, palatal, intravenous, topical, subcutaneous, transcutaneous, transdermal, intramuscular, intra-joint, parenteral, intra-arteriole, intradermal, intraventricular, intracranial, intraperitoneal, intravesical, intrathecal, intralesional, intranasal, rectal, vaginal, or by inhalation. By "co-administer" it is meant that an anticancer drug is administered at the same time, just prior to, or just after the administration of a second drug (*e.g*., another anticancer drug, a drug useful for reducing the side-effects associated with anticancer drug therapy, a radiotherapeutic agent, a hormonal therapeutic agent, an immunotherapeutic agent, *etc*.)*.*

A therapeutically effective amount of an anticancer drug may be administered repeatedly, *e.g*., at least 2, 3, 4, 5, 6, 7, 8, or more times, or the dose may be administered by continuous infusion. The dose may take the form of solid, semi-solid, lyophilized powder, or liquid dosage forms, such as, for example, tablets, pills, pellets, capsules, powders, solutions, suspensions, emulsions, suppositories, retention enemas, creams, ointments, lotions, gels, aerosols, foams, or the like, preferably in unit dosage forms suitable for simple administration of precise dosages.

As used herein, the term "unit dosage form" refers to physically discrete units suitable as unitary dosages for human subjects and other mammals, each unit containing a predetermined quantity of an anticancer drug calculated to produce the desired onset, tolerability, and/or therapeutic effects, in association with a suitable pharmaceutical excipient (*e.g.,* an ampoule). In addition, more concentrated dosage forms may be prepared, from which the more dilute unit dosage forms may then be produced. The more concentrated dosage forms thus will contain substantially more than, *e.g.,* at least 1, 2, 3, 4, 5, 6, 7, 8, 9, 10, or more times the amount of the anticancer drug.

Methods for preparing such dosage forms are known to those skilled in the art (*see, e.g.,* REMINGTON'S PHARMACEUTICAL SCIENCES, 18TH ED., Mack Publishing Co., Easton, PA (1990)). The dosage forms typically include a conventional pharmaceutical carrier or excipient and may additionally include other medicinal agents, carriers, adjuvants, diluents, tissue permeation enhancers, solubilizers, and the like. Appropriate excipients can be tailored to the particular dosage form and route of administration by methods well known in the art (*see, e.g., REMINGTON'S PHARMACEUTICAL SCIENCES, supra*).

Examples of suitable excipients include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline, syrup, methylcellulose, ethylcellulose, hydroxypropylmethylcellulose, and polyacrylic acids such as Carbopols, *e.g.,* Carbopol 941, Carbopol 980, Carbopol 981, *etc.* The dosage forms can additionally include lubricating agents such as talc, magnesium stearate, and mineral oil; wetting agents; emulsifying agents; suspending agents; preserving agents such as methyl-, ethyl-, and propyl-hydroxy-benzoates (*i.e.,* the parabens); pH adjusting agents such as inorganic and organic acids and bases; sweetening agents; and flavoring agents. The dosage forms may also comprise biodegradable polymer beads, dextran, and cyclodextrin inclusion complexes.

For oral administration, the therapeutically effective dose can be in the form of tablets, capsules, emulsions, suspensions, solutions, syrups, sprays, lozenges, powders, and sustained-release formulations. Suitable excipients for oral administration include pharmaceutical grades of mannitol, lactose, starch, magnesium stearate, sodium saccharine, talcum, cellulose, glucose, gelatin, sucrose, magnesium carbonate, and the like.

In some embodiments, the therapeutically effective dose takes the form of a pill, tablet, or capsule, and thus, the dosage form can contain, along with an anticancer drug, any of the following: a diluent such as lactose, sucrose, dicalcium phosphate, and the like; a disintegrant such as starch or derivatives thereof; a lubricant such as magnesium stearate and the like; and a binder such a starch, gum acacia, polyvinylpyrrolidone, gelatin, cellulose and derivatives thereof An anticancer drug can also be formulated into a suppository disposed, for example, in a polyethylene glycol (PEG) carrier.

Liquid dosage forms can be prepared by dissolving or dispersing an anticancer drug and optionally one or more pharmaceutically acceptable adjuvants in a carrier such as, for example, aqueous saline (*e.g.,* 0.9% w/v sodium chloride), aqueous dextrose, glycerol, ethanol, and the like, to form a solution or suspension, *e.g*., for oral, topical, or intravenous administration. An anticancer drug can also be formulated into a retention enema.

For topical administration, the therapeutically effective dose can be in the form of emulsions, lotions, gels, foams, creams, jellies, solutions, suspensions, ointments, and transdermal patches. For administration by inhalation, an anticancer drug can be delivered as a dry powder or in liquid form via a nebulizer. For parenteral administration, the therapeutically effective dose can be in the form of sterile injectable solutions and sterile packaged powders. Preferably, injectable solutions are formulated at a pH of from about 4.5 to about 7.5.

The therapeutically effective dose can also be provided in a lyophilized form. Such dosage forms may include a buffer, *e.g.*, bicarbonate, for reconstitution prior to administration, or the buffer may be included in the lyophilized dosage form for reconstitution with, *e.g*., water. The lyophilized dosage form may further comprise a suitable vasoconstrictor, *e.g*., epinephrine. The lyophilized dosage form can be provided in a syringe, optionally packaged in combination with the buffer for reconstitution, such that the reconstituted dosage form can be immediately administered to a subject.

A subject can also be monitored at periodic time intervals to assess the efficacy of a certain therapeutic regimen. For example, the activation states of certain oncogenic fusion proteins and/or signal transduction molecules may change based on the therapeutic effect of treatment with one or more of the anticancer drugs described herein. The subject can be monitored to assess response and understand the effects of certain drugs or treatments in an individualized approach. Additionally, subjects who initially respond to a specific anticancer drug or combination of anticancer drugs may become refractory to the drug or drug combination, indicating that these subjects have developed acquired drug resistance. These subjects can be discontinued on their current therapy and an alternative treatment prescribed in accordance with the methods of the present invention.

In certain aspects, the methods described herein can be used in conjunction with panels of gene expression markers that predict the likelihood of cancer prognosis and/or recurrence in various populations. These gene panels can be useful for identifying subjects who are unlikely to experience recurrence and, thus, unlikely to benefit from adjuvant chemotherapy. The expression panels can be used to identify subjects who can safely avoid adjuvant chemotherapy, without negatively affecting disease-free and overall survival outcomes.

In addition, in certain other aspects, the methods described herein can be used in conjunction with panels of gene expression markers that identify the original tumors for cancers of unknown primary (CUP). These gene panels can be useful in identifying subjects with metastatic cancer who would benefit from therapy consistent with that given to subjects diagnosed initially with cancer. Suitable systems include, but are not limited to, the Aviara CancerTYPE ID assay, an RT-PCR-based expression assay that measures 92 genes to identify the primary site of origin for 39 tumor types; and the Pathwork^{®} Tissue of Origin Test, which measures the expression of more than 1600 genes on a microarray and compares a tumor's gene expression "signature" against those of 15 known tissue types."

### VIII. Examples

The following examples are offered to illustrate, but not to limit, the claimed invention.

### Example 1. Single Cell Detection Using a Proximity Dual Detector Microarray ELISA with Tyramide Signal Amplification.

This example illustrates a multiplex, high-throughput, proximity dual detector microarray sandwich ELISA having superior dynamic range that is suitable for analyzing the activation states of fusion proteins and signal transduction molecules in cellular extracts prepared from isolated cells. In particular embodiments, the proximity assay is in the format of an addressable microarray.
1) Capture antibody was printed on a 16-pad FAST slide (Whatman Inc.) with a serial dilution of from 1 mg/ml to 0.004 mg/ml. Alternatively, a 2-fold dilution series of each capture antibody (0.25 mg/ml, 0.125 mg/ml, and 0.0625 mg/ml) can be used, and double and quadruple spots can be made for each antibody dilution. For detecting the BCR-ABL fusion protein, an exemplary capture antibody comprises an anti-BCR monoclonal or polyclonal antibody.
2) After drying overnight, the slide was blocked with Whatman blocking buffer.
3) 80 µl of cell lysate was added onto each pad with a 10-fold serial dilution. The slide was incubated for two hours at room temperature.
4) After six washes with TBS-Tween, 80 µl of detection antibodies for the proximity assay diluted in TBS-Tween/2% BSA/1% FBS was added to the slides. For detecting the BCR-ABL fusion protein, exemplary detection antibodies include: (1) an anti-ABL monoclonal or polyclonal antibody directly conjugated to glucose oxidase (GO); and (2) a monoclonal or polyclonal antibody recognizing phosphorylated ABL directly conjugated to horseradish peroxidase (HRP). The incubation was for 2 hours at room temperature.
5) Alternatively, the detection step can utilize a biotin-conjugate of the antibody recognizing phosphorylated ABL. In these instances, after six washes an additional sequential step of incubation with streptavidin-HRP for 1 hour was included.
6) Alternatively, the detection step can utilize an oligonucleotide-mediated GO conjugate of the anti-ABL antibody. Either the directly conjugated or biotin-steptavidin (SA) linked conjugate of HRP to the phosphorylated ABL antibody can be used.
6) For signal amplification, 80 µl of biotin-tyramide at 5 µg/ml was added and reacted for 15 min. The slide was washed six times with TBS-Tween, twice with 20% DMSO/TBS-Tween, and once with TBS.
7) 80 µl of SA-Alexa 555 was added and incubated for 30 min. The slide was then washed twice, dried for 5 minutes, and scanned on a microarray scanner (Perkin-Elmer, Inc.).

The proximity assay microarray format described herein advantageously exhibits very low background (*e.g*., compared to two-antibody assays) due to the increased specificity obtained by detecting the proximity between two detection antibodies.

### Example 2. Generation of Activation Profiles for Drug Selection.

The compositions and methods of the present invention can be applied for drug selection for cancer treatment. As a non-limiting example, the present invention finds utility in identifying the presence and/or activity of one or a plurality of oncogenic fusion proteins associated with hematological malignancies in order to provide the appropriate treatment for patients with these types of cancer. A typical protocol entails the generation of two profiles, a reference activation profile and a test activation profile, which are then compared to determine the efficacy of a particular drug treatment regimen (*see,* Figure 2).

### Reference Activation Profile

To derive a reference activation profile, a blood sample is obtained from a patient having a specific type of cancer (*e.g*., leukemia such as CML) prior to anticancer drug treatment. Tumor cells are isolated from the blood sample using, *e.g*., any of the techniques as described in greater detail herein. The isolated cells can be stimulated *in vitro* with one or more growth factors. The stimulated cells are then lysed to produce a cellular extract. The cellular extract is applied to an addressable array containing a dilution series of a panel of capture antibodies specific for one or a plurality of oncogenic fusion proteins (alone or in combination with one or a plurality of signal transduction molecules) whose activation states may be altered in the patient's type of cancer. Single detection or proximity assays are performed using the appropriate detection antibodies (*e.g*., activation state-independent antibodies and/or activation state-dependent antibodies) to determine the activation state of each analyte of interest. A reference activation profile is thus generated providing the activation states of specific analytes such as oncogenic fusion proteins in the patient's cancer in the absence of any anticancer drugs.

### Test Activation Profile

To obtain a test activation profile, a second blood sample is obtained from the patient having the specific type of cancer (*e.g*., leukemia such as CML) either prior to anticancer drug treatment or after administration of an anticancer drug (*e.g.,* at any time throughout the course of cancer treatment). Tumor cells are isolated from the blood sample. If isolated cells are obtained from a patient who has not received treatment with an anticancer drug, the isolated cells are incubated with anticancer drugs which target one or more of the activated oncogenic fusion proteins and/or signal transduction molecules determined from the reference activation profile described above. For example, if it is determined from the reference activation profile that the BCR-ABL fusion protein is activated, then the cells can be incubated with imatinib (Gleevec^{®}). The isolated cells can then be stimulated *in vitro* with one or more growth factors. The isolated cells are then lysed to produce a cellular extract. The cellular extract is applied to the addressable array and single detection or proximity assays are performed to determine the activation state of each analyte of interest. A test activation profile for the patient is thus generated providing the activation states of specific analytes such as oncogenic fusion proteins in the patient's cancer in the presence of specific anticancer drugs.

### Drug selection

The anticancer drugs are determined to be suitable or unsuitable for treatment of the patient's cancer by comparing the test activation profile to the reference activation profile. For example, if drug treatment causes most or all of the oncogenic fusion proteins and/or signal transduction molecules to be substantially less activated than in the absence of the drugs, *e.g.,* a change from strong activation without the drugs to weak or very weak activation with the drugs, then the treatment is determined to be suitable for the patient's cancer. In such instances, treatment is either initiated with the suitable anticancer drug in a patient who has not received drug therapy or subsequent treatment is continued with the suitable anticancer drug in a patient already receiving the drug. However, if the drug treatment is deemed unsuitable for treatment of the patient's cancer, different drugs are selected and used to generate a new test activation profile, which is then compared to the reference activation profile. In such instances, treatment is either initiated with a suitable anticancer drug in a patient who has not received drug therapy or subsequent treatment is changed to a suitable anticancer drug in a patient currently receiving the unsuitable drug.

The protocol described in this example is also useful for identifying patients who are resistant to therapy with a tyrosine kinase inhibitor such as imatinib due to mutations in the target protein kinase (*e.g*., BCR-ABL), non-compliance with the therapeutic regimen, and/or administration of a suboptimal drug dose.

### Example 3. Exemplary Oncogenic Fusion Proteins Associated With Cancer.

This example provides a non-exhaustive list of translocations in human tumors that cause the formation of oncogenic fusion proteins and their associated neoplasms.

Burkitt's lymphoma: c-myc gene translocation t(8;14)(q24;q32). The most common chimeric oncoprotein is c-myc/IGH.

AML: translocation of a part of chromosome 8 to chromosome 21 The resulting chimeric oncoprotein is RUNX1/ETO. Another translocation t(12;15)(p13;q25) results in the TEL/TrkC (kinase) chimeric oncoprotein.

CML: Philadelphia chromosome is a translocation which results in BCR/ABL (kinase).

Ewing sarcoma: translocation between chromosomes 11 and 22. The resulting chimeric oncoprotein is EWS/FLI (transcription factor).

ALL: Chimeric oncogenic proteins:

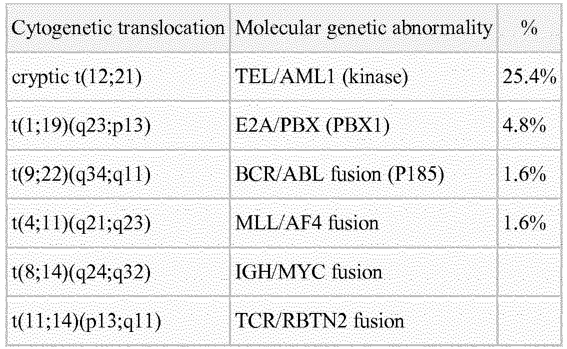

DFSP: Over 95% of DFSP tumors have the chromosomal translocation t(17;22), which results in the chimeric oncoprotein COL1A1/PDGF (binds and activates PDGFR).

Acute promyelocytic leukemia: a translocation denoted as t(15;17)(q22;q12). The resulting chimeric oncoprotein is RARα/PML (transcription complex protein).

Pro-B-cell acute lymphoblastic leukemia: translocation t(17;19), which results in the chimeric oncoprotein E2A/HLF (apoptosis inhibitor).

Acute pre-B-cell leukemia: translocation t(1;19). The chimeric oncoprotein is E2A/Pbx1 (kinase substrate).

Rhabdomyosarcoma : translocation of t(2:13)(q35;q14), which results in the chimeric oncoprotein PAX3/FKHR (transcription factor).

A soft tissue malignancy of very young children: t(12;15)(p13;q25) rearrangement which results in the following chimeric oncoprotein: protein tyrosine kinase ETV6/NTRK3 (kinase).

Papillary thyroid carcinoma: the chimeric oncoprotein is RET/PTC (kinase).

Prostate cancer: the chimeric oncoprotein is TMRSS/ERG (kinase).

Additional examples of translocations in human tumors that cause the formation of oncogenic fusion proteins and their associated neoplasms:

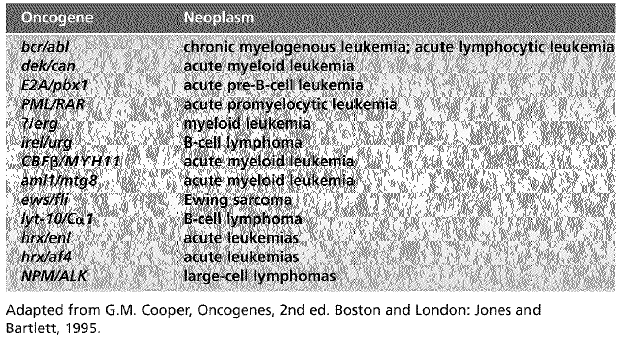

### Example 4. Proximity-Mediated Immunoassay for the Detection of Total and Activated Levels of Oncogenic Fusion Proteins.

### Introduction

The oncogene BCR-ABL is formed by a translocation of the normal ABL gene located at the long arm of chromosome 9 to the N-terminal part of the BCR gene located at the long arm of chromosome 22 to form the Philadelphia chromosome. Depending on where the translocated ABL gene is spliced onto the N-terminal of the BCR gene, different lengths of the BCR-ABL gene products are produced, with the most prevalent being the p210 BCR-ABL gene product as the oncogene that causes CML and a subset of ALL. Other additional gene products, such as p185 and p230 BCR-ABL, are also produced. In particular, the p210 BCR-ABL gene product is responsible for causing AML. This protein contains 1790 amino acids and is composed of an oligomerization domain (OLI) from BCR at the N-terminus, followed by the S/T kinase domain from BCR, an insert of amino acid sequence from BCR which is not present in normal BCR, followed by the SH3, SH2 and Y kinase domains from ABL as well as the C-terminal proline-rich domain of ABL.

### Clinical Background

While most patients with chronic myelogenous leukemia (CML) in the chronic phase respond well to imatinib, some patients do not achieve the desirable end point, and others may eventually lose response or are intolerant. With regard to 400 mg/day imatinib (Gleevec) treatment: there is a complete cytogenetic response (CCyR) in 70-80% of patients; there is a loss of CCyR at a rate of 4 to 7%/yr in the first 3 years and then 1 to 2%/yr thereafter; the overall survival rate after 7 years is 90%, the even free survival rate after 7 years is 81%; and the relapse rate is about 30% in 5 years. There is a better response to 800 mg/day imatinib (Gleevec) treatment (CCyR = 95%; Relapse Rate = 5%), but such a dose of imatinib is also more toxic. Nilotinib has an efficacy similar to 800 mg/day imatinib, but it is more toxic than imatinib.

Complete BCR-ABL inhibition yields better response to Gleevec, but incomplete BCR-ABL inhibition results in relapse to Gleevec. Therefore, real-time detection of the level of expression and the degree of activation of BCR-ABL would benefit CML patients under targeted therapy by adjusting drug dose to effectively inhibit the target while minimizing toxicity. For example, those patients with incomplete inactivation with 400 mg/day of Gleevec could be placed on a higher dose (*e.g.*, 800 mg/day) sooner to ensure response.

### Diagnostic Challenge

High baseline levels of full-length BCR and ABL make it very difficult to detect the relatively low in abundance BCR-ABL fusion protein. In addition, current assays that detect BCR-ABL phosphorylation levels do not have the sensitivity to detect phosphorylation in clinical blood samples. Furthermore, antibodies against fusion proteins such as BCR-ABL are not very specific for multiple variants of the fusion protein.

### Novel BCR-ABL Detection Assays

Figure 3A illustrates an exemplary proximity assay (300) for detecting the presence (total level) and/or activation state (phosphorylation level) of an oncogenic fusion protein such as BCR-ABL (310). Figure 3B illustrates an alternative embodiment (400) of the proximity assays of the present invention for detecting the presence (total level) and/or activation state (phosphorylation level) of BCR-ABL (410) using junction antibodies.

Non-limiting examples of antibodies suitable for use in the methods for measuring BCR-ABL total and/or activated protein levels as illustrated in Figures 3A-3B include those set forth in Table 1 above.

### Results

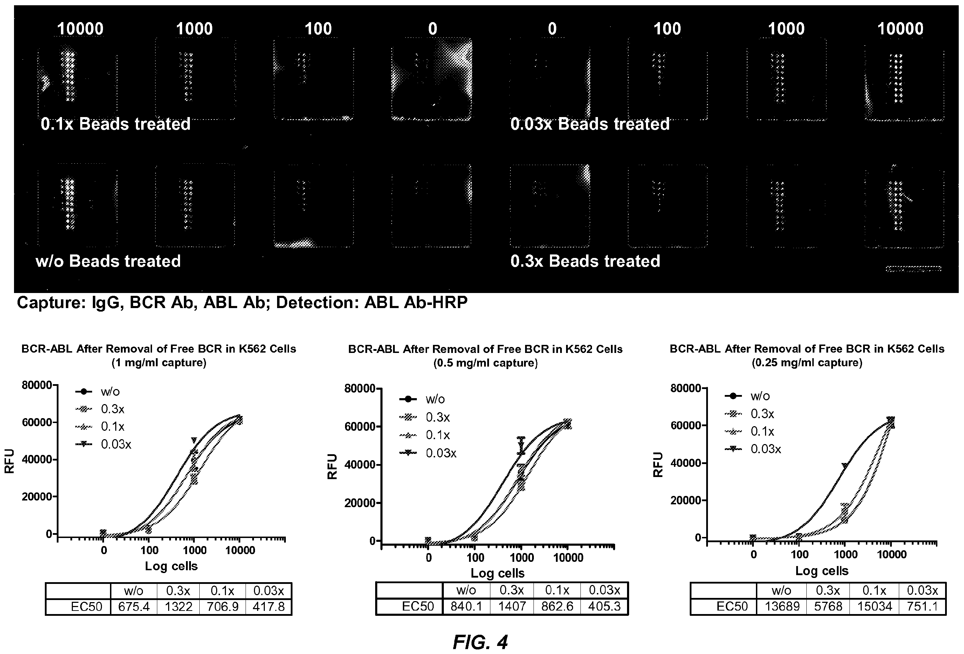

Figure 4 illustrates the BCR-ABL signal in K562 cells (*i.e.*, cells from a human chronic myelogenous leukemia cell line) after removal of free full-length BCR using an antibody specific for the carboxyl-terminal region of full-length BCR conjugated to beads in accordance with the exemplary proximity assay depicted in Figure 3A. In particular, Figure 4 demonstrates that the BCR-ABL signal is not affected by removal of free BCR from a patient sample using the novel proximity assay of the present invention.

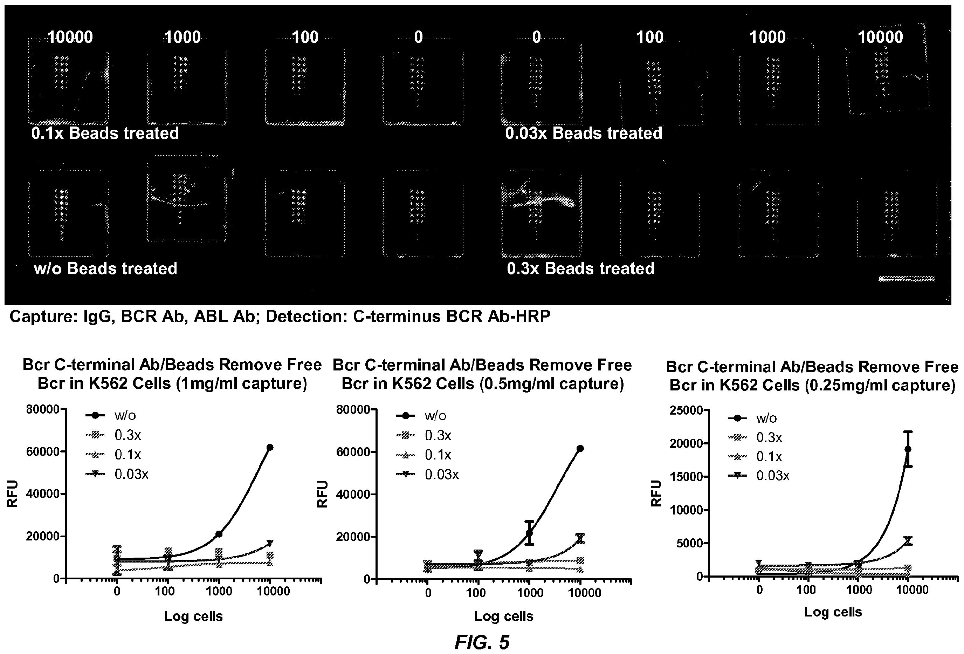

Figure 5 illustrates the BCR signal in K562 cells after removal of free BCR using an antibody specific for the carboxyl-terminal region of full-length BCR conjugated to beads in accordance with the exemplary proximity assay depicted in Figure 3A. As shown in Figure 5, a decrease in BCR signal at 10,000 cells demonstrates that only BCR is removed using the novel proximity assay of the present invention.

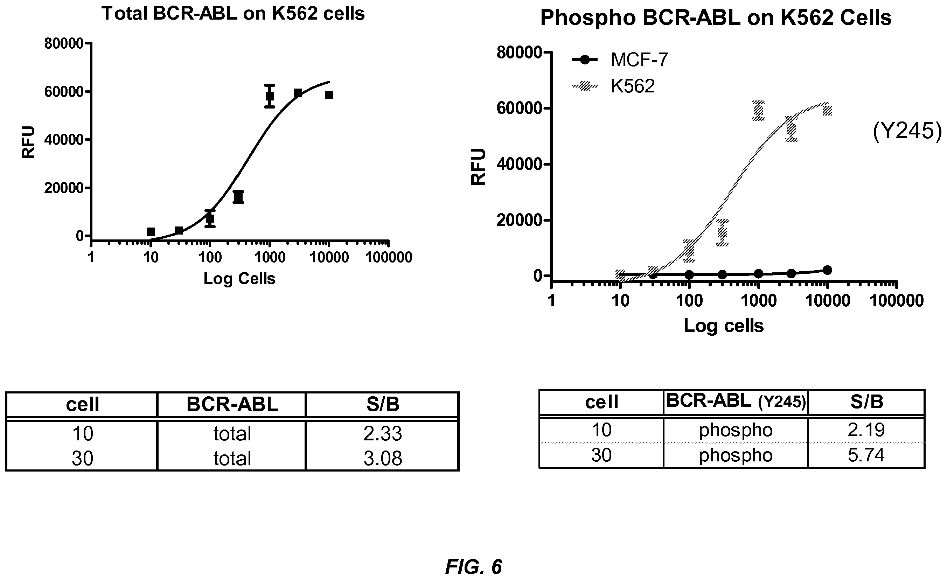

Figure 6 illustrates the detection of total and phosphorylated levels of BCR-ABL in K562 cells using the exemplary proximity assay depicted in Figure 3A.

### Conclusion

This example demonstrates that the exemplary proximity assays depicted in Figures 3A and 3B for detecting the presence and/or activation state of BCR-ABL is advantageous for at least the following reasons: (1) full-length BCR proteins can be removed from a patient sample such as blood or bone marrow aspirate using a depletion-tag specific for the C-terminal region of BCR; (2) once full-length BCR is removed from the patient sample, capture antibodies specific for the N-terminal region of BCR can capture BCR-ABL fusion proteins; and (3) once BCR-ABL fusion proteins are captured, their levels of expression and activation can be detected with high sensitivity via proximity channeling, wherein a single detectable signal which correlates to total or activated BCR-ABL protein levels is generated only upon the binding of all three antibodies, resulting in increased assay specificity, lower background, and simplified detection.

### Example 5. Detection of Total and Activated Levels of BCR-ABL Without Interference from Full-Length BCR and/or ABL.

This example provides additional experimental data demonstrating the advantages of the exemplary proximity assays depicted in Figures 3A and 3B for detecting the presence (total level) and/or activation state (phosphorylation level) of BCR-ABL. In one particular embodiment, full-length BCR proteins are removed from an extract of cells (*e.g.*, malignant white blood cells such as leukemia cells) obtained from a patient sample or from an extract of cells obtained from a cell line (*e.g.*, a leukemia cell line) using a depletion-tag specific for the C-terminal region of BCR. Once full-length BCR is removed from the cellular extract, capture antibodies specific for the N-terminal region of BCR can capture BCR-ABL fusion proteins. Once BCR-ABL fusion proteins are captured, their levels of expression and activation can be detected with high sensitivity via proximity channeling, wherein a single detectable signal which correlates to total or activated BCR-ABL protein levels is generated only upon the binding of all three antibodies, advantageously resulting in increased assay specificity, lower background, and simplified detection.

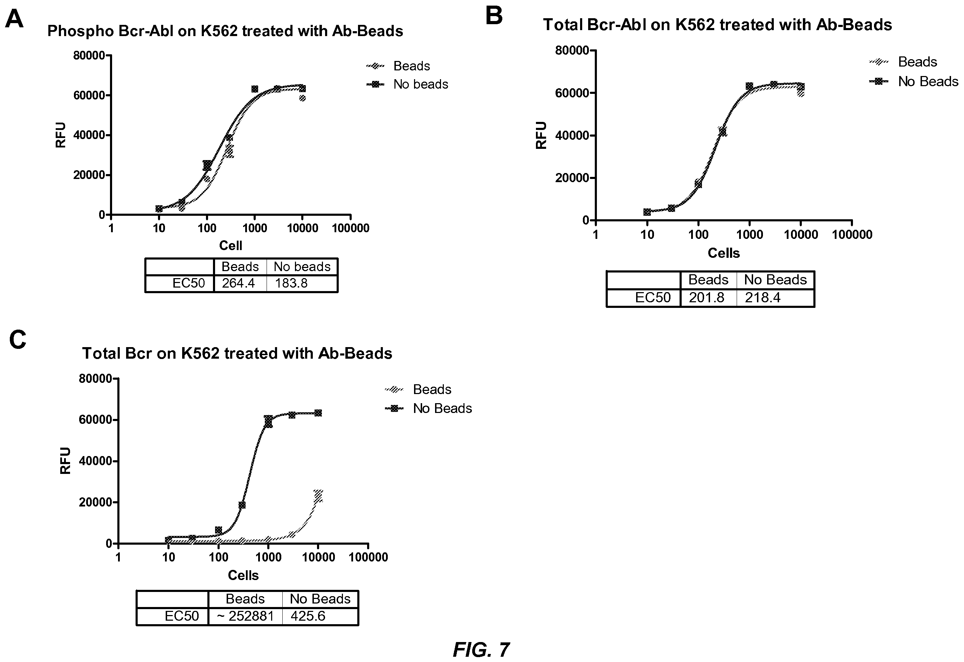

Figure 7 illustrates the phosphorylation level ("Phospho BCR-ABL") and total amount ("Total BCR-ABL") of BCR-ABL detected in K562 cells (*i.e.*, cells from a human chronic myelogenous leukemia cell line) after removal of free full-length BCR using an antibody specific for the carboxyl-terminal region of full-length BCR conjugated to beads in accordance with the exemplary proximity assay depicted in Figure 3A. In particular, Figures 7A and 7B show that the phosphorylated and total BCR-ABL signals were not changed after contacting an extract of K562 cells with antibodies specific for the C-terminus of full-length BCR coupled to beads to remove free BCR protein using the novel proximity assay of the present invention. Figure 7C illustrates that free full-length BCR was indeed removed from the cellular extract after treatment with BCR C-terminal antibody-coupled beads.

Figure 8 provides another illustration of the phosphorylated BCR-ABL signal in K562 cells after removal of free BCR using an antibody specific for the carboxyl-terminal region of full-length BCR conjugated to beads in accordance with the exemplary proximity assay depicted in Figure 3A. In particular, Figure 8A provides a microarray comparison of the phosphorylated BCR-ABL signal detected in K562 cell lysates with or without removal of full-length BCR ("Non-beads treated" = BCR not removed versus "Beads treated" = BCR removed with beads containing an antibody specific for the C-terminus of full-length BCR conjugated thereto). Figure 8B provides a graphical depiction of the microarray data with Relative Fluorescence Units (RFU) as a function of cell number. These figures demonstrate that the phospho BCR-ABL signal was not changed upon removal of full-length BCR from an extract of K562 cells using the novel proximity assay of the present invention.

Figure 9 provides another illustration of the total BCR-ABL signal in K562 cells after removal of free BCR using an antibody specific for the carboxyl-terminal region of full-length BCR conjugated to beads in accordance with the exemplary proximity assay depicted in Figure 3A. In particular, Figure 9A provides a microarray comparison of the total BCR-ABL signal detected in K562 cell lysates with or without removal of full-length BCR ("Non-beads treated" = BCR not removed versus "Beads treated" = BCR removed with beads containing an antibody specific for the C-terminus of full-length BCR conjugated thereto). Figure 9B provides a graphical depiction of the microarray data with Relative Fluorescence Units (RFU) as a function of cell number. These figures demonstrate that the total BCR-ABL signal was not changed upon removal of full-length BCR from an extract of K562 cells using the novel proximity assay of the present invention.

Figure 10 provides another illustration of the removal of free full-length BCR from an extract of K562 cells after contacting the cellular extract with BCR C-terminal antibody-coupled beads. In particular, Figure 10A provides a microarray comparison of the total BCR signal detected in K562 cell lysates with or without removal of full-length BCR ("Non-beads treated" = BCR not removed versus "Beads treated" = BCR removed with beads containing an antibody specific for the C-terminus of full-length BCR conjugated thereto). Figure 10B provides a graphical depiction of the microarray data with Relative Fluorescence Units (RFU) as a function of cell number. These figures demonstrate that full-length BCR was virtually depleted from the cellular extract when treated with beads containing an antibody specific for the C-terminus of full-length BCR. In fact, cellular extracts containing, *e.g.*, less than 1000 cells, did not produce a BCR signal that was significantly above background (0 cells) when incubated with BCR C-terminal antibody-coupled beads.

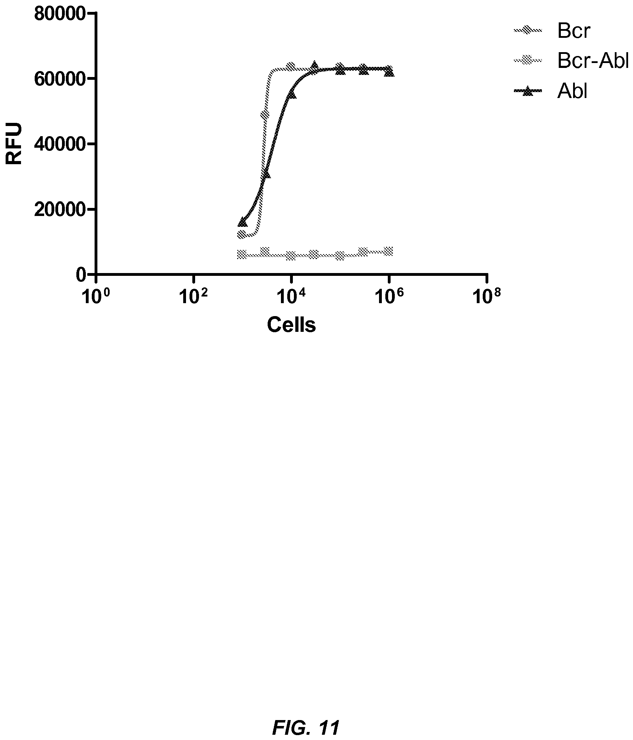

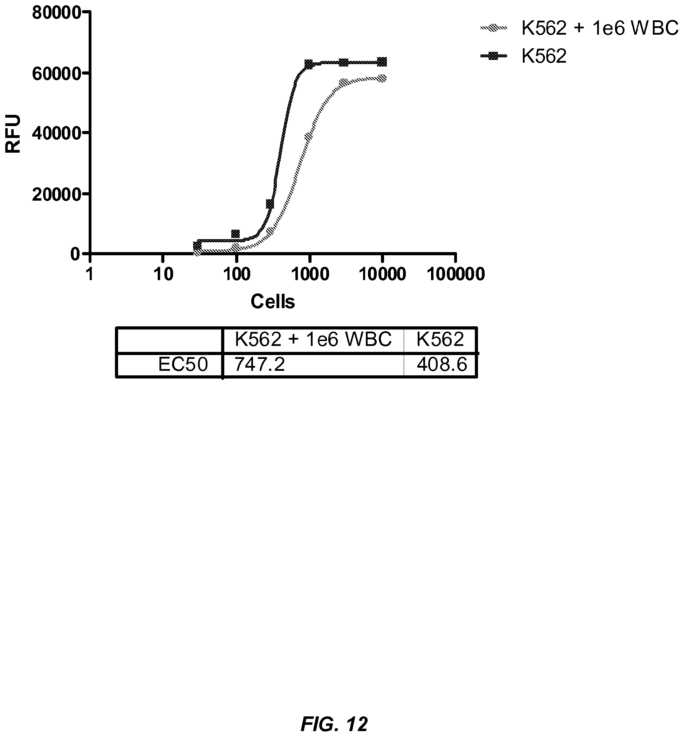

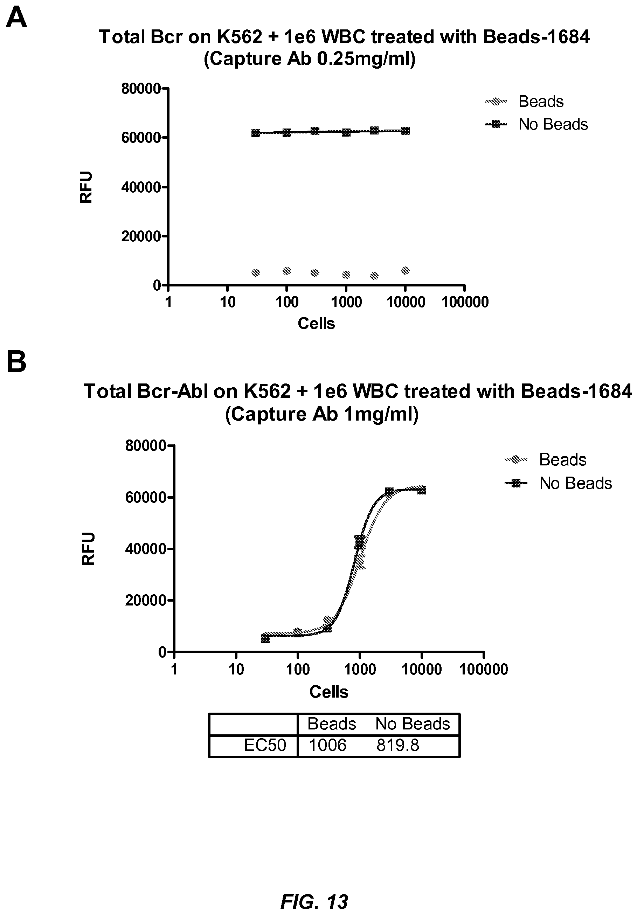

Figure 11 illustrates that free full-length BCR and ABL proteins, but not BCR-ABL fusion protein, are present in white blood cells (WBCs). Figure 12 shows that the free full-length BCR present in WBCs inhibited the phospho BCR-ABL signal in K562 cell extracts when such K562 cell extracts were spiked with WBC extracts. Figure 13 illustrates the total BCR-ABL signal in K562 cells spiked with WBC extracts after removal of free BCR using an antibody specific for the carboxyl-terminal region of full-length BCR conjugated to beads. In particular, Figure 13A shows that the free BCR signal was saturated when the K562 cell extracts were spiked with WBC extracts. After treatment with BCR C-terminal antibody-coupled beads, the free BCR was removed. Figure 13B shows that the BCR-ABL signal was not changed with or without beads treatment in the same experiment.

In some embodiments, full-length ABL protein can additionally or alternatively be removed from the extract of cells (*e.g.*, malignant white blood cells such as leukemia cells) obtained from a patient sample or from the extract of cells obtained from a cell line (*e.g.*, a leukemia cell line) prior to capture and detection of BCR-ABL expression and/or activation by using a depletion tag specific for the amino-terminal region of full-length ABL. A non-limiting example of such a depletion tag is a bead to which an antibody specific for the N-terminal region of full-length ABL is attached.

### Example 6. Detection of Total and Activated Levels of BCR-ABL in Cells Treated With Inhibitors of BCR-ABL Kinase Activity.

This example demonstrates that BCR-ABL inhibitors such as imatinib (Gleevec^{®}), nilotinib (Tasigna^{®}), and dasatinib (Sprycel^{®}) dose-dependently inhibited activation (*i.e.*, phosphorylation), but not expression (*i.e.*, total levels), of BCR-ABL protein in K562 cells (*i.e.*, cells from a human chronic myelogenous leukemia cell line).

In particular, Figures 14A and 14B illustrate that the BCR-ABL inhibitor imatinib (Gleevec^{®}) dose-dependently inhibited activation (*i.e.*, phosphorylation), but not expression (*i.e.*, total levels), of BCR-ABL protein in K562 cells. Figure 14C shows the phospho/total ratio of BCR-ABL upon imatinib inhibition, which correlates with the percent inhibition of the phospho BCR-ABL signal with imatinib treatment.

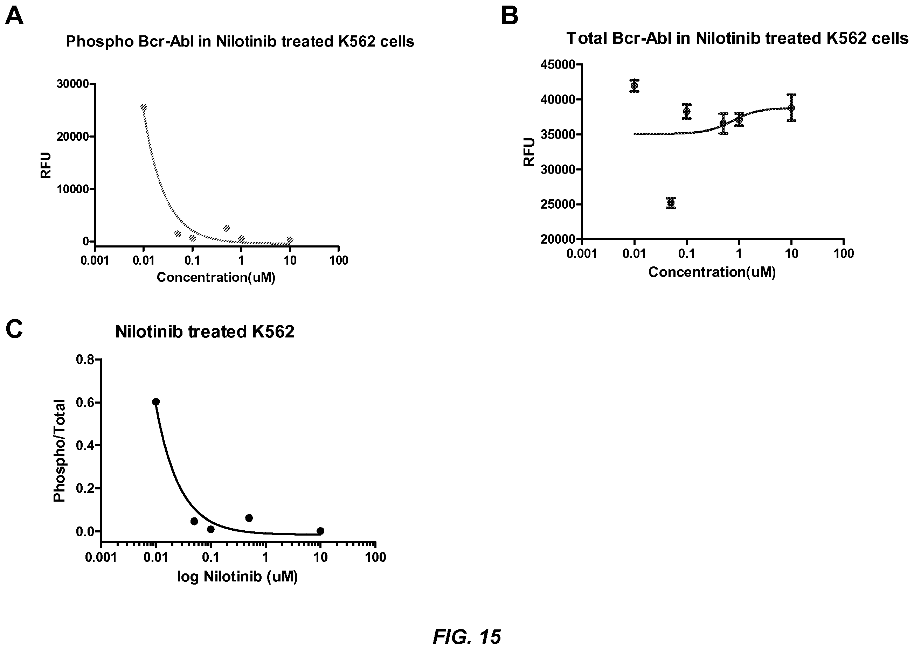

Figures 15A and 15B illustrate that the BCR-ABL inhibitor nilotinib (Tasigna^{®}) dose-dependently inhibited activation (*i.e*., phosphorylation), but not expression (*i.e*., total levels), of BCR-ABL protein in K562 cells. Figure 15C shows the phospho/total ratio of BCR-ABL upon nilotinib inhibition, which correlates with the percent inhibition of the phospho BCR-ABL signal with nilotinib treatment.

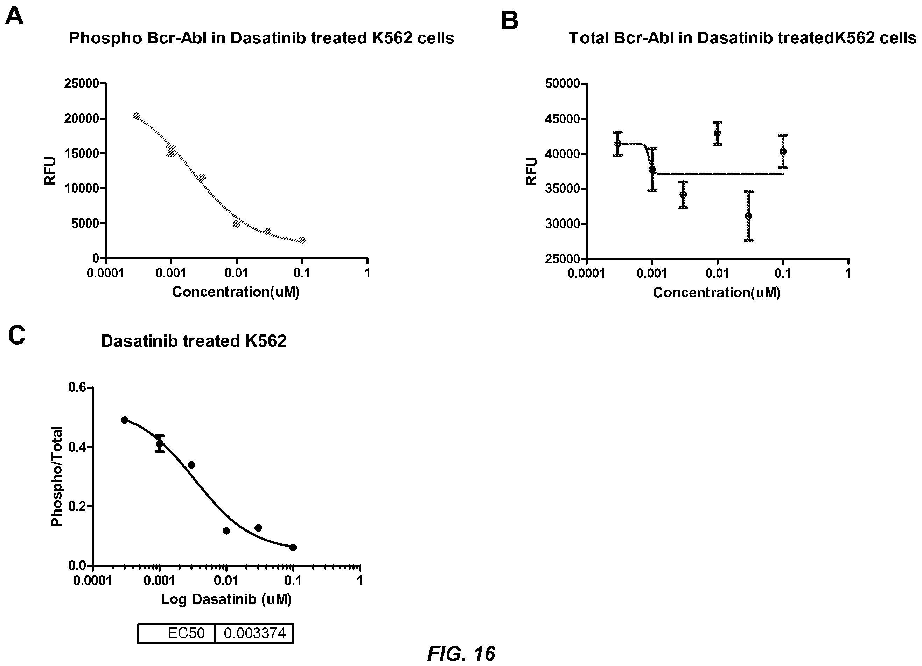

Figures 16A and 16B illustrate that the BCR-ABL inhibitor dasatinib (Sprycel^{®}) dose-dependently inhibited activation (*i.e*., phosphorylation), but not expression (*i.e*., total levels), of BCR-ABL protein in K562 cells. Figure 16C shows the phospho/total ratio of BCR-ABL upon dasatinib inhibition, which correlates with the percent inhibition of the phospho BCR-ABL signal with dasatinib treatment.

### Example 7. Detection of Total and Activated Levels of the BCR-ABL Substrate CRKL in Various Cancer Cell Lines.

This example demonstrates the detection of total and activated (phosphorylated) levels of the BCR-ABL substrate CRKL as determined by a sandwich ELISA. In other embodiments, the presence and/or activation state of a BCR-ABL substrate such as CRKL can be measured using a proximity assay such as a Collaborative Proximity Immunoassay (COPIA) described in PCT Application No. PCT/US2010/042182, filed July 15, 2010, and US Patent Publication Nos. 20080261829, 20090035792, and 20100167945.

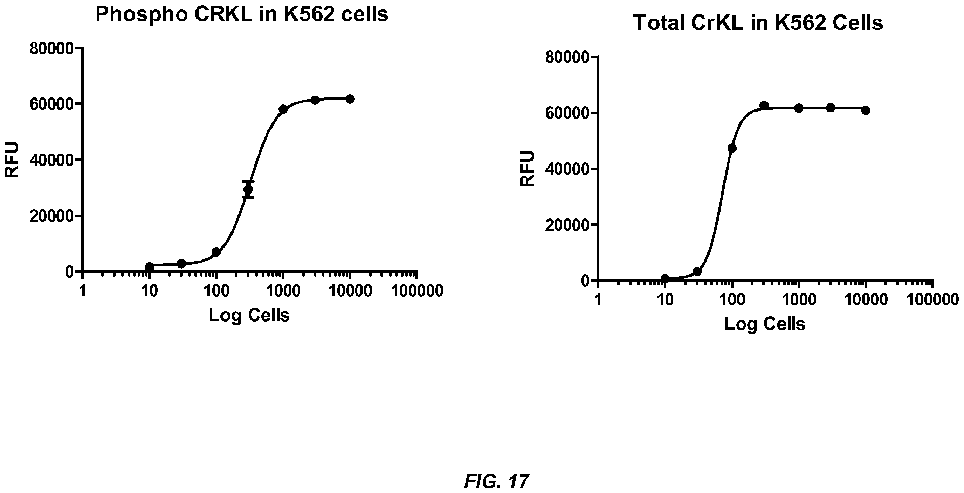

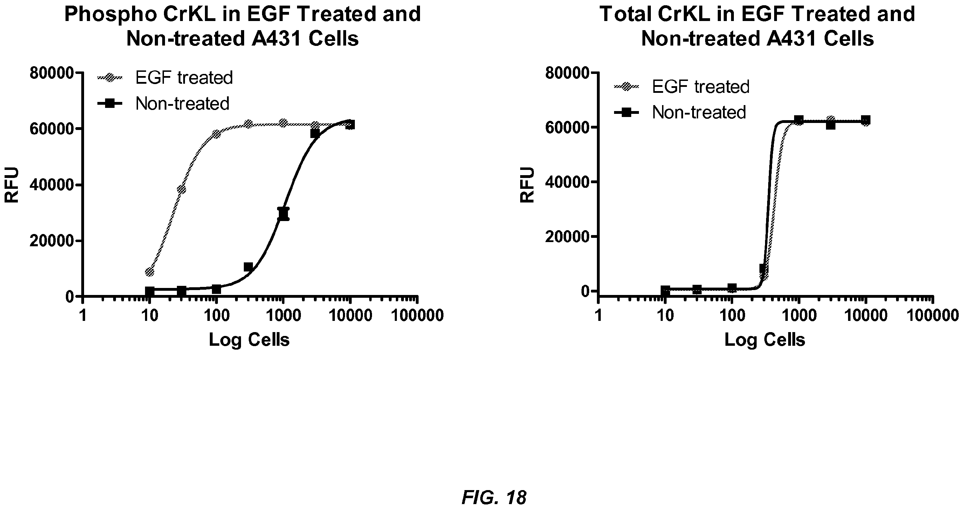

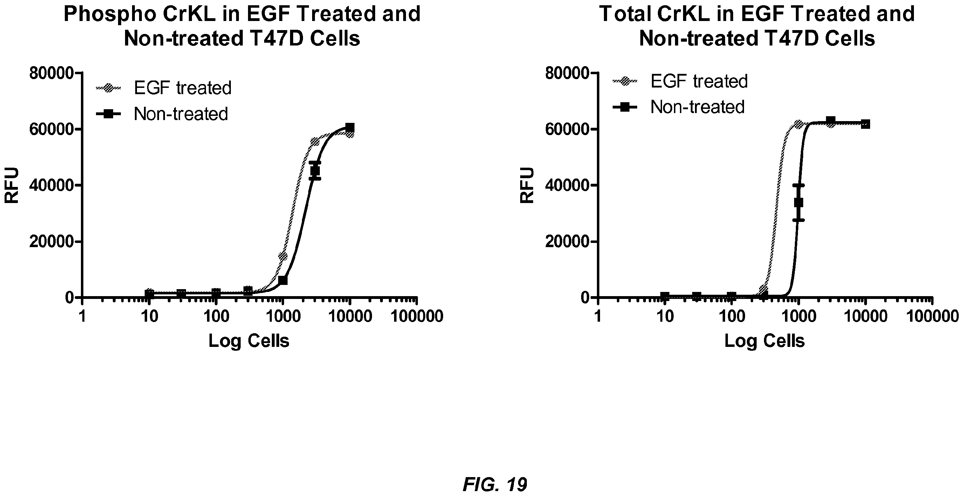

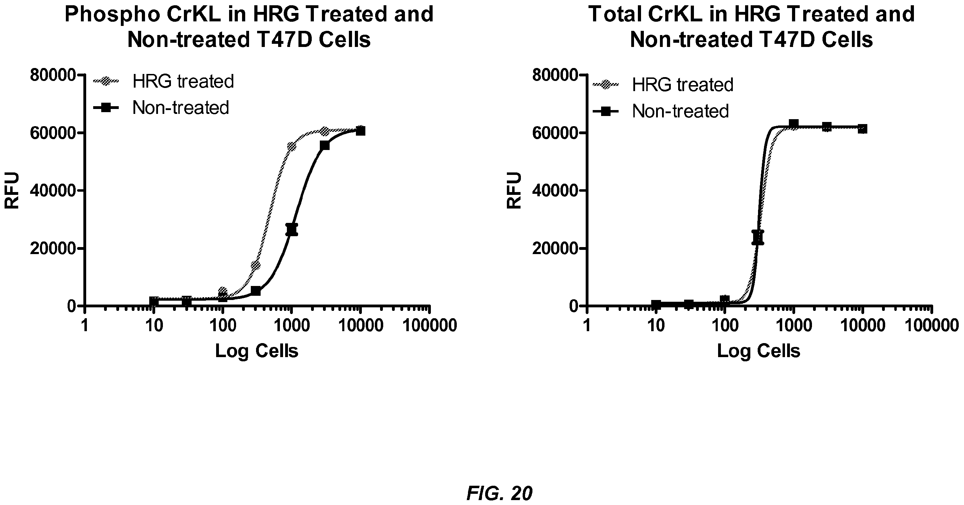

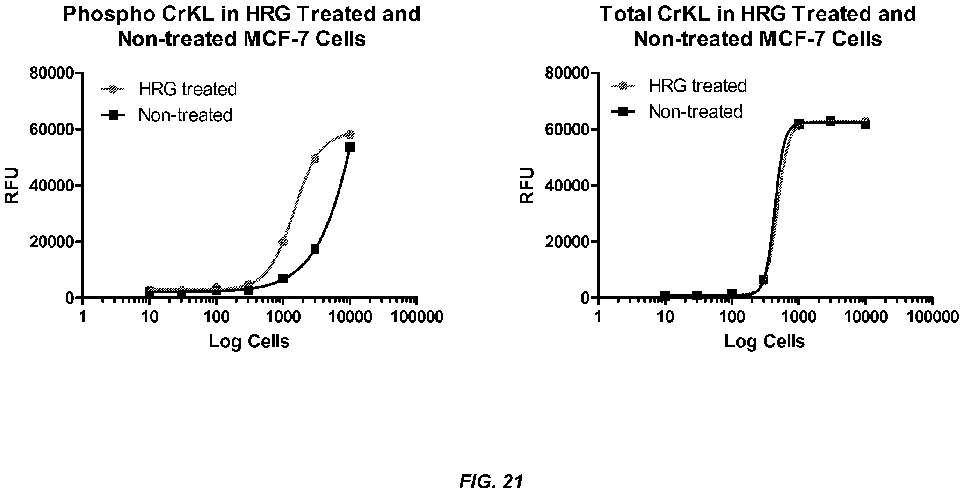

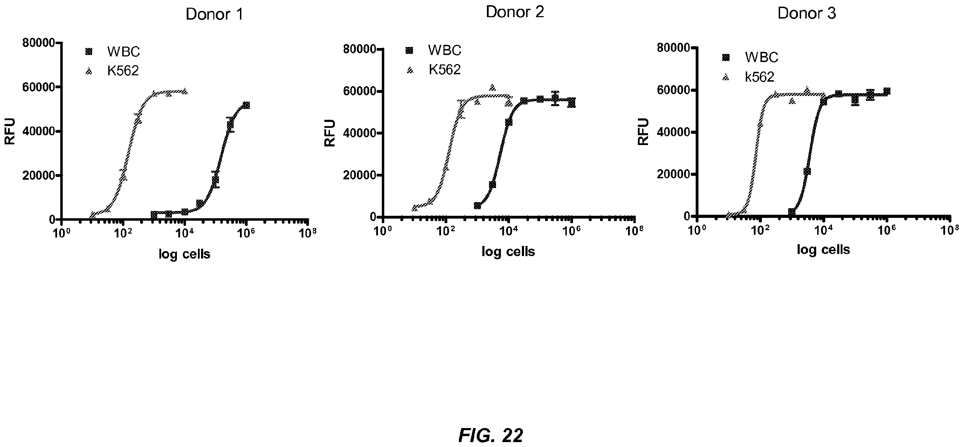

Figure 17 illustrates that CRKL is both present and activated (*i.e.*, phosphorylated) in K562 cells (*i.e.*, cells from a human chronic myelogenous leukemia cell line). Figure 18 shows that CRKL is present in A431 cells (*i.e.*, cells from a human epidermoid carcinoma cell line) and is activated (*i.e.*, phosphorylated) upon EGF treatment. Figure 19 shows that CRKL is present in T47D cells (*i.e.*, cells from a human ductal breast epithelial tumor cell line) but is not activated (*i.e.*, phosphorylated) upon EGF treatment. Figure 20 shows that CRKL is present in T47D cells and is activated (*i.e.*, phosphorylated) at low levels upon heregulin (HRG) treatment. Figure 21 shows that CRKL is present in MCF-7 cells (*i.e.*, cells from a human breast adenocarcinoma cell line) and is activated (*i.e.*, phosphorylated) at low levels upon heregulin (HRG) treatment. Figure 22 illustrates the presence of activated (*i.e.*, phosphorylated) CRKL in the white blood cells (WBCs) of patient samples, with the level of activation being different between donors.

### Example 8. Detection of Total and Activated Levels of the BCR-ABL Substrate JAK2 in Various Cancer Cell Lines.

This example demonstrates the detection of activated (phosphorylated) levels of the BCR-ABL substrate JAK2 as determined by a sandwich ELISA. In other embodiments, the presence and/or activation state of a BCR-ABL substrate such as JAK2 can be measured using a proximity assay such as a Collaborative Proximity Immunoassay (COPIA) described in PCT Application No. PCT/US2010/042182, filed July 15, 2010, and US Patent Publication Nos. 20080261829, 20090035792, and 20100167945.

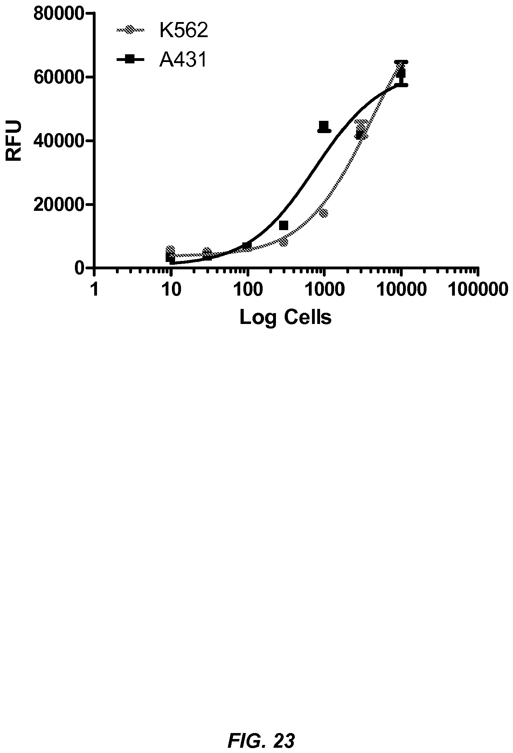

Figure 23 illustrates that JAK2 is activated (*i.e.*, phosphorylated) in K562 cells (*i.e.*, cells from a human chronic myelogenous leukemia cell line) and A431 cells (*i.e.*, cells from a human epidermoid carcinoma cell line).

### Example 9. Isolation of Cells From Blood Without Dilution of Anticancer Drug.

This example demonstrates the recovery of K562 cells (*i.e.*, cells from a human chronic myelogenous leukemia cell line) from blood spiked with K562 cells using magnetic bead capture with anti-CD45 antibodies, followed by the preparation of a K562 cell lysate and determination of the expression and/or activation status of one or more oncogenic fusion proteins (*e.g.*, BCR-ABL), substrates thereof, pathways thereof, or combinations thereof. This example also demonstrates the recovery of white blood cells from patient blood samples using magnetic bead capture with anti-CD45 and/or anti-CD 15 antibodies, followed by the preparation of a cell lysate and determination of the expression and/or activation status of one or more oncogenic fusion proteins (*e.g.*, BCR-ABL), substrates thereof, pathways thereof, or combinations thereof. By eliminating the need for any wash steps after cell isolation, the methods described herein are advantageous because cells of interest can be recovered from blood without changing the intracellular concentration of an anticancer drug such as a tyrosine kinase inhibitor. As such, the methods described in this example are contrary to the art-accepted practice of washing cells after isolation (*e.g.*, washing bead-bound cells), and provide cell lysates from recovered cells without substantial dilution of an anticancer drug such as a tyrosine kinase inhibitor (*e.g.*, Gleevec^{®}, Tasigna^{®}, Sprycel^{®}, *etc.*) inside the cells.

### K562 Cell Recovery from 1ml of Blood Using CD45 Dynabeads

1. Prepare Buffer 1
   1.1 Add 500mg ofBSAto 500ml of PBS
   1.2 Add 2ml of 0.5M EDTA
2. Prepare Blood and Spike with K562 Cells
   2.1 Obtain 10mls of whole blood from donor
   2.2 Dilute 1:1 dilution of blood with Buffer 1
   2.3 Do a cell count of K562 cells using the automated CellCounter
   2.4 Add 5e6, 1e6, 0.1e6, and 0 K562 cells separately into 1ml of diluted blood for each concentration
3. Wash DynaBeads (Invitrogen Cat. No. 111.53D)
   3.1 Transfer 100µl beads for every 1e7 cells in a 1.5ml eppendorf tube
   3.2 Add 1ml of Buffer 1 and mix gently
   3.3 Place the tube on the magnet for 1 min.
   3.4 Remove the supernatant
   3.5 Remove the tube from the magnet and resuspend in equal amounts of Buffer 1 as the starting volume of beads transferred
4. Cell Isolation
   4.1 Add 100µl of washed beads to each K562 spiked blood sample.
   4.2 Incubate samples on a rotator in a cold room (or room temperature) for 20 min, 2hrs or 1 hr
   4.3 Place samples on magnet and remove supernatant
5. Preparation of Cell Lysate
   5.1 Add 1 ml of cold Lysis Buffer to beads bound with 5e6 K562 cells
   5.2 Add 500 µl of Lysis Buffer to beads bound with 1e6 K562 cells
   5.3 Add 100 µl of Lysis Buffer to beads bounds with 0.1e6 K562 cells
   5.4 Vortex and place on ice for 20'; vortex intermittently
   5.5 Centrifuge for 15' on max speed at 4 degrees
   5.6 Transfer the supernatant to an eppendorf tube to run microarray, *e.g.*, by performing the proximity-mediated immunoassay described herein

### CML Cell Isolation From Patient Blood Sample Using CD45 and/or CD15 Dynabeads

1. Prepare Buffer 1
   1.1 Add 500mg ofBSAto 500ml of PBS
   1.2 Add 2ml of 0.5M EDTA
2. Prepare Patient Blood
   2.1 Obtain 2-3mls of whole blood from patient using EDTA (or Heparin) as an anticoagulant
   2.2 Add or do not add protease inhibitors
3. Wash DynaBeads (Invitrogen Cat. No. 111.53D)
   3.1 Transfer 100µl (200µl, 300µl) beads for every 1ml of blood sample in a 1.5ml eppendorftube
   3.2 Add 1ml of Buffer 1 and mix gently
   3.3 Place the tube on the magnet for 1 min.
   3.4 Remove the supernatant
   3.5 Remove the tube from the magnet and resuspend in 100µl of Buffer 1 as the starting volume of beads transferred
4. Cell Isolation
   4.1 Add the 100µl of washed beads to 1 ml of blood sample in a 1.5ml eppendorf tube
   4.2 Incubate samples on a rotator in a cold room (or room temperature) for 20 min, 2hrs or 1 hr
   4.3 Place samples on magnet and remove supernatant
5. Preparation of Cell Lysate
   5.1 Add 100 µl of Lysis Buffer to beads bounds with cells
   5.2 Vortex and place on ice for 20'; vortex intermittently
   5.3 Centrifuge for 15' on max speed at 4 degrees
   5.4 Transfer the supernatant to an eppendorf tube to run microarray, *e.g.*, by performing the proximity-mediated immunoassay described herein

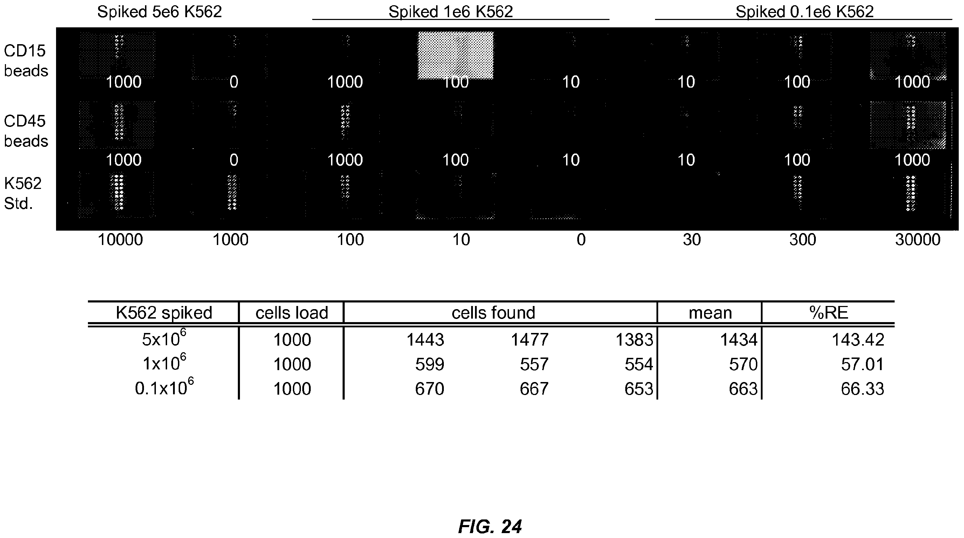

Figure 24 illustrates that phosphorylated BCR-ABL can be detected and measured in cell lysates prepared from K562 cells isolated from blood using anti-CD45 magnetic beads. In particular, the 4G10 antibody (Millipore), which binds to the phospho-tyrosine residue in the Abl portion of the fusion protein, was used to detect phosphorylated BCR-ABL levels. A similar assay can be performed on cell lysates prepared from white blood cells (*e.g.*, chronic myelogenous leukemia (CML) cells) isolated from the blood of patient samples using anti-CD45 and/or anti-CD 15 magnetic beads to detect and measure the presence and/or level of phosphorylated BCR-ABL.

### Example 10. Detection of Both Total Oncogenic Fusion Protein and Total Native Full-Length Protein Levels in Patient Samples.

This example demonstrates a method for the simultaneous detection of the total amount and/or activation state of an oncogenic fusion protein in combination with one or both of the native full-length proteins containing sequences or domains found within the oncogenic fusion protein. In one particular embodiment, the present method enables the detection and/or measurement of both total BCR-ABL levels as well as total native full-length BCR and/or ABL levels in a biological sample such as a blood or bone marrow aspirate sample.

In certain embodiments, native protein levels (*e.g.*, full-length BCR and/or ABL levels) are determined along with oncogenic fusion protein levels (*e.g.*, BCR-ABL levels) in a multiplexed manner on a single pad. In these embodiments, native full-length protein can be advantageously isolated along with oncogenic fusion protein such that the levels of these molecules are determined on the same pad.

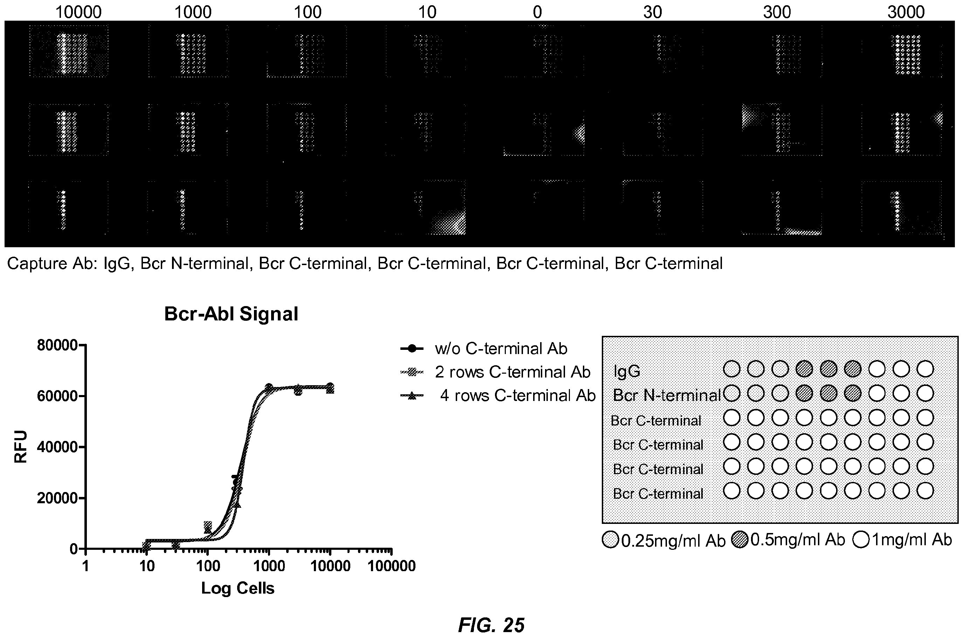

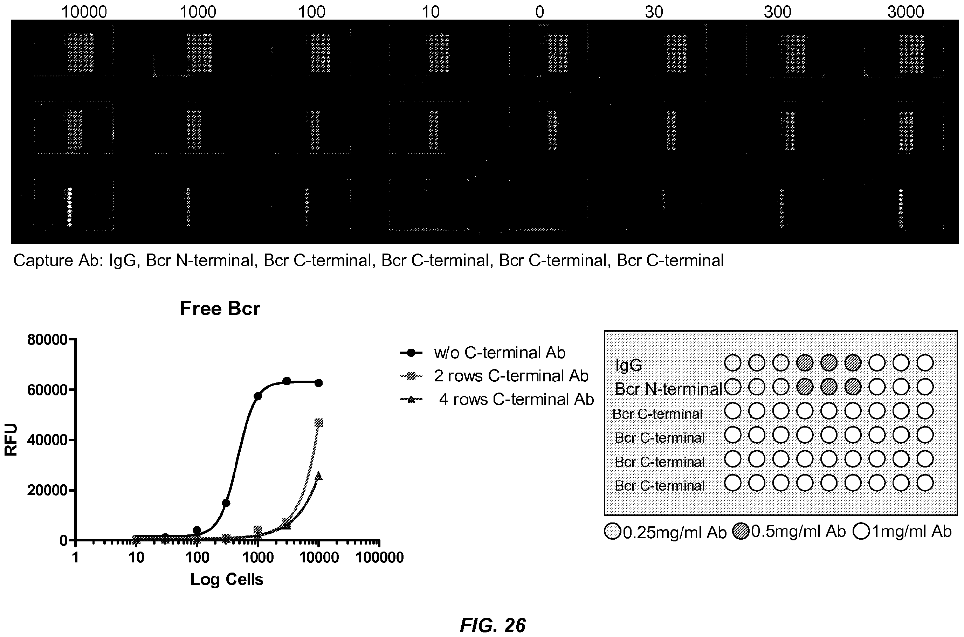

Figure 25 illustrates that total BCR-ABL levels were not changed when an antibody directed to the C-terminus of native full-length BCR (which C-terminal domain is not present in BCR-ABL) was spotted on the same slide in the same pad as an antibody directed to the N-terminal region of BCR-ABL. Figure 26 illustrates that the free native BCR signal detected with an N-terminal-specific BCR antibody was reduced when an antibody directed to the C-terminus of native BCR was spotted on the same slide in the same pad.

In particular embodiments, the method described in this example can be used to detect and/or measure total BCR-ABL levels as well as total native full-length BCR or ABL levels, and a ratio of total BCR-ABL levels to native full-length BCR or ABL levels can be calculated. In some instances, the ratio of BCR-ABL levels to native full-length BCR or ABL levels is calculated to provide a more accurate determination of response indicators such as, for example, a major molecular response, a complete molecular response, a complete cytogenetic response, and combinations thereof. In other instances, the method described in this example can be used to monitor changes in the expression of BCR-ABL with respect to a control such as native full-length BCR or ABL (*e.g.*, by calculating a ratio of total BCR-ABL levels to native full-length BCR or ABL levels) as a function of therapy (*e.g.*, tyrosine kinase inhibitor therapy).

## Claims

1. A method for determining the level or activation state of an oncogenic fusion protein, the method comprising:
(a) producing a cellular extract comprising an extract of cells isolated from a sample, wherein the isolated cells are not washed prior to lysis to produce the cellular extract;
(b) contacting a cellular extract with a first binding moiety specific for a first domain of a first full-length protein under conditions suitable to transform the first full-length protein present in the cellular extract into a complex comprising the first full-length protein and the first binding moiety, wherein the first domain of the first full-length protein is absent from a corresponding oncogenic fusion protein comprising a second, different domain of the first full-length protein fused to a first domain of a second, different full-length protein;
(c) removing the complex from step (b) from the cellular extract to form a cellular extract devoid of the first full-length protein;
(d) contacting the cellular extract from step (c) with a second binding moiety, a third binding moiety, and a fourth binding moiety under conditions suitable to transform the oncogenic fusion protein present in the cellular extract into a complex comprising the oncogenic fusion protein and the second, third, and fourth binding moieties;
wherein the second binding moiety is specific for the second, different domain of the first full-length protein, wherein the second binding moiety is restrained on a solid support in an addressable array,
wherein the third binding moiety is labeled with a glucose oxidase and is specific for one of the following: (i) the first domain of the second, different full-length protein; (ii) the second, different domain of the first full-length protein; or (iii) the site of fusion between the second, different domain of the first full-length protein and the first domain of the second, different full-length protein,
wherein the fourth binding moiety is labeled with a first member of a signal amplification pair and is specific for the first domain of the second, different full-length protein, and
wherein the glucose oxidase generates an oxidizing agent which channels to and reacts with the first member of the signal amplification pair;
(e) incubating the complex from step (d) with a second member of the signal amplification pair to generate an amplified signal; and
(f) detecting the amplified signal generated from the first and second members of the signal amplification pair, thereby determining the level or activation state of the oncogenic fusion protein.

2. The method of claim 1, wherein the sample is selected from the group consisting of whole blood, serum, plasma, urine, sputum, bronchial lavage fluid, tears, nipple aspirate, lymph, saliva, fine needle aspirate (FNA), and combinations thereof.

3. The method of claim 2, wherein the sample is obtained from a patient having cancer.

4. The method of claim 3, wherein the cancer is caused by the formation of an oncogenic fusion protein due to a chromosomal translocation in the cancer cells and wherein the cancer is optionally a hematological malignancy, osteogenic sarcoma, or soft tissue sarcoma, and wherein the hematological malignancy is optionally a leukemia such as a chronic myelogenous leukemia (CML) or lymphoma.

5. The method of claim 2, wherein: (i) the isolated cells are selected from the group consisting of circulating tumor cells, leukocytes, and combinations thereof; or (ii) the isolated cells are stimulated in vitro with growth factors.

6. The method of claim 5(ii), wherein: (i) the isolated cells are incubated with an anticancer drug prior to growth factor stimulation; or (ii) the isolated cells are lysed following growth factor stimulation to produce the cellular extract.

7. The method of any one of claims 1 to 4, wherein the oncogenic fusion protein is selected from the group consisting of BCR-ABL, DEK-CAN, E2A-PBX1, RARa-PML, 1REL-URG, CBF/3-MYH11, AML1-MTG8, EWS-FLI, LYT-10-Cc, HRX-ENL, HRX-AF4, NPM-ALK, IGH-MYC, RUNX1-ETO, TEL-TRKC, TEL-AML1, MLL-AF4, TCR-RBTN2, COL1A1-PDGF, E2A-HLF, PAX3-FKHR, ETV6-NTRK3, RET-PTC, TMRSS-ERG, TPR-MET, and combinations thereof

8. The method of any one of claims 1 to 7, wherein the oncogenic fusion protein is BCR-ABL, and optionally:
wherein the first full-length protein is BCR, or wherein the first domain of the first full-length protein comprises the carboxyl-terminal region of BCR (BCR-C), wherein the second, different domain of the first full-length protein comprises the amino-terminal region of BCR (BCR-N), or wherein the second, different full-length protein is ABL, or wherein the first domain of the second, different full-length protein comprises the carboxyl-terminal region of ABL (ABL-C), or wherein the first full-length protein is ABL, or wherein the second, different full-length protein is BCR.

9. The method of any one of claims 1 to 8, wherein the activation state is selected from the group consisting of a phosphorylation state, ubiquitination state, complexation state, and combinations thereof, or further comprising determining the level or activation state of one or more signal transduction molecules, said one or more signal transduction molecules optionally being a BCR- ABL substrate, said BCR-ABL substrate optionally being selected from the group consisting of CRKL, JAK2, STAT5, VAV, BAP-1, and combinations thereof.

10. The method of any one of claims 1 to 9, wherein: (i) the first binding moiety comprises a first antibody, said first antibody optionally being attached to a solid support, said solid support optionally being selected from the group consisting of glass, plastic, chips, pins, filters, beads, paper, membrane, fiber bundles, and combinations thereof; or (ii) the second binding moiety comprises a second antibody, said second antibody optionally being attached to a solid support.

11. The method of claim 1, wherein the cellular extract from step (c) is contacted with a dilution series of the second binding moiety to form a plurality of complexes comprising the oncogenic fusion protein and the second binding moiety.

12. The method of any one of claims 1 to 11, wherein the third and fourth binding moieties comprise third and fourth antibodies, respectively.

13. The method of claim 12, wherein:
(i) the third and fourth antibodies are both activation state-independent antibodies, and optionally wherein the amplified signal generated from the first and second members of the signal amplification pair is correlative to the total amount of the oncogenic fusion protein; or
(ii) the third antibody is an activation state-independent antibody and the fourth antibody is an activation state-dependent antibody, wherein the amplified signal generated from the first and second members of the signal amplification pair is optionally correlative to the amount of activated oncogenic fusion protein.

14. The method of any one of claims 1 to 13, wherein the third binding moiety is directly labeled with the glucose oxidase, or wherein the fourth binding moiety is directly labeled with the first member of the signal amplification pair, or wherein the fourth binding moiety is labeled with the first member of the signal amplification pair via binding between a first member of a binding pair conjugated to the second detection antibody and a second member of the binding pair conjugated to the first member of the signal amplification pair.

15. The method of any one of claims 1 to 14, further comprising:
(g) contacting the cellular extract with a fifth binding moiety specific for a second domain of the second, different full-length protein under conditions suitable to transform the second, different full-length protein present in the cellular extract into a complex comprising the second, different full-length protein and the fifth binding moiety, wherein the second domain of the second, different full-length protein is absent from the oncogenic fusion protein; and
(h) removing the complex from step (g) from the cellular extract to form a cellular extract devoid of the second, different full-length protein,
wherein step (g) is performed before, during, or after step (b),
and wherein the fifth binding moiety optionally comprises a fifth antibody, wherein the fifth antibody is optionally attached to a solid support, said solid support optionally being selected from the group consisting of glass, plastic, chips, pins, filters, beads, paper, membrane, fiber bundles, and combinations thereof.

16. A method for optimizing therapy and/or reducing toxicity in a subject having cancer and receiving a course of therapy for the treatment of cancer, the method comprising:
(a) lysing isolated cancer cells from a subject after administration of an anticancer drug to produce a cellular extract, wherein the isolated cancer cells are not washed prior to lysis;
(b) measuring a level of expression and/or activation of an oncogenic fusion protein in the cellular extract in accordance with the method of any one of claims 1 to 15; and
(c) comparing the measured level of expression and/or activation of the oncogenic fusion protein to a level of expression and/or activation of the oncogenic fusion protein measured at an earlier time during the course of therapy; and
(d) determining a subsequent dose of the course of therapy for the subject or whether a different course of therapy should be administered to the subject based upon the comparison from step (c).

17. The method of claim 16, wherein the oncogenic fusion protein is BCR-ABL, or wherein the subject is determined to have cancer cells that express the oncogenic fusion protein prior to receiving the course of therapy, said subject optionally being positive for BCR- ABL.

18. The method of any one of claims 16 or 17, wherein both the level of expression and the level of activation of the oncogenic fusion protein are measured in the cellular extract, said step (b) optionally further comprises calculating a ratio of activated to total oncogenic fusion protein levels, said step (c) optionally comprises comparing the calculated ratio of activated to total oncogenic fusion protein levels to a ratio of activated to total oncogenic fusion protein levels calculated for the subject at an earlier time.

19. The method of claim 18, wherein:
(i) the calculated ratio of activated to total oncogenic fusion protein levels correlates with the percent inhibition of the activated oncogenic fusion protein upon anticancer drug treatment;
(ii) the calculated ratio of activated to total oncogenic fusion protein levels is relative to the level of a control protein; or
(iii) wherein the calculated ratio of activated to total oncogenic fusion protein levels comprises a ratio of phospho/total BCR-ABL protein levels
and wherein the control protein optionally comprises a native full-length protein containing sequences or domains found within the oncogenic fusion protein, or wherein the control protein is selected from the group consisting of full-length BCR, full-length ABL, and combinations thereof.

20. The method of any one of claims 16 to 29, wherein less than about 50% inhibition of the level of activation of the oncogenic fusion protein indicates a need to increase the subsequent dose of the course of therapy or to administer a different course of therapy, or wherein less than about 50% inhibition of the level of activation of the oncogenic fusion protein indicates a lack of compliance by the subject to the course of therapy, the existence of possible side effects or toxicity associated with the course of therapy, or combinations thereof, or wherein greater than about 80% inhibition of the level of activation of the oncogenic fusion protein indicates that the subject is on the correct course of therapy at the correct dose.

21. The method of any one of claims 16 to 20, wherein the cancer is chronic myelogenous leukemia (CML), or wherein the anticancer drug is selected from the group consisting of a monoclonal antibody, tyrosine kinase inhibitor, chemotherapeutic agent, hormonal therapeutic agent, radiotherapeutic agent, vaccine, and combinations thereof, said tyrosine kinase inhibitor is selected from the group consisting of imatinib mesylate, nilotinib, dasatinib, bosutinib (SKI-606), and combinations thereof

22. The method of any one of claims 16 to 21, wherein steps (c) to (e) alternatively comprise:
(c') measuring a level of expression and/or activation of an oncogenic fusion protein and one or more signal transduction molecules in its pathway in the cellular extract; and
(d') comparing the measured level of expression and/or activation of the oncogenic fusion protein and signal transduction molecules to a level of expression and/or activation of the oncogenic fusion protein and signal transduction molecules measured at an earlier time during the course of therapy; and
(e') determining a subsequent dose of the course of therapy for the subject or whether a different course of therapy should be administered to the subject based upon the comparison from step (d').

23. A method for selecting a suitable anticancer drug for the treatment of a cancer, or for identifying the response of a cancer to treatment with an anticancer drug, or for predicting the response of a subject having cancer to treatment with an anticancer drug, or for determining whether a subject having cancer is resistant to treatment with an anticancer drug, the method comprising:
(a) lysing isolated cancer cells from a subject after administration of an anticancer drug, or prior to incubation with an anticancer drug, to produce a cellular extract, wherein the isolated cancer cells are not washed prior to lysis;
(b) measuring a level of expression and/or activation of an oncogenic fusion protein in the cellular extract in accordance with the method of anyone of claims 1 to 15; and
(c) determining whether the anticancer drug is suitable or unsuitable for the treatment of the cancer by comparing the level of expression and/or activation detected for the oncogenic fusion protein with a reference level and/or activation profile generated in the absence of the anticancer drug, identifying the cancer as responsive or non-responsive to treatment with the anticancer drug by comparing the level of expression and/or activation detected for the oncogenic fusion protein with a reference level and/or activation profile generated in the absence of the anticancer drug,
predicting the likelihood that the subject will respond to treatment with the anticancer drug by comparing the level of expression and/or activation detected for the oncogenic fusion protein with a reference level and/or activation profile generated in the absence of the anticancer drug,
determining whether the subject is resistant or sensitive to treatment with the anticancer drug by comparing the level of expression and/or activation detected for the oncogenic fusion protein with a reference level and/or activation profile generated in the absence of the anticancer drug or in the presence of the anticancer drug at an earlier time, respectively.

24. The method of claim 23, wherein the cancer is chronic myelogenous leukemia (CML), or wherein the anticancer drug is selected from the group consisting of a monoclonal antibody, tyrosine kinase inhibitor, chemotherapeutic agent, hormonal therapeutic agent, radiotherapeutic agent, vaccine, and combinations thereof, said monoclonal antibody optionally being selected from the group consisting of trastuzumab, alemtuzumab, bevacizumab, cetuximab, gemtuzumab, panitumumab, rituximab, tositumomab, and combinations thereof, said tyrosine kinase inhibitor optionally being selected from the group consisting of imatinib mesylate, nilotinib, dasatinib, bosutinib (SKI-606), gefitinib, sunitinib, erlotinib, lapatinib, canertinib (CI 1033), semaxinib (SU5416), vatalanib (PTK787/ZK222584), sorafenib (BAY 43-9006), leflunomide (SU101), vandetanib (ZD6474), and combinations thereof, said chemotherapeutic agent optionally being selected from the group consisting of pemetrexed, gemcitabine, sirolimus (rapamycin), rapamycin analogs, platinum compounds, carboplatin, cisplatin, satraplatin, paclitaxel, docetaxel (Taxotere®), temsirolimus (CCI-779), everolimus (RAD001), and combinations thereof, said hormonal therapeutic agent optionally being selected from the group consisting of aromatase inhibitors, selective estrogen receptor modulators, steroids, finasteride, gonadotropinreleasing hormone agonists, pharmaceutically acceptable salts thereof, stereoisomers thereof, derivatives thereof, analogs thereof, and combinations thereof, said radiotherapeutic agent optionally being selected from the group consisting of ⁴⁷Sc, ^{6a}CU, ⁶⁷CU, ⁸⁹Sr, ⁸⁶Y, ⁸⁷Y, ⁹⁰Y, ¹⁰⁵Rh, ¹¹¹A, ¹¹¹In, ^{117m}Sn, ¹⁴⁹Pm, ¹⁵³Sm, ¹⁶⁶Ho, ¹⁷⁷Lu, ¹⁸⁶Re, ¹⁸⁸Re, ²¹¹At, ²¹²Bi, and combinations thereof,

25. The method of any one of claims 1 to 7, wherein the oncogenic fusion protein is BCR-ABL and wherein the first binding moiety is an anti-BCR antibody specific for a C-terminal domain of full-length BCR protein, wherein the second binding moiety is optionally an anti-BCR antibody specific for an N-terminal domain of full-length BCR protein, wherein the third binding moiety is optionally an anti-ABL antibody specific for a C-terminal domain of full-length ABL protein, and wherein the fourth binding moiety is optionally an antibody which binds to phospho-ABL-C.

26. The method of claim 1, wherein the solid support is selected from the group consisting of glass, plastic, chips, filters, paper, membrane, and combinations thereof.

## Patentansprüche

1. Verfahren zum Bestimmen des Pegels oder Aktivierungszustands eines onkogenen Fusionsproteins, das Verfahren umfassend:
(a) Erzeugen eines Zellenextrakts umfassend einen Extrakt isolierter Zellen aus einer Probe, wobei die isolierten Zellen vor einer Lyse zum Erzeugen des Zellenextrakts nicht gewaschen werden;
(b) Inkontaktbringen eines Zellenextrakts mit einem ersten Binderest, der spezifisch für eine erste Domäne eines ersten Volllängenproteins ist, unter geeigneten Bedingungen, um das erste Volllängenprotein, das im Zellenextrakt vorhanden ist, in einen Komplex umfassend das erste Volllängenprotein und den ersten Binderest umzuwandeln, wobei die erste Domäne des ersten Volllängenproteins an einem entsprechenden onkogenen Fusionsprotein umfassend eine zweite, verschiedene Domäne des ersten Volllängenproteins, das an einer ersten Domäne eines zweiten, verschiedenen Volllängenproteins fusioniert ist, fehlt;
(c) Entfernen des Komplexes von Schritt (b) aus dem Zellenextrakt, um ein Zellenextrakt ohne das erste Volllängenprotein zu bilden;
(d) Inkontaktbringen des Zellenextrakts von Schritt (c) mit einem zweiten Binderest, einem dritten Binderest und einem vierten Binderest unter geeigneten Bedingungen, um das onkogene Fusionsprotein, das im Zellenextrakt vorhanden ist, in einen Komplex umfassend das onkogene Fusionsprotein und den zweiten, dritten und vierten Binderest umzuwandeln;
wobei der zweite Binderest spezifisch für die zweite, verschiedene Domäne des ersten Volllängenproteins ist, wobei der zweite Binderest an einem festen Untergrund in einem adressierbaren Array zurückgehalten wird,
wobei der dritte Binderest mit einer Glucoseoxidase markiert ist und spezifisch für eines der folgenden ist: (i) die erste Domäne des zweiten, verschiedenen Volllängenproteins; (ii) die zweite, verschiedene Domäne des ersten Volllängenproteins; oder (iii) die Fusionsstelle zwischen der zweiten, verschiedenen Domäne des ersten Volllängenproteins und der ersten Domäne des zweiten, verschiedenen Volllängenproteins,
wobei der vierte Binderest mit einem ersten Element eines Signalverstärkungspaar markiert ist und spezifisch für die erste Domäne des zweiten, verschiedenen Volllängenproteins ist, und
wobei die Glucoseoxidase ein Oxidationsmittel erzeugt, das zum ersten Element des Signalverstärkungspaars kanalisiert und damit reagiert;
(e) Inkubieren des Komplexes von Schritt (d) mit einem zweiten Element des Signalverstärkungspaars, um ein verstärktes Signal zu erzeugen; und
(f) Detektieren des verstärkten Signals, das vom ersten und zweiten Element des Signalverstärkungspaars erzeugt wird, wodurch der Pegel oder Aktivierungszustand des onkogenen Fusionsproteins bestimmt wird.

2. Verfahren nach Anspruch 1, wobei die Probe ausgewählt ist von der Gruppe bestehend aus Vollblut, Serum, Plasma, Urin, Sputum, Bronchiallavage-Fluid, Tränen, Mamillenaspirat, Lymphe, Saliva, Feinnadelaspirat (FNA) und Kombinationen davon.

3. Verfahren nach Anspruch 2, wobei die Probe von einem Patienten erlangt wird, der Krebs hat.

4. Verfahren nach Anspruch 3, wobei der Krebs durch die Bildung eines onkogenen Fusionsproteins aufgrund einer chromosomalen Translokation in den Krebszellen verursacht wird, und wobei der Krebs optional ein hämatologisches Malignom, osteogenes Sarkom oder Weichgewebesarkom ist, und wobei das hämatologische Malignom optional eine Leukämie ist, wie z. B. eine chronische myelogene Leukämie (CML) oder Lymphom.

5. Verfahren nach Anspruch 2, wobei: (i) die isolierten Zellen ausgewählt sind von der Gruppe bestehend aus zirkulierenden Tumorzellen, Leukozyten und Kombinationen davon; oder (ii) die isolierten Zellen *in vitro* mit Wachstumsfaktoren stimuliert werden.

6. Verfahren nach Anspruch 5(ii), wobei: (i) die isolierten Zellen vor einer Wachstumsfaktorstimulation mit einem Antikrebs-Medikament inkubiert werden; oder (ii) die isolierten Zellen nach einer Wachstumsfaktorstimulation lysiert werden, um den Zellenextrakt zu erzeugen.

7. Verfahren nach einem der Ansprüche 1 bis 4, wobei das onkogene Fusionsprotein ausgewählt ist von der Gruppe bestehend aus BCR-ABL, DEK-CAN, E2A-PBX1, RARa-PML, 1REL-URG, CBF/3-MYH11, AML1-MTG8, EWS-FLI, LYT-10-Cc, HRX-ENL, HRX- AF4, NPM-ALK, IGH-MYC, RUNX1-ETO, TEL-TRKC, TEL-AML1, MLL-AF4, TCR-RBTN2, COL1A1-PDGF, E2A-HLF, PAX3-FKHR, ETV6-NTRK3, RET-PTC, TMRSS-ERG, TPR-MET und Kombinationen davon

8. Verfahren nach einem der Ansprüche 1 bis 7, wobei das onkogene Fusionsprotein BCR-ABL ist, und optional:
wobei das erste Volllängenprotein BCR ist, oder wobei die erste Domäne des ersten Volllängenproteins die Carboxyl-terminale Region von BCR (BCR-C) umfasst, wobei die zweite, verschiedene Domäne des ersten Volllängenproteins die Amino-terminale Region von BCR (BCR-N) umfasst, oder wobei das zweite, verschiedene Volllängenprotein ABL ist, oder wobei die erste Domäne des zweiten, verschiedenen Volllängenproteins die Carboxyl-terminale Region von ABL (ABL-C) umfasst, oder wobei das erste Volllängenprotein ABL ist, oder wobei das zweite, verschiedene Volllängenprotein BCR ist.

9. Verfahren nach einem der Ansprüche 1 bis 8, wobei der Aktivierungszustand ausgewählt ist von der Gruppe bestehend aus einem Phosphorylierungszustand, Ubiquitinierungszustand, Komplexierungszustand und Kombinationen davon, oder ferner umfassend ein Bestimmen des Pegels oder Aktivierungszustands von einem oder mehreren Signaltransduktionsmolekülen, wobei das eine oder die mehreren Signaltransduktionsmoleküle optional ein BCR-ABL-Substrat sind, wobei das BCR-ABL-Substrat optional ausgewählt ist von der Gruppe bestehend aus CRKL, JAK2, STAT5, VAV, BAP-1 und Kombinationen davon.

10. Verfahren nach einem der Ansprüche 1 bis 9, wobei: (i) der erste Binderest einen ersten Antikörper umfasst, wobei der erste Antikörper optional an einem festen Untergrund befestigt ist, wobei der feste Untergrund optional ausgewählt ist von der Gruppe bestehend aus Glas, Kunststoff, Spänen, Stiften, Filtern, Kugeln, Papier, Membran, Faserbündeln und Kombinationen davon; oder (ii) der zweite Binderest einen zweiten Antikörper umfasst, wobei der zweite Antikörper optional an einem festen Untergrund befestigt ist.

11. Verfahren nach Anspruch 1, wobei der Zellenextrakt von Schritt (c) mit einer Verdünnungsreihe des zweiten Binderests in Kontakt gebracht wird, um eine Vielzahl von Komplexen umfassend das onkogene Fusionsprotein und den zweiten Binderest zu bilden.

12. Verfahren nach einem der Ansprüche 1 bis 11, wobei der dritte und vierte Binderest jeweils dritte und vierte Antikörper, umfassen.

13. Verfahren nach Anspruch 12, wobei:
(i) der dritte und vierte Antikörper beide vom Aktivierungszustand unabhängige Antikörper sind, und optional wobei das verstärkte Signal, das vom ersten und zweiten Element des Signalverstärkungspaar erzeugt wird, korrelativ zur Gesamtmenge des onkogenen Fusionsproteins ist; oder
(ii) der dritte Antikörper ein vom Aktivierungszustand unabhängiger Antikörper ist und der vierte Antikörper ein vom Aktivierungszustand abhängiger Antikörper ist, wobei das verstärkte Signal, das vom ersten und zweiten Element des Signalverstärkungspaar erzeugt wird, optional korrelativ zur Menge des aktivierten onkogenen Fusionsproteins ist.

14. Verfahren nach einem der Ansprüche 1 bis 13, wobei der dritte Binderest direkt mit der Glucoseoxidase markiert ist, oder wobei der vierte Binderest direkt mit dem ersten Element des Signalverstärkungspaars markiert ist, oder wobei der vierte Binderest mit dem ersten Element des Signalverstärkungspaars mittels einer Bindung zwischen einem ersten Element eines Bindungspaars, das an den zweiten Detektionsantikörpers konjugiert ist, und einem zweiten Element des Bindungspaars, das an das erste Element des Signalverstärkungspaar konjugiert ist, markiert ist.

15. Verfahren nach einem der Ansprüche 1 bis 14, ferner umfassend:
(g) Inkontaktbringen des Zellenextrakts mit einem fünften Binderest, der spezifisch für eine zweite Domäne des zweiten, verschiedenen Volllängenproteins ist, unter geeigneten Bedingungen, um das zweite, verschiedene Volllängenprotein, das im Zellenextrakt vorhanden ist, in einen Komplex umfassend das zweite, verschiedene Volllängenprotein und den fünften Binderest umzuwandeln, wobei die zweite Domäne des zweiten, verschiedenen Volllängenproteins an dem onkogenen Fusionsprotein fehlt; und
(h) Entfernen des Komplexes von Schritt (g) aus dem Zellenextrakt, um ein Zellenextrakt ohne das zweite, verschiedene Volllängenprotein zu bilden,
wobei Schritt (g) vor, während oder nach Schritt (b) ausgeführt wird,
und wobei der fünfte Binderest optional einen fünften Antikörper umfasst, wobei der fünfte Antikörper optional an einem festen Untergrund befestigt ist, wobei der feste Untergrund optional ausgewählt ist von der Gruppe bestehend aus Glas, Kunststoff, Spänen, Stiften, Filtern, Kugeln, Papier, Membran, Faserbündeln und Kombinationen davon.

16. Verfahren zum Optimieren einer Therapie und/oder zum Reduzieren einer Toxizität in einem Subjekt, das Krebs hat und einen Therapieverlauf zur Behandlung von Krebs erhält, das Verfahren umfassend:
(a) Lysieren isolierter Krebszellen von einem Subjekt nach Verabreichung eines Antikrebs-Medikaments zum Erzeugen eines Zellenextrakts, wobei die isolierten Krebszellen vor einer Lyse nicht gewaschen werden;
(b) Messen eines Expressions- und/oder Aktivierungspegels eines onkogenen Fusionsproteins im Zellenextrakt gemäß dem Verfahren nach einem der Ansprüche 1 bis 15; und
(c) Vergleichen des gemessenen Expressions- und/oder Aktivierungspegels des onkogenen Fusionsproteins mit einem Expressions- und/oder Aktivierungspegel des onkogenen Fusionsproteins, der zu einer früheren Zeit im Verlauf der Therapie gemessen wurde; und
(d) Bestimmen einer nachfolgenden Dosierung des Therapieverlaufs für das Subjekt, oder ob dem Subjekt ein anderer Therapieverlauf verabreicht werden soll, beruhend auf dem Vergleich von Schritt (c).

17. Verfahren nach Anspruch 16, wobei das onkogene Fusionsprotein BCR-ABL, oder wobei bestimmt wird, dass das Subjekt Krebszellen hat, die das onkogene Fusionsprotein exprimieren, bevor der Therapieverlauf erhalten wird, wobei das Subjekt optional positiv für BCR- ABL ist.

18. Verfahren nach einem der Ansprüche 16 oder 17, wobei der Expressionspegel und der Aktivierungspegel des onkogenen Fusionsproteins im Zellenextrakt gemessen werden, wobei der Schritt (b) optional ferner ein Berechnen eines Verhältnisses von aktivierten zu gesamten onkogenen Fusionsproteinpegeln umfasst, wobei der Schritt (c) optional ein Vergleichen des berechneten Verhältnisses von aktivierten zu gesamten onkogenen Fusionsproteinpegeln mit einem Verhältnis von aktivierten zu gesamten onkogenen Fusionsproteinpegeln, die für das Subjekt zu einer früheren Zeit berechnet wurde, umfasst.

19. Verfahren nach Anspruch 18, wobei:
(i) das berechnete Verhältnis von aktivierten zu gesamten onkogenen Fusionsproteinpegeln mit der prozentuellen Hemmung des aktivierten onkogenen Fusionsproteins bei einer Antikrebs-Medikament-Behandlung umfasst;
(ii) das berechnete Verhältnis von aktivierten zu gesamten onkogenen Fusionsproteinpegeln sich auf den Pegel eines Kontrollproteins bezieht; oder
(iii) wobei das berechnete Verhältnis von aktivierten zu gesamten onkogenen Fusionsproteinpegeln ein Verhältnis von Phospho/gesamten BCR-ABL-Proteinpegeln umfasst
und wobei das Kontrollprotein optional ein Stamm-Volllängenprotein umfasst, das Sequenzen oder Domänen enthält, die im onkogenen Fusionsprotein vorgefunden werden, oder wobei das Kontrollprotein ausgewählt ist von der Gruppe bestehend aus Volllängen-BCR, Volllängen-ABL und Kombinationen davon.

20. Verfahren nach einem der Ansprüche 16 bis 29, wobei eine Hemmung des Aktivierungspegels des onkogenen Fusionsproteins von weniger als 50 % auf eine Notwendigkeit hinweist, die nachfolgende Dosierung des Therapieverlaufs zu erhöhen, oder einen anderen Therapieverlauf zu verabreichen, oder wobei eine Hemmung des Aktivierungspegels des onkogenen Fusionsproteins von weniger als 50 % auf ein Nichteinhalten des Therapieverlaufs durch das Subjekt, auf ein Vorliegen möglicher Nebenwirkungen oder Toxizität in Verbindung mit dem Therapieverlauf oder Kombinationen davon hinweist, oder wobei eine Hemmung des Aktivierungspegels des onkogenen Fusionsproteins von mehr als 80 % darauf hinweist, dass sich das Subjekt im korrekten Therapieverlauf mit korrekter Dosierung befindet.

21. Verfahren nach einem der Ansprüche 16 bis 20, wobei der Krebs chronische myelogener Leukämie (CML) ist, oder wobei das Antikrebs-Medikament ausgewählt ist von der Gruppe bestehend aus einem monoklonalen Antikörper, Tyrosinkinase-Hemmer, chemotherapeutischen Mittel, hormonalen therapeutischen Mittel, strahlentherapeutischen Mittel, Impfstoff und Kombinationen davon, wobei der Tyrosinkinase-Hemmer ausgewählt ist von der Gruppe bestehend aus Imatinibmesylat, Nilotinib, Dasatinib, Bosutinib (SKI-606) und Kombinationen davon.

22. Verfahren nach einem der Ansprüche 16 bis 21, wobei die Schritte (c) bis (e) alternativ umfassen:
(c') Messen eines Expressions- und/oder Aktivierungspegels eines onkogenen Fusionsproteins und eines oder mehrerer Signaltransduktionsmoleküle auf seinem Weg im Zellenextrakt; und
(d') Vergleichen des gemessenen Expressions- und/oder Aktivierungspegels des onkogenen Fusionsproteins und der Signaltransduktionsmoleküle mit einem Expressions- und/oder Aktivierungspegel des onkogenen Fusionsproteins und der Signaltransduktionsmoleküle, der zu einer früheren Zeit im Verlauf der Therapie gemessen wurde; und
(e') Bestimmen einer nachfolgenden Dosierung des Therapieverlaufs für das Subjekt, oder ob dem Subjekt ein anderer Therapieverlauf verabreicht werden soll, beruhend auf dem Vergleich von Schritt (d').

23. Verfahren zum Auswählen eines Antikrebs-Medikaments für die Behandlung eines Krebses, oder zum Identifizieren des Ansprechens eines Krebses auf eine Behandlung mit einem Antikrebs-Medikament, oder zum Vorhersagen des Ansprechens eines Subjekts, das Krebs hat, auf eine Behandlung mit einem Antikrebs-Medikament, oder zum Bestimmen, ob ein Subjekt, das Krebs hat, resistent auf eine Behandlung mit einem Antikrebs-Medikament ist, das Verfahren umfassend:
(a) Lysieren isolierter Krebszellen von einem Subjekt nach Verabreichung eines Antikrebs-Medikaments oder vor einer Inkubation mit einem Antikrebs-Medikament, um einen Zellenextrakt zu erzeugen, wobei die isolierten Krebszellen vor einer Lyse nicht gewaschen werden;
(b) Messen eines Expressions- und/oder Aktivierungspegels eines onkogenen Fusionsproteins im Zellenextrakt gemäß dem Verfahren nach einem der Ansprüche 1 bis 15; und
(c) Bestimmen ob das Antikrebs-Medikament für die Behandlung des Krebses geeignet oder ungeeignet ist, durch Vergleichen des detektierten Expressions- und/oder Aktivierungspegels für das onkogene Fusionsprotein mit einem Referenzpegel und/oder Aktivierungsprofil, das In Abwesenheit des Antikrebs-Medikaments erzeugt wird, Identifizieren des Krebses als ansprechend oder nicht ansprechend auf eine Behandlung mit dem Antikrebs-Medikament durch Vergleichen des detektierten Expressions- und/oder Aktivierungspegels für das onkogene Fusionsprotein mit einem Referenzpegel und/oder Aktivierungsprofil, das In Abwesenheit des Antikrebs-Medikaments erzeugt wird,
Vorhersehen der Wahrscheinlichkeit, dass das Subjekt auf eine Behandlung mit dem Antikrebs-Medikament ansprechen wird, durch Vergleichen des detektierten Expressions- und/oder Aktivierungspegels für das onkogene Fusionsprotein mit einem Referenzpegel und/oder Aktivierungsprofil, das In Abwesenheit des Antikrebs-Medikaments erzeugt wird,
Bestimmen, ob das Subjekt auf eine Behandlung mit dem Antikrebs-Medikament resistent oder empfänglich ist, durch Vergleichen des detektierten Expressions- und/oder Aktivierungspegels für das onkogene Fusionsprotein mit einem Referenzpegel und/oder Aktivierungsprofil, das in Abwesenheit des Antikrebs-Medikaments bzw. in der Anwesenheit des Antikrebs-Medikaments zu einer früheren Zeit erzeugt wird.

24. Verfahren nach Anspruch 23, wobei der Krebs chronische myelogener Leukämie (CML) ist, oder wobei das Antikrebs-Medikament ausgewählt ist von der Gruppe bestehend aus einem monoklonalen Antikörper, Tyrosinkinase-Hemmer, chemotherapeutischen Mittel, hormonalen therapeutischen Mittel, strahlentherapeutischen Mittel, Impfstoff und Kombinationen davon, wobei der monoklonale Antikörper optional ausgewählt ist von der Gruppe bestehend aus Trastuzumab, Alemtuzumab, Bevacizumab, Cetuximab, Gemtuzumab, Panitumumab, Rituximab, Tositumomab und Kombinationen davon, wobei der Tyrosinkinase-Hemmer optional ausgewählt ist von der Gruppe bestehend aus Imatinib Mesylat, Nilotinib, Dasatinib, Bosutinib (SKI-606), Gefitinib, Sunitinib, Erlotinib, Lapatinib, Canertinib (CI 1033), Semaxinib (SU5416), Vatalanib (PTK787/ZK222584), Sorafenib (BAY 43-9006), Leflunomid (SU101), Vandetanib (ZD6474) und Kombinationen davon, wobei das chemotherapeutische Mittel optional ausgewählt ist von der Gruppe bestehend aus Pemetrexed, Gemcitabin, Sirolimus (Rapamycin), Rapamycin-Analoge, Platinverbindungen, Carboplatin, Cisplatin, Satraplatin, Paclitaxel, Docetaxel (Taxotere®), Temsirolimus (CCI-779), Everolimus (RAD001) und Kombinationen davon, wobei das hormonale therapeutische Mittel optional ausgewählt ist von der Gruppe bestehend aus Aromatase-Hemmern, selektiven Östrogenrezeptor-Modulatoren, Steroiden, Finasterid, Gonadotropin-freisetzenden Hormonagonisten, pharmazeutisch annehmbaren Salzen davon, Stereoisomeren davon, Derivaten davon, Analogen davon und Kombinationen davon, wobei das strahlentherapeutische Mittel optional ausgewählt ist von der Gruppe bestehend aus ⁴⁷Sc, ⁶⁴CU, ⁶⁷CU, ⁸⁹Sr, ⁸⁶Y, ⁸⁷Y, ⁹⁰Y, ¹⁰⁵Rh, ¹¹¹A, ¹¹¹In, ^{117m}Sn, ¹⁴⁹Pm, ¹⁵³Sm, ¹⁶⁶Ho, ¹⁷⁷Lu, ¹⁸⁶Re, ¹⁸⁸Re, ²¹¹At, ²¹²Bi und Kombinationen davon.

25. Verfahren nach einem der Ansprüche 1 bis 7, wobei das onkogene Fusionsprotein BCR-ABL ist, und wobei der erste Binderest ein Anti-BCR-Antikörper ist, der spezifisch für eine C-terminale Domäne von Volllängen-BCR-Protein ist, wobei der zweite Binderest optional ein Anti-BCR-Antikörper ist, der spezifisch für eine N-terminale Domäne von Volllängen-BCR-Protein ist, wobei der dritte Binderest optional ein Anti-ABL-Antikörper ist, der spezifisch für eine C-terminale Domäne von Volllängen-ABL-Protein ist, und wobei der vierte Binderest optional ein Antikörper ist, der an Phospho-ABL-C bindet.

26. Verfahren nach Anspruch 1, wobei der feste Untergrund ausgewählt ist von der Gruppe bestehend aus Glas, Kunststoff, Spänen, Filtern, Papier, Membran und Kombinationen davon.

## Revendications

1. Procédé permettant de déterminer le taux ou l'état d'activation d'une protéine de fusion oncogène, le procédé comprenant :
(a) la production d'un extrait cellulaire comprenant un extrait de cellules isolées à partir d'un échantillon, les cellules isolées n'étant pas lavées avant la lyse pour produire l'extrait cellulaire ;
(b) la mise en contact d'un extrait cellulaire avec une première fraction de liaison spécifique pour un premier domaine d'une première protéine de pleine longueur dans des conditions appropriées pour transformer la première protéine de pleine longueur présente dans l'extrait cellulaire en un complexe comprenant la première protéine de pleine longueur et la première fraction de liaison, le premier domaine de la première protéine de pleine longueur étant absent d'une protéine de fusion oncogène correspondante comprenant un deuxième domaine différent de la première protéine de pleine longueur fusionné avec un premier domaine d'une deuxième protéine de pleine longueur différente ;
(c) le retrait du complexe de l'étape (b) de l'extrait cellulaire pour former un extrait cellulaire dépourvu de la première protéine de pleine longueur ;
(d) la mise en contact de l'extrait cellulaire de l'étape (c) avec une deuxième fraction de liaison, une troisième fraction de liaison et une quatrième fraction de liaison dans des conditions appropriées pour la transformation de la protéine de fusion oncogène présente dans l'extrait cellulaire en un complexe comprenant la protéine de fusion oncogène et les deuxième, troisième et quatrième fractions de liaison ;
la deuxième fraction de liaison étant spécifique du deuxième domaine différent de la première protéine de pleine longueur, la deuxième fraction de liaison étant retenue sur un support solide dans un réseau adressable,
la troisième fraction de liaison étant marquée avec une glucose oxydase et étant spécifique de l'un des éléments suivants : (i) le premier domaine de la deuxième protéine de pleine longueur différente ; (ii) le deuxième domaine différent de la première protéine de pleine longueur ; ou (iii) le site de fusion entre le deuxième domaine différent de la première protéine de pleine longueur et le premier domaine de la deuxième protéine de pleine longueur différente,
la quatrième fraction de liaison étant marquée avec un premier élément d'une pair d'amplification de signal et étant spécifique du premier domaine de la deuxième protéine de pleine longueur différente, et
la glucose oxydase générant un agent oxydant qui communique et réagit avec le premier élément de la paire d'amplification de signal ;
(e) l'incubation du complexe de l'étape (d) avec un second élément de la paire d'amplification de signal pour générer un signal amplifié ; et
(f) la détection du signal amplifié généré à partir des premier et second éléments de la paire d'amplification de signal pour ainsi déterminer le taux ou l'état d'activation de la protéine de fusion oncogène.

2. Procédé de la revendication 1, dans lequel l'échantillon est choisi dans le groupe constitué par le sang total, le sérum, le plasma, l'urine, les crachats, un fluide de lavage bronchique, les larmes, un prélèvement par aspiration mammaire, la lymphe, la salive, un prélèvement par ponction à l'aiguille fine (FNA) et leurs combinaisons.

3. Procédé de la revendication 2, dans lequel l'échantillon est prélevé sur un patient atteint du cancer.

4. Procédé de la revendication 3, dans lequel le cancer est causé par la formation d'une protéine de fusion oncogène due à une translocation chromosomique dans les cellules cancéreuses et dans lequel le cancer est éventuellement une hémopathie maligne, un sarcome ostéogénique ou un sarcome des tissus mous, et dans lequel l'hémopathie maligne est éventuellement une leucémie telle qu'une leucémie aiguë myéloblastique (LAM) ou un lymphome.

5. Procédé de la revendication 2, dans lequel : (i) les cellules isolées sont choisies dans le groupe constitué par les cellules tumorales en circulation, les leucocytes et leurs combinaisons ; ou (ii) les cellules isolées sont stimulées in vitro avec des facteurs de croissance.

6. Procédé de la revendication 5(ii), dans lequel : (i) les cellules isolées sont incubées avec nu médicament anticancéreux avant la stimulation par facteur de croissance ; ou (ii) les cellules isolées sont lysées après la stimulation par facteur de croissance pour la production de l'extrait cellulaire.

7. Procédé de l'une quelconque des revendications 1 à 4, dans lequel la protéine de fusion oncogène est choisie dans le groupe constitué par BCR-ABL, DEK-CAN, E2A-PBX1, RARa-PML, 1REL-URG, CBF/3-MYH11, AML1-MTG8, EWS-FLI, LYT-10-Cc, HRX-ENL, HRX- AF4, NPM-ALK, IGH-MYC, RUNX1-ETO, TEL-TRKC, TEL-AML1, MLL-AF4, TCR-RBTN2, COL1A1-PDGF, E2A-HLF, PAX3-FKHR, ETV6-NTRK3, RET-PTC, TMRSS-ERG, TPR-MET, et leurs combinaisons.

8. Procédé de l'une quelconque des revendications 1 à 7, dans lequel la protéine de fusion oncogène est BCR-ABL, et éventuellement :
dans lequel la première protéine de pleine longueur est BCR, ou dans lequel le premier domaine de la première protéine de pleine longueur comprend la région carboxyl-terminale de BCR (BCR-C), dans lequel le deuxième domaine différent de la première protéine de pleine longueur comprend la région amino-terminale de BCR (BCR-N), ou dans lequel la deuxième protéine de pleine longueur différente est ABL, ou dans lequel le premier domaine de la deuxième protéine de pleine longueur différente comprend la région carboxyl-terminale de ABL (ABL-C), ou dans lequel la première protéine de pleine longueur est ABL, ou dans lequel la deuxième protéine de pleine longueur différente est BCR.

9. Procédé de l'une quelconque des revendications 1 à 8, dans lequel l'état d'activation est choisi dans groupe constitué par un état de phosphorylation, un état d'ubiquitination, un état de complexation et leurs combinaisons, ou comprenant en outre la détermination du taux ou de l'état d'activation d'au moins une molécule de transduction de signal, ladite au moins une molécule de transduction de signal étant éventuellement un substrat BCR-ABL, ledit substrat BCR-ABL étant éventuellement choisi dans le groupe constitué par CRKL, JAK2, STAT5, VAV, BAP-1, et leurs combinaisons.

10. Procédé de l'une quelconque des revendications 1 à 9, dans lequel : (i) la première fraction de liaison comprend un premier anticorps, ledit premier anticorps étant éventuellement fixé à un support solide, ledit support solide étant éventuellement choisi dans le groupe constitué par le verre, le plastique, des puces, des broches, des filtres, des billes, du papier, une membrane, des faisceaux de fibres, et leurs combinaisons ; ou (ii) la deuxième fraction de liaison comprend un deuxième anticorps, ledit deuxième anticorps étant éventuellement fixé à un support solide.

11. Procédé de la revendication 1, dans lequel l'extrait cellulaire de l'étape (c) est mis en contact avec une série de dilutions de la deuxième fraction de liaison pour la formation d'une pluralité de complexes comprenant la protéine de fusion oncogène et la deuxième fraction de liaison.

12. Procédé de l'une quelconque des revendications 1 à 11, dans lequel les troisième et quatrième fractions de liaison comprennent des troisième et quatrième anticorps, respectivement.

13. Procédé de la revendication 12, dans lequel :
(i) les troisième et quatrième anticorps sont tous deux des anticorps indépendants de l'état d'activation, et le signal amplifié généré à partir des premier et second éléments de la paire d'amplification de signal étant éventuellement corrélatif de la quantité totale de la protéine de fusion oncogène ; ou
(ii) le troisième anticorps est un anticorps indépendant de l'état d'activation et le quatrième anticorps est un anticorps dépendant de l'état d'activation, le signal amplifié généré à partir des premier et second éléments de la paire d'amplification de signal étant éventuellement corrélatif de la quantité de la protéine de fusion oncogène.

14. Procédé de l'une quelconque des revendications 1 à 13, dans lequel la troisième fraction de liaison est directement marquée avec la glucose oxydase, ou dans lequel la quatrième fraction de liaison est directement marquée avec le premier élément de la paire d'amplification de signal, ou dans lequel la quatrième fraction de liaison est marquée avec le premier élément de la paire d'amplification de signal par le biais d'une liaison entre un premier élément d'une paire de liaison conjugué avec le deuxième anticorps de détection et un second élément de la paire de liaison conjugué avec le premier élément de la paire d'amplification de signal.

15. Procédé de l'une quelconque des revendications 1 à 14, comprenant en outre :
(g) la mise en contact de l'extrait cellulaire avec une cinquième fraction de liaison spécifique d'un deuxième domaine de liaison de la deuxième protéine de pleine longueur différente dans des conditions appropriées pour une transformation de la deuxième protéine de pleine longueur différente présente dans l'extrait cellulaire en un complexe comprenant la deuxième protéine de pleine longueur différente et la cinquième fraction de liaison, le deuxième domaine de la deuxième protéine de pleine longueur différente étant absent de la protéine de fusion oncogène ; et
(h) le retrait du complexe de l'étape (g) de l'extrait cellulaire pour former un extrait cellulaire dépourvu de la deuxième protéine de pleine longueur différente,
l'étape (g) étant réalisée avent, pendant ou après l'étape (b),
et la cinquième fraction de liaison comprenant éventuellement un cinquième anticorps, le cinquième anticorps étant éventuellement fixé à un support solide, ledit support solide étant éventuellement choisi dans le groupe constitué par le verre, le plastique, des puces, des broches, des filtres, des billes, du papier, une membrane, des faisceaux de fibres, et leurs combinaisons.

16. Procédé permettant d'optimiser une thérapie et/ou de réduire la toxicité chez un sujet atteint du cancer et recevant un cycle de traitement pour le traitement du cancer, le procédé comprenant :
(a) la lyse de cellules cancéreuses isolées chez un sujet après l'administration d'un médicament anticancéreux pour produire un extrait cellulaire, les cellules cancéreuses isolées n'étant pas lavées avant la lyse ;
(b) la mesure d'un taux d'expression et/ou d'activation d'une protéine de fusion oncogène dans l'extrait cellulaire en conformité avec le procédé de l'une quelconque des revendications 1 à 15; et et
(c) la comparaison du taux mesuré d'expression et/ou d'activation de la protéine de fusion oncogène à un taux d'expression et/ou d'activation de la protéine de fusion oncogène mesurée à un moment antérieur au cours du cycle de traitement ; et
(d) la détermination d'une dose ultérieure du cycle de traitement pour le sujet ou du fait qu'un cycle de traitement différent doit être ou non administré au sujet d'après la comparaison de l'étape (c).

17. Procédé de la revendication 16, dans lequel la protéine de fusion oncogène est BCR-ABL, ou dans lequel il est déterminé que le sujet présente des cellules cancéreuses qui expriment la protéine de fusion oncogène avant la réception du cycle de traitement, ledit sujet étant éventuellement positif à BCR- ABL.

18. Procédé de l'une quelconque des revendications 16 ou 17, dans lequel le taux d'expression et le taux d'activation de la protéine de fusion oncogène sont tous deux mesurés dans l'extrait cellulaire, ladite étape (b) comprenant éventuellement en outre le calcul d'un rapport entre le taux de protéine de fusion oncogène activée et le taux de protéine de fusion oncogène totale, ladite étape (c) comprenant éventuellement la comparaison du rapport calculé entre le taux de protéine fusion oncogène activée et le taux de protéine de fusion oncogène totale à un rapport entre le taux de protéine de fusion oncogène activée et le taux de protéine de fusion oncogène totale calculé pour le sujet à un moment antérieur.

19. Procédé de la revendication 18, dans lequel :
(i) le rapport entre le taux de protéine de fusion oncogène activée et le taux de protéine de fusion oncogène totale est en corrélation avec le pourcentage d'inhibition de la protéine de fusion oncogène activée lors d'un traitement médicamenteux anticancéreux ;
(ii) le rapport calculé entre le taux de protéine de fusion oncogène activée et le taux de protéine de fusion oncogène totale est relatif au taux d'une protéine témoin ; ou
(iii) dans lequel le rapport calculé entre le taux de protéine de fusion oncogène activée et le taux de protéine de fusion oncogène totale comprend un rapport entre les taux de protéine BCR-ABL phospho/totale
et dans lequel la protéine témoin comprend éventuellement une protéine de pleine longueur native comprenant des séquences ou domaines présents dans la protéine de fusion oncogène, ou dans lequel la protéine témoin est choisie dans le groupe constitué par BCR pleine longueur, ABL pleine longueur, et leurs combinaisons.

20. Procédé de l'une quelconque des revendications 16 à 29, dans lequel une inhibition inférieure à environ 50 % du taux d'activation de la protéine de fusion oncogène indique une nécessité d'augmenter la dose ultérieure du cycle de traitement ou d'administrer un cycle de traitement différent, ou dans lequel une inhibition inférieure à environ 50 % du taux d'activation de la protéine de fusion oncogène indique la non-compliance du sujet au cycle de traitement, l'existence de possibles effets secondaires ou d'une toxicité associée au cycle de traitement, ou une de leurs combinaisons, ou dans lequel une inhibition supérieure à environ 80 % du taux d'activation de la protéine de fusion oncogène indique que le sujet est suit un cycle de traitement correct à une dose correcte.

21. Procédé de l'une quelconque des revendications 16 à 20, dans lequel le cancer est la leucémie myéloïde chronique (LMC), ou dans lequel le médicament anticancéreux est choisi dans le groupe constitué par un anticorps monoclonal, un inhibiteur de tyrosine kinase, un agent chimiothérapeutique, un agent hormonothérapeutique, un agent radiothérapeutique, un vaccin, et leurs combinaisons, ledit inhibiteur de tyrosine kinase étant choisi dans le groupe constitué par le mésylate d'imatinib, le nilotinib, le dasatinib, le bosutinib (SKI-606), et leurs combinaisons.

22. Procédé de l'une quelconque des revendications 16 à 21, dans lequel les étapes (c) à (e) comprennent en variante :
(c') la mesure d'un taux d'expression et/ou d'activation d'une protéine de fusion oncogène et d'au moins une molécule de transduction de signal sur sa voie dans l'extrait cellulaire ; et
(d') la comparaison du taux mesuré d'expression et/ou d'activation de la protéine de fusion oncogène et des molécules de transduction de signal à un taux d'expression et/ou d'activation de la protéine de fusion oncogène et de molécules de transduction de signal mesuré à un moment antérieur au cours du cycle de traitement ; et
(e') la détermination d'une dose ultérieure du cycle de traitement pour le sujet ou du fait qu'un cycle de traitement différent doit ou non être administré au sujet d'après la comparaison de l'étape (d').

23. Procédé permettant de choisir un médicament anticancéreux approprié pour le traitement d'un cancer, ou d'identifier la réponse d'un cancer à un traitement avec un médicament anticancéreux, ou de prédire la réponse d'un sujet atteint de cancer à un traitement avec un médicament anticancéreux, ou de déterminer si un sujet atteint de cancer est résistant à un traitement avec un médicament anticancéreux, le procédé comprenant :
(a) la lyse de cellules cancéreuses isolées chez un sujet après l'administration d'un médicament anticancéreux, ou avant l'incubation avec un médicament anticancéreux, pour produire un extrait cellulaire, les cellules cancéreuses isolées n'étant pas lavées avant la lyse ;
(b) la mesure d'un taux d'expression et/ou d'activation d'une protéine de fusion oncogène dans l'extrait cellulaire en conformité avec le procédé de l'une quelconque des revendications 1 à 15; et
(c) la détermination du fait que le médicament anticancéreux est approprié ou inapproprié pour le traitement du cancer par une comparaison du taux d'expression et/ou d'activation détecté pour la protéine de fusion oncogène à un taux et/ou un profil d'activation de référence généré en l'absence du médicament anticancéreux, l'identification du cancer comme réactif ou non réactif au traitement avec le médicament anticancéreux par comparaison du taux d'expression et/ou d'activation détecté pour la protéine de fusion oncogène à un taux et/ou un profil d'activation de référence généré en l'absence du médicament anticancéreux,
la prédiction de la probabilité pour le sujet de répondre à un traitement avec le médicament anticancéreux par comparaison du taux d'expression et/ou d'activation détecté pour la protéine de fusion oncogène à un taux et/ou un profil d'activation de référence généré en l'absence du médicament anticancéreux,
la détermination du fait que le sujet est résistant ou sensible à un traitement avec le médicament anticancéreux par comparaison du taux d'expression et/ou d'activation détecté pour la protéine de fusion oncogène à un taux et/ou profil d'activation de référence généré en l'absence du médicament anticancéreux ou en présence du médicament anticancéreux à un moment antérieur, respectivement.

24. Procédé de la revendication 23, dans lequel le cancer est la leucémie myéloïde chronique (LMC), ou dans lequel le médicament anticancéreux est choisi dans le groupe constitué par un anticorps monoclonal, un inhibiteur de tyrosine, un agent chimiothérapeutique, un agent hormonothérapeutique, un agent radiothérapeutique, un vaccin, et leurs combinaisons, ledit anticorps monoclonal étant éventuellement choisi dans le groupe constitué par le trastuzumab, l'alemtuzumab, le bevacizumab, le cetuximab, le gemtuzumab, le panitumumab, le rituximab, le tositumomab, et leurs combinaisons, ledit inhibiteur de tyrosine kinase étant éventuellement choisi dans le groupe constitué par le mésylate d'imatinib, le nilotinib, le dasatinib, le bosutinib (SKI-606), le géfitinib, le sunitinib, l'erlotinib, le lapatinib, le canertinib (CI 1033), le semaxinib (SU5416), le vatalanib (PTK787/ZK222584), le sorafénib (BAY 43-9006), le léflunomide (SU101), le vandétanib (ZD6474), et leurs combinaisons, ledit agent chimiothérapeutique étant éventuellement choisi dans le groupe constitué par le pémétrexed, la gemcitabine, le sirolimus (rapamycine), les analogues de la rapamycine, les composés du platine, le carboplatine, le cisplatine, le satraplatine, le paclitaxel, le docétaxel (Taxotere®), le temsirolimus (CCI-779), l'évérolimus (RAD001), et leurs combinaisons, ledit agent hormonothérapeutique étant éventuellement choisi dans le groupe constitué par les inhibiteurs d'aromatase, les modulateurs sélectifs des récepteurs d'oestrogène, les stéroïdes, le finastéride, les agonistes de l'hormone de libération des gonadotrophines, leurs sels pharmaceutiquement acceptables, leurs dérivés, leurs analogues, et leurs combinaisons, ledit agent radiothérapeutique étant éventuellement choisi dans le groupe constitué par ¹⁷Sc, ⁶⁴CU, ⁶⁷CU, ⁸⁹Sr, ⁸⁶Y, ⁸⁷Y, ⁹⁰Y, ¹⁰⁵Rh, ¹¹¹A, ¹¹¹In, ^{117m}Sn, ¹⁴⁹Pm, ¹⁵³Sm, ¹⁶⁶Ho, ¹⁷⁷Lu, ¹⁸⁶Re, ¹⁸⁸Re, ²¹¹At, ²¹²Bi, et leurs combinaisons.

25. Procédé de l'une quelconque des revendications 1 à 7, dans lequel la protéine de fusion oncogène est BCR-ABL et dans lequel la première fraction de liaison est un anticorps anti-BCR spécifique d'un domaine C-terminal de la protéine BCR de pleine longueur, dans lequel la deuxième fraction de liaison est éventuellement un anticorps anti-BCR spécifique d'un domaine N-terminal de la protéine BCR de pleine longueur, dans lequel la troisième fraction de liaison est éventuellement un anticorps anti-ABL spécifique d'une domaine C-terminal de la protéine ABL de pleine longueur, et dans lequel la quatrième fraction de liaison est éventuellement un anticorps qui se lie à phospho-ABL-C.

26. Procédé de la revendication 1, dans lequel le support solide est choisi dans le groupe constitué par le verre, le plastique, les puces, les filtres, du papier, une membrane, et leurs combinaisons.
